(19) Europäisches Patentamt
European Patent Office
Office européen des brevets

(11) EP 1 602 380 A1

(12) **EUROPEAN PATENT APPLICATION**
published in accordance with Art. 158(3) EPC

(43) Date of publication:
**07.12.2005 Bulletin 2005/49**

(21) Application number: **04719631.6**

(22) Date of filing: **11.03.2004**

(51) Int Cl.[7]: **A61K 45/00**, A61P 1/14, A61P 3/12, A61P 7/06

(86) International application number:
**PCT/JP2004/003227**

(87) International publication number:
**WO 2004/080485 (23.09.2004 Gazette 2004/39)**

(84) Designated Contracting States:
**AT BE BG CH CY CZ DE DK EE ES FI FR GB GR HU IE IT LI LU MC NL PL PT RO SE SI SK TR**
Designated Extension States:
**AL LT LV MK**

(30) Priority: **13.03.2003 JP 2003068963**

(71) Applicant: **Takeda Chemical Industries, Ltd.**
**Osaka-shi, Osaka 541-0045 (JP)**

(72) Inventors:
- **MATSUMOTO, Hirokazu, Takeda Pharmaceutical Co. Ltd Ibaraki 300-4293 (JP)**
- **TAKAGI, Koh, Takeda Pharmaceutical Co. Ltd. Ibaraki 300-4293 (JP)**
- **MORI, Masaaki, Takeda Pharmaceutical Co. Ltd. Ibaraki 300-4293 (JP)**

(74) Representative: **Lewin, John Harvey**
**Takeda Euro IP Department,**
**11-12 Charles II Street**
**London SW1Y 4QU (GB)**

(54) **PREVENTIVE/REMEDY FOR DISEASES IN UPPER DIGESTIVE TRACT**

(57) A compound or its salts that inhibit the activity of the polypeptide or receptor of the present invention and the antibody of the present invention as well as the antisense DNA of the present invention are useful as, e.g., gastric acid secretion inhibitors, mucosa protectants, mineral absorption promoters, etc., which are less toxic and safe, and can be used as, e.g., agents for preventing/treating upper digestive tract disorders, antibacterial agents to Helicobacter pylori, etc. A compound or its salts that promote the activity of the polypeptide or receptor of the present invention, the polypeptide or receptor of the present invention and the DNA of the present invention can be used as, e.g., gastric acid secretion promoters and can be used as, e.g., agents for preventing/treating dyspepsia, bone metabolism disorders, anemia, etc. A compound or its salts that promote the activity of the polypeptide or receptor of the present invention, the polypeptide of the present invention, etc. can also be used as test agents for gastric secretory function. Furthermore, the polypeptide or receptor of the present invention and the DNA of the present invention are also useful for screening the agents for the prevention/treatment described above.

FIG. 1

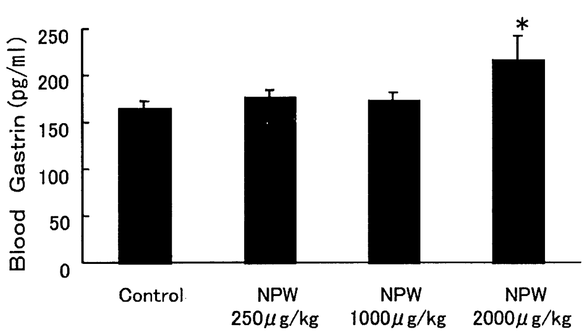


EP 1 602 380 A1

SEQUENCE LISTING

<110> Takeda Pharmaceutical Company Limited

<120> Agents for preventing and/or treating upper digestive tract disorders

<130> G05-0050

<150> JP2003-68963
<151> 2003-03-13

<160> 80

<210> 1
<211> 23
<212> PRT
<213> Homo sapiens

<400> 1
Trp Tyr Lys His Val Ala Ser Pro Arg Tyr His Thr Val Gly Arg Ala
1               5                   10                  15
Ala Gly Leu Leu Met Gly Leu
            20

<210> 2
<211> 30
<212> PRT
<213> Homo sapiens

<400> 2
Trp Tyr Lys His Val Ala Ser Pro Arg Tyr His Thr Val Gly Arg Ala
1               5                   10                  15
Ala Gly Leu Leu Met Gly Leu Arg Arg Ser Pro Tyr Leu Trp
            20                  25                  30

<210> 3

<211> 69
<212> DNA
<213> Homo sapiens

<400> 3
tggtacaagc acgtggcgag tccccgctac cacacggtgg gccgcgccgc tggcctgctc 60
atggggctg 69

<210> 4
<211> 90
<212> DNA
<213> Homo sapiens

<400> 4
tggtacaagc acgtggcgag tccccgctac cacacggtgg gccgcgccgc tggcctgctc 60
atggggctgc gtcgctcacc ctatctgtgg 90

<210> 5
<211> 375
<212> DNA
<213> Homo sapiens

<400> 5
aactccactg cgcgcccaaa cccagccgag ccggttcgtg gcccgccccg ccgggcggcc 60
gtcgacgcga gcgccctggc gtggcgccca ggggagcggg gggctcccgc gagccggccg 120
cggctggcac tgctgctgct tctgctcctg ctgccgctgc cctccggcgc gtggtacaag 180
cacgtggcga gtccccgcta ccacacggtg ggccgcgccg ctggcctgct catggggctg 240
cgtcgctcac cctatctgtg gcgccgcgcg ctgcgcgcgg ccgccgggcc cctggccagg 300
gacaccctct cccccgaacc cgcagcccgc gaggctcctc tcctgctgcc ctcgtgggtt 360
caggagctgt gggag 375

<210> 6
<211> 125
<212> PRT
<213> Homo sapiens

<400> 6
Asn Ser Thr Ala Arg Pro Asn Pro Ala Glu Pro Val Arg Gly Pro Pro
1 5 10 15
Arg Arg Ala Ala Val Asp Ala Ser Ala Leu Ala Trp Arg Pro Gly Glu
20 25 30
Arg Gly Ala Pro Ala Ser Arg Pro Arg Leu Ala Leu Leu Leu Leu Leu
35 40 45
Leu Leu Leu Pro Leu Pro Ser Gly Ala Trp Tyr Lys His Val Ala Ser
50 55 60
Pro Arg Tyr His Thr Val Gly Arg Ala Ala Gly Leu Leu Met Gly Leu
65 70 75 80
Arg Arg Ser Pro Tyr Leu Trp Arg Arg Ala Leu Arg Ala Ala Ala Gly
85 90 95
Pro Leu Ala Arg Asp Thr Leu Ser Pro Glu Pro Ala Ala Arg Glu Ala
100 105 110
Pro Leu Leu Leu Pro Ser Trp Val Gln Glu Leu Trp Glu
115 120 125

<210> 7
<211> 29
<212> PRT
<213> Homo sapiens

<400> 7
Trp Tyr Lys His Val Ala Ser Pro Arg Tyr His Thr Val Gly Arg Ala
1 5 10 15
Ala Gly Leu Leu Met Gly Leu Arg Arg Ser Pro Tyr Leu
20 25

<210> 8
<211> 28
<212> PRT
<213> Homo sapiens

<400> 8
Trp Tyr Lys His Val Ala Ser Pro Arg Tyr His Thr Val Gly Arg Ala

1 5 10 15
Ala Gly Leu Leu Met Gly Leu Arg Arg Ser Pro Tyr
20 25

<210> 9
<211> 27
<212> PRT
<213> Homo sapiens

<400> 9
Trp Tyr Lys His Val Ala Ser Pro Arg Tyr His Thr Val Gly Arg Ala
1 5 10 15
Ala Gly Leu Leu Met Gly Leu Arg Arg Ser Pro
20 25

<210> 10
<211> 26
<212> PRT
<213> Homo sapiens

<400> 10
Trp Tyr Lys His Val Ala Ser Pro Arg Tyr His Thr Val Gly Arg Ala
1 5 10 15
Ala Gly Leu Leu Met Gly Leu Arg Arg Ser
20 25

<210> 11
<211> 25
<212> PRT
<213> Homo sapiens

<400> 11
Trp Tyr Lys His Val Ala Ser Pro Arg Tyr His Thr Val Gly Arg Ala
1 5 10 15
Ala Gly Leu Leu Met Gly Leu Arg Arg
20 25

<210> 12
<211> 24
<212> PRT
<213> Homo sapiens

<400> 12
Trp Tyr Lys His Val Ala Ser Pro Arg Tyr His Thr Val Gly Arg Ala
1               5                   10                  15
Ala Gly Leu Leu Met Gly Leu Arg
            20

<210> 13
<211> 87
<212> DNA
<213> Homo sapiens

<400> 13
tggtacaagc acgtggcgag tccccgctac cacacggtgg gccgcgccgc tggcctgctc 60
atggggctgc gtcgctcacc ctatctg 87

<210> 14
<211> 84
<212> DNA
<213> Homo sapiens

<400> 14
tggtacaagc acgtggcgag tccccgctac cacacggtgg gccgcgccgc tggcctgctc 60
atggggctgc gtcgctcacc ctat 84

<210> 15
<211> 81
<212> DNA
<213> Homo sapiens

<400> 15

tggtacaagc acgtggcgag tccccgctac cacacggtgg gccgcgccgc tggcctgctc 60
atggggctgc gtcgctcacc c 81

<210> 16
<211> 78
<212> DNA
<213> Homo sapiens

<400> 16
tggtacaagc acgtggcgag tccccgctac cacacggtgg gccgcgccgc tggcctgctc 60
atggggctgc gtcgctca 78

<210> 17
<211> 75
<212> DNA
<213> Homo sapiens

<400> 17
tggtacaagc acgtggcgag tccccgctac cacacggtgg gccgcgccgc tggcctgctc 60
atggggctgc gtcgc 75

<210> 18
<211> 72
<212> DNA
<213> Homo sapiens

<400> 18
tggtacaagc acgtggcgag tccccgctac cacacggtgg gccgcgccgc tggcctgctc 60
atggggctgc gt 72

<210> 19
<211> 719
<212> DNA
<213> Homo sapiens

<400> 19

ggcggggcca ccgagcggtt atagctgggc ctgcagggga cccacggctc gcctccagcc 60
tcctgcgctc cggtacctgg gcgtcccaac tccactgcgc gcccaaaccc agccgagccg 120
gttcgtggcc cgccccgccg ggcggccgtc gacgcgagcg ccctggcgtg gcgcccaggg 180
gagcgggggg ctcccgcgag ccggccgcgg ctggcactgc tgctgcttct gctcctgctg 240
ccgctgccct ccggcgcgtg gtacaagcac gtggcgagtc cccgctacca cacggtgggc 300
cgcgccgctg gcctgctcat ggggctgcgt cgctcaccct atctgtggcg ccgcgcgctg 360
cgcgcggccg ccgggcccct ggccagggac accctctccc ccgaacccgc agcccgcgag 420
gctcctctcc tgctgccctc gtgggttcag gagctgtggg agacgcgacg caggagctcc 480
caggcaggga tccccgtccg tgcgccccgg agcccgcgcg ccccagagcc tgcgctggaa 540
ccggagtccc tggacttcag cggagctggc cagagacttc ggagagacgt ctcccgccca 600
gcggtggacc ccgcagcaaa ccgccttggc ctgccctgcc tggcccccgg accgttctga 660
cagcgtcccc cgcccgcccg tggcgcctcc gcgcctgacc caggaggagt ggccgcgcg 719

<210> 20
<211> 165
<212> PRT
<213> Homo sapiens

<400> 20
Leu Ala Trp Arg Pro Gly Glu Arg Gly Ala Pro Ala Ser Arg Pro Arg
1 5 10 15
Leu Ala Leu Leu Leu Leu Leu Leu Leu Leu Pro Leu Pro Ser Gly Ala
20 25 30
Trp Tyr Lys His Val Ala Ser Pro Arg Tyr His Thr Val Gly Arg Ala
35 40 45
Ala Gly Leu Leu Met Gly Leu Arg Arg Ser Pro Tyr Leu Trp Arg Arg
50 55 60
Ala Leu Arg Ala Ala Ala Gly Pro Leu Ala Arg Asp Thr Leu Ser Pro
65 70 75 80
Glu Pro Ala Ala Arg Glu Ala Pro Leu Leu Leu Pro Ser Trp Val Gln
85 90 95
Glu Leu Trp Glu Thr Arg Arg Arg Ser Ser Gln Ala Gly Ile Pro Val
100 105 110
Arg Ala Pro Arg Ser Pro Arg Ala Pro Glu Pro Ala Leu Glu Pro Glu
115 120 125
Ser Leu Asp Phe Ser Gly Ala Gly Gln Arg Leu Arg Arg Asp Val Ser

130 135 140
Arg Pro Ala Val Asp Pro Ala Ala Asn Arg Leu Gly Leu Pro Cys Leu
145 150 155 160
Ala Pro Gly Pro Phe
165

<210> 21
<211> 17
<212> PRT
<213> Porcine

<400> 21
Trp Tyr Lys His Thr Ala Ser Pro Arg Tyr His Thr Val Gly Arg Ala
1 5 10 15
Ala

<210> 22
<211> 565
<212> DNA
<213> Porcine

<400> 22
cctccggagc cagttcctgg tccgccccgc cgggagccgt cagcatgaac ccccgggcac 60
gcggcatggg agcgcggggc ccgggaccgg gggccactgc gaggcgccgg ctgctggcat 120
tgctgttact gctgctgctg ctgccgctgc ccgcccgtgc ctggtacaag cacacggcga 180
gtccccgcta ccacacggtg ggccgcgccg cgggcctgct catggggctg cgccgctcgc 240
cctacatgtg gcgccgcgcg ctgcgcccgg cggccgggcc cctggcctgg gacactttcg 300
gccaggacgt gccccctcgg ggaccctccg ccaggaacgc cctctctccg gggcccgccc 360
ctcgcgacgc tccgctgctt ccccccgggg ttcagacact gtggcaggtg cgacgcggaa 420
gcttccgctc cgggatcccg gtcagtgcgc cccgcagccc gcgcgcccgg gggtccgagc 480
cgcaaccgga attgggcgcc tcttcctgga cctcggcgga gtagaccaga gccttcggag 540
agtcttcagc tcagcggtgg tctgc 565

<210> 23
<211> 159
<212> PRT

<213> Porcine

<400> 23
Met Asn Pro Arg Ala Arg Gly Met Gly Ala Arg Gly Pro Gly Pro Gly
1 5 10 15
Ala Thr Ala Arg Arg Arg Leu Leu Ala Leu Leu Leu Leu Leu Leu Leu
20 25 30
Leu Pro Leu Pro Ala Arg Ala Trp Tyr Lys His Thr Ala Ser Pro Arg
35 40 45
Tyr His Thr Val Gly Arg Ala Ala Gly Leu Leu Met Gly Leu Arg Arg
50 55 60
Ser Pro Tyr Met Trp Arg Arg Ala Leu Arg Pro Ala Ala Gly Pro Leu
65 70 75 80
Ala Trp Asp Thr Phe Gly Gln Asp Val Pro Pro Arg Gly Pro Ser Ala
85 90 95
Arg Asn Ala Leu Ser Pro Gly Pro Ala Pro Arg Asp Ala Pro Leu Leu
100 105 110
Pro Pro Gly Val Gln Thr Leu Trp Gln Val Arg Arg Gly Ser Phe Arg
115 120 125
Ser Gly Ile Pro Val Ser Ala Pro Arg Ser Pro Arg Ala Arg Gly Ser
130 135 140
Glu Pro Gln Pro Glu Leu Gly Ala Ser Ser Trp Thr Ser Ala Glu
145 150 155

<210> 24
<211> 23
<212> PRT
<213> Porcine

<400> 24
Trp Tyr Lys His Thr Ala Ser Pro Arg Tyr His Thr Val Gly Arg Ala
1 5 10 15
Ala Gly Leu Leu Met Gly Leu
20

<210> 25

<211> 30
<212> PRT
<213> Porcine

<400> 25
Trp Tyr Lys His Thr Ala Ser Pro Arg Tyr His Thr Val Gly Arg Ala
1 5 10 15
Ala Gly Leu Leu Met Gly Leu Arg Arg Ser Pro Tyr Met Trp
20 25 30

<210> 26
<211> 69
<212> DNA
<213> Porcine

<400> 26
tggtacaagc acacggcgag tccccgctac cacacggtgg gccgcgccgc gggcctgctc 60
atggggctg 69

<210> 27
<211> 90
<212> DNA
<213> Porcine

<400> 27
tggtacaagc acacggcgag tccccgctac cacacggtgg gccgcgccgc gggcctgctc 60
atggggctgc gccgctcgcc ctacatgtgg 90

<210> 28
<211> 684
<212> DNA
<213> Rattus norvegicus

<400> 28
tgtagtcgca ccaactgact agtctcttcc atcctccgga gctccgacgt tctcggggac 60
ataaaccctg ttcttgtcct aacccgccaa ggggccatgg acttgagcgc gctggcgtcg 120

agcagagaag tacggggccc tgggcccggg gctccggtga accggcccct gctaccgcta 180
ctgctgcttc tgctcttgct acctctgccc gccagcgcct ggtacaagca cgtggcgagc 240
cctcgctatc acacagtggg tcgtgcctcc gggctgctca tggggctgcg ccgctcgccc 300
tacctgtggc gccgtgcctt gggtggggcc gctggaccgc tcgtggggct cccgggacag 360
atggcccgca gcgctctcct gcttccttcc cccgggcagg agctgtggga ggtacgaagc 420
aggagttcac cggcaggact tcccgtgcat gcaacccgga gtctgcggga cctggaggga 480
gccggccaac ctgagcagtc gctaagcttt cagtcctgga cttcagcaga gcccgctgct 540
agagccttcg gtgagacgct tcgtgcccag ccatggttcc tgcagcaaat catctttgcc 600
gatcctgtca ggctcgacga ccgtctcaag aaccgatggc gcccccgtgc ttgacctaag 660
caggagcaca gcttgtagct ccag 684

<210> 29
<211> 185
<212> PRT
<213> Rattus norvegicus

<400> 29
Met Asp Leu Ser Ala Leu Ala Ser Ser Arg Glu Val Arg Gly Pro Gly
1 5 10 15
Pro Gly Ala Pro Val Asn Arg Pro Leu Leu Pro Leu Leu Leu Leu Leu
20 25 30
Leu Leu Leu Pro Leu Pro Ala Ser Ala Trp Tyr Lys His Val Ala Ser
35 40 45
Pro Arg Tyr His Thr Val Gly Arg Ala Ser Gly Leu Leu Met Gly Leu
50 55 60
Arg Arg Ser Pro Tyr Leu Trp Arg Arg Ala Leu Gly Gly Ala Ala Gly
65 70 75 80
Pro Leu Val Gly Leu Pro Gly Gln Met Ala Arg Ser Ala Leu Leu Leu
85 90 95
Pro Ser Pro Gly Gln Glu Leu Trp Glu Val Arg Ser Arg Ser Ser Pro
100 105 110
Ala Gly Leu Pro Val His Ala Thr Arg Ser Leu Arg Asp Leu Glu Gly
115 120 125
Ala Gly Gln Pro Glu Gln Ser Leu Ser Phe Gln Ser Trp Thr Ser Ala
130 135 140
Glu Pro Ala Ala Arg Ala Phe Gly Glu Thr Leu Arg Ala Gln Pro Trp

145 150 155 160
Phe Leu Gln Gln Ile Ile Phe Ala Asp Pro Val Arg Leu Asp Asp Arg
165 170 175
Leu Lys Asn Arg Trp Arg Pro Arg Ala
180 185

<210> 30
<211> 23
<212> PRT
<213> Rattus norvegicus

<400> 30
Trp Tyr Lys His Val Ala Ser Pro Arg Tyr His Thr Val Gly Arg Ala
1 5 10 15
Ser Gly Leu Leu Met Gly Leu
20

<210> 31
<211> 30
<212> PRT
<213> Rattus norvegicus

<400> 31
Trp Tyr Lys His Val Ala Ser Pro Arg Tyr His Thr Val Gly Arg Ala
1 5 10 15
Ser Gly Leu Leu Met Gly Leu Arg Arg Ser Pro Tyr Leu Trp
20 25 30

<210> 32
<211> 69
<212> DNA
<213> Rattus norvegicus

<400> 32
tggtacaagc acgtggcgag ccctcgctat cacacagtgg gtcgtgcctc cgggctgctc 60
atggggctg 69

<210> 33
<211> 90
<212> DNA
<213> Rattus norvegicus

<400> 33
tggtacaagc acgtggcgag ccctcgctat cacacagtgg gtcgtgcctc cgggctgctc 60
atggggctgc gccgctcgcc ctacctgtgg 90

<210> 34
<211> 659
<212> DNA
<213> Mus musculus

<400> 34
tgactggtct ccatcctctg gagctccgac gtgctcgttc tcggagacat aaacccagtt 60
cttgtcctaa ccctccaagg ggcaattgac gtgagcgcgc tggcgtctaa cagagaagta 120
cggggccctg ggcccgggac tcccaggaac cggcccctgc tgcccctgct gctgcttctg 180
ctcttgctac cgctgcccgc cagcgcctgg tataagcacg tggcgagtcc ccgctatcac 240
acagtgggtc gtgcctccgg gctgctcatg gggctgcgcc gctcgcccta ccagtggcgc 300
cgtgccctgg gcggggctgc tggacccctc tcccggctcc caggaccggt cgcccgcggc 360
gctctcctgc ttccttcctc agggcaggag ctgtgggagg tacgaagcag gagctcacct 420
gcagggcttc ccgtccatgc accctggagt ccgcgggacc tggagggagt ccgccaaccg 480
gagcagtcgc taagccttca ctcctggatc tcagaggagc ccgctgctag agccttcgga 540
gagacgcttc gtgcccagcc atggttcctg cagcaagtca tctttgccga tcctgtcagg 600
cccaagaacc gatggcgccc ccatgcttga cctaggcagg agcacagctt gaagctcca 659

<210> 35
<211> 176
<212> PRT
<213> Mus musculus

<400> 35
Leu Ala Ser Asn Arg Glu Val Arg Gly Pro Gly Pro Gly Thr Pro Arg
1 5 10 15

Asn Arg Pro Leu Leu Pro Leu Leu Leu Leu Leu Leu Leu Leu Pro Leu
20 25 30
Pro Ala Ser Ala Trp Tyr Lys His Val Ala Ser Pro Arg Tyr His Thr
35 40 45
Val Gly Arg Ala Ser Gly Leu Leu Met Gly Leu Arg Arg Ser Pro Tyr
50 55 60
Gln Trp Arg Arg Ala Leu Gly Gly Ala Ala Gly Pro Leu Ser Arg Leu
65 70 75 80
Pro Gly Pro Val Ala Arg Gly Ala Leu Leu Leu Pro Ser Ser Gly Gln
85 90 95
Glu Leu Trp Glu Val Arg Ser Arg Ser Ser Pro Ala Gly Leu Pro Val
100 105 110
His Ala Pro Trp Ser Pro Arg Asp Leu Glu Gly Val Arg Gln Pro Glu
115 120 125
Gln Ser Leu Ser Leu His Ser Trp Ile Ser Glu Glu Pro Ala Ala Arg
130 135 140
Ala Phe Gly Glu Thr Leu Arg Ala Gln Pro Trp Phe Leu Gln Gln Val
145 150 155 160
Ile Phe Ala Asp Pro Val Arg Pro Lys Asn Arg Trp Arg Pro His Ala
165 170 175

<210> 36
<211> 23
<212> PRT
<213> Mus musculus

<400> 36
Trp Tyr Lys His Val Ala Ser Pro Arg Tyr His Thr Val Gly Arg Ala
1 5 10 15
Ser Gly Leu Leu Met Gly Leu
20

<210> 37
<211> 30
<212> PRT
<213> Mus musculus

<400> 37
Trp Tyr Lys His Val Ala Ser Pro Arg Tyr His Thr Val Gly Arg Ala
1               5                   10                  15
Ser Gly Leu Leu Met Gly Leu Arg Arg Ser Pro Tyr Gln Trp
            20                  25                  30

<210> 38
<211> 69
<212> DNA
<213> Mus musculus

<400> 38
tggtataagc acgtggcgag tccccgctat cacacagtgg gtcgtgcctc cgggctgctc    60
atggggctg    69

<210> 39
<211> 90
<212> DNA
<213> Mus musculus

<400> 39
tggtataagc acgtggcgag tccccgctat cacacagtgg gtcgtgcctc cgggctgctc    60
atggggctgc gccgctcgcc ctaccagtgg    90

<210> 40
<211> 23
<212> PRT
<213> Artificial Sequence

<220>
<221> VARIANT
<222> 21
<223> Xaa on the 21st position means Met(O)

<400> 40

Trp Tyr Lys His Val Ala Ser Pro Arg Tyr His Thr Val Gly Arg Ala
1 5 10 15
Ala Gly Leu Leu Xaa Gly Leu
20

<210> 41
<211> 22
<212> PRT
<213> Homo sapiens

<400> 41
Trp Tyr Lys His Val Ala Ser Pro Arg Tyr His Thr Val Gly Arg Ala
1 5 10 15
Ala Gly Leu Leu Met Gly
20

<210> 42
<211> 21
<212> PRT
<213> Homo sapiens

<400> 42
Trp Tyr Lys His Val Ala Ser Pro Arg Tyr His Thr Val Gly Arg Ala
1 5 10 15
Ala Gly Leu Leu Met
20

<210> 43
<211> 20
<212> PRT
<213> Homo sapiens

<400> 43
Trp Tyr Lys His Val Ala Ser Pro Arg Tyr His Thr Val Gly Arg Ala
1 5 10 15
Ala Gly Leu Leu

20

<210> 44
<211> 19
<212> PRT
<213> Homo sapiens

<400> 44
Trp Tyr Lys His Val Ala Ser Pro Arg Tyr His Thr Val Gly Arg Ala
1 5 10 15
Ala Gly Leu

<210> 45
<211> 18
<212> PRT
<213> Homo sapiens

<400> 45
Trp Tyr Lys His Val Ala Ser Pro Arg Tyr His Thr Val Gly Arg Ala
1 5 10 15
Ala Gly

<210> 46
<211> 17
<212> PRT
<213> Homo sapiens

<400> 46
Trp Tyr Lys His Val Ala Ser Pro Arg Tyr His Thr Val Gly Arg Ala
1 5 10 15
Ala

<210> 47
<211> 16
<212> PRT
<213> Homo sapiens

<400> 47
Trp Tyr Lys His Val Ala Ser Pro Arg Tyr His Thr Val Gly Arg Ala
1 5 10 15

<210> 48
<211> 23
<212> PRT
<213> Artificial Sequence

<220>
<221> VARIANT
<222> 21
<223> Xaa on the 21st position means Met(O)

<400> 48
Trp Tyr Lys His Thr Ala Ser Pro Arg Tyr His Thr Val Gly Arg Ala
1 5 10 15
Ala Gly Leu Leu Xaa Gly Leu
20

<210> 49
<211> 23
<212> PRT
<213> Artificial Sequence

<220>
<221> VARIANT
<222> 21
<223> Xaa on the 21st position means Met(O)

<400> 49
Trp Tyr Lys His Val Ala Ser Pro Arg Tyr His Thr Val Gly Arg Ala
1 5 10 15
Ser Gly Leu Leu Xaa Gly Leu
20

<210> 50
<211> 23
<212> PRT
<213> Artificial Sequence

<220>
<221> VARIANT
<222> 21
<223> Xaa on the 1st position means Fmoc Trp

<400> 50
Xaa Tyr Lys His Val Ala Ser Pro Arg Tyr His Thr Val Gly Arg Ala
1 5 10 15
Ala Gly Leu Leu Met Gly Leu
20

<210> 51
<211> 23
<212> PRT
<213> Artificial Sequence

<220>
<221> VARIANT
<222> 1
<223> Xaa on the 1st position means Ac Trp

<400> 51
Xaa Tyr Lys His Val Ala Ser Pro Arg Tyr His Thr Val Gly Arg Ala
1 5 10 15
Ala Gly Leu Leu Met Gly Leu
20

<210> 52
<211> 22
<212> PRT

<213> Homo sapiens

<400> 52
Tyr Lys His Val Ala Ser Pro Arg Tyr His Thr Val Gly Arg Ala Ala
1 5 10 15
Gly Leu Leu Met Gly Leu
20

<210> 53
<211> 20
<212> PRT
<213> Homo sapiens

<400> 53
His Val Ala Ser Pro Arg Tyr His Thr Val Gly Arg Ala Ala Gly Leu
1 5 10 15
Leu Met Gly Leu
20

<210> 54
<211> 15
<212> PRT
<213> Homo sapiens

<400> 54
Arg Tyr His Thr Val Gly Arg Ala Ala Gly Leu Leu Met Gly Leu
1 5 10 15

<210> 55
<211> 9
<212> PRT
<213> Homo sapiens

<400> 55
Arg Ala Ala Gly Leu Leu Met Gly Leu
1 5

<210> 56
<211> 22
<212> PRT
<213> Artificial Sequence

<220>
<221> VARIANT
<222> 1
<223> Xaa on the 1st position means Ac Tyr

<400> 56
Xaa Lys His Val Ala Ser Pro Arg Tyr His Thr Val Gly Arg Ala Ala
1 5 10 15
Gly Leu Leu Met Gly Leu
20

<210> 57
<211> 23
<212> PRT
<213> Artificial Sequence

<220>
<221> VARIANT
<222> 1
<223> Xaa on the 1st position means DTrp

<400> 57
Xaa Tyr Lys His Val Ala Ser Pro Arg Tyr His Thr Val Gly Arg Ala
1 5 10 15
Ala Gly Leu Leu Met Gly Leu
20

<210> 58
<211> 22
<212> PRT

<213> Artificial Sequence

<220>
<221> VARIANT
<222> 1
<223> Xaa on the 1st position means 3-Indolepropanoyl Tyr

<400> 58
Xaa Lys His Val Ala Ser Pro Arg Tyr His Thr Val Gly Arg Ala Ala
1 5 10 15
Gly Leu Leu Met Gly Leu
20

<210> 59
<211> 66
<212> DNA
<213> Homo sapiens

<400> 59
tggtacaagc acgtggcgag tccccgctac cacacggtgg gccgcgccgc tggcctgctc 60
atgggg 66

<210> 60
<211> 63
<212> DNA
<213> Homo sapiens

<400> 60
tggtacaagc acgtggcgag tccccgctac cacacggtgg gccgcgccgc tggcctgctc 60
atg 63

<210> 61
<211> 60
<212> DNA
<213> Homo sapiens

<400> 61
tggtacaagc acgtggcgag tccccgctac cacacggtgg gccgcgccgc tggcctgctc 60

<210> 62
<211> 57
<212> DNA
<213> Homo sapiens

<400> 62
tggtacaagc acgtggcgag tccccgctac cacacggtgg gccgcgccgc tggcctg 57

<210> 63
<211> 54
<212> DNA
<213> Homo sapiens

<400> 63
tggtacaagc acgtggcgag tccccgctac cacacggtgg gccgcgccgc tggc 54

<210> 64
<211> 51
<212> DNA
<213> Homo sapiens

<400> 64
tggtacaagc acgtggcgag tccccgctac cacacggtgg gccgcgccgc t 51

<210> 65
<211> 48
<212> DNA
<213> Homo sapiens

<400> 65
tggtacaagc acgtggcgag tccccgctac cacacggtgg gccgcgcc 48

<210> 66

<211> 66
<212> DNA
<213> Homo sapiens

<400> 66
tacaagcacg tggcgagtcc ccgctaccac acggtgggcc gcgccgctgg cctgctcatg 60
gggctg 66

<210> 67
<211> 60
<212> DNA
<213> Homo sapiens

<400> 67
cacgtggcga gtccccgcta ccacacggtg ggccgcgccg ctggcctgct catggggctg 60

<210> 68
<211> 45
<212> DNA
<213> Homo sapiens

<400> 68
cgctaccaca cggtgggccg cgccgctggc ctgctcatgg ggctg 45

<210> 69
<211> 27
<212> DNA
<213> Homo sapiens

<400> 69
cgcgccgctg gcctgctcat ggggctg 27

<210> 70
<211> 51
<212> DNA
<213> Porcine

<400> 70
tggtacaagc acacggcgag tccccgctac cacacggtgg gccgcgccgc g 51

<210> 71
<211> 20
<212> PRT
<213> Artificial Sequence

<220>

<223> Designed peptide

<400> 71
Trp Phe Lys His Val Ala Ser Pro Arg Tyr His Thr Val Gly Arg Ala
1 5 10 15
Ala Gly Leu Leu
20

<210> 72
<211> 60
<212> DNA
<213> Artificial Sequence

<220>

<223> Designed polynucleotide

<400> 72
tggttcaagc acgtggcgag tccccgctac cacacggtgg gccgcgccgc tggcctgctc 60

<210> 73
<211> 333
<212> PRT
<213> Homo sapiens

<400> 73

Met Gln Ala Ala Gly His Pro Glu Pro Leu Asp Ser Arg Gly Ser Phe
1 5 10 15

Ser Leu Pro Thr Met Gly Ala Asn Val Ser Gln Asp Asn Gly Thr Gly
20 25 30

His Asn Ala Thr Phe Ser Glu Pro Leu Pro Phe Leu Tyr Val Leu Leu
35 40 45

Pro Ala Val Tyr Ser Gly Ile Cys Ala Val Gly Leu Thr Gly Asn Thr
50 55 60

Ala Val Ile Leu Val Ile Leu Arg Ala Pro Lys Met Lys Thr Val Thr
65 70 75 80

Asn Val Phe Ile Leu Asn Leu Ala Val Ala Asp Gly Leu Phe Thr Leu
85 90 95

Val Leu Pro Val Asn Ile Ala Glu His Leu Leu Gln Tyr Trp Pro Phe
100 105 110

Gly Glu Leu Leu Cys Lys Leu Val Leu Ala Val Asp His Tyr Asn Ile
115 120 125

Phe Ser Ser Ile Tyr Phe Leu Ala Val Met Ser Val Asp Arg Tyr Leu
130 135 140

Val Val Leu Ala Thr Val Arg Ser Arg His Met Pro Trp Arg Thr Tyr
145 150 155 160

Arg Gly Ala Lys Val Ala Ser Leu Cys Val Trp Leu Gly Val Thr Val
165 170 175

Leu Val Leu Pro Phe Phe Ser Phe Ala Gly Val Tyr Ser Asn Glu Leu
180 185 190

Gln Val Pro Ser Cys Gly Leu Ser Phe Pro Trp Pro Glu Gln Val Trp
195 200 205

Phe Lys Ala Ser Arg Val Tyr Thr Leu Val Leu Gly Phe Val Leu Pro
210 215 220

Val Cys Thr Ile Cys Val Leu Tyr Thr Asp Leu Leu Arg Arg Leu Arg
225 230 235 240

Ala Val Arg Leu Arg Ser Gly Ala Lys Ala Leu Gly Lys Ala Arg Arg
245 250 255

Lys Val Thr Val Leu Val Leu Val Val Leu Ala Val Cys Leu Leu Cys
260 265 270

Trp Thr Pro Phe His Leu Ala Ser Val Val Ala Leu Thr Thr Asp Leu

275 280 285
Pro Gln Thr Pro Leu Val Ile Ser Met Ser Tyr Val Ile Thr Ser Leu
290 295 300
Ser Tyr Ala Asn Ser Cys Leu Asn Pro Phe Leu Tyr Ala Phe Leu Asp
305 310 315 320
Asp Asn Phe Arg Lys Asn Phe Arg Ser Ile Leu Arg Cys
325 330

<210> 74
<211> 999
<212> DNA
<213> Homo sapiens

<400> 74
atgcaggccg ctgggcaccc agagcccctt gacagcaggg gctccttctc cctccccacg 60
atgggtgcca acgtctctca ggacaatggc actggccaca atgccacctt ctccgagcca 120
ctgccgttcc tctatgtgct cctgcccgcc gtgtactccg ggatctgtgc tgtggggctg 180
actggcaaca cggccgtcat ccttgtaatc ctaagggcgc ccaagatgaa gacggtgacc 240
aacgtgttca tcctgaacct ggccgtcgcc gacgggctct tcacgctggt actgcccgtc 300
aacatcgcgg agcacctgct gcagtactgg cccttcgggg agctgctctg caagctggtg 360
ctggccgtcg accactacaa catcttctcc agcatctact tcctagccgt gatgagcgtg 420
gaccgatacc tggtggtgct ggccaccgtg aggtcccgcc acatgccctg gcgcacctac 480
cggggggcga aggtcgccag cctgtgtgtc tggctgggcg tcacggtcct ggttctgccc 540
ttcttctctt tcgctggcgt ctacagcaac gagctgcagg tcccaagctg tgggctgagc 600
ttcccgtggc ccgagcgggt ctggttcaag gccagccgtg tctacacttt ggtcctgggc 660
ttcgtgctgc ccgtgtgcac catctgtgtg ctctacacag acctcctgcg caggctgcgg 720
gccgtgcggc tccgctctgg agccaaggct ctaggcaagg ccaggcggaa ggtgaccgtc 780
ctggtcctcg tcgtgctggc cgtgtgcctc ctctgctgga cgcccttcca cctggcctct 840
gtcgtggccc tgaccacgga cctgccccag accccactgg tcatcagtat gtcctacgtc 900
atcaccagcc tcacgtacgc caactcgtgc ctgaacccct tcctctacgc ctttctagat 960
gacaacttcc ggaagaactt ccgcagcata ttgcggtgc 999

<210> 75
<211> 329
<212> PRT
<213> Rattus norvegicus

<400> 75

Met His Asn Leu Ser Leu Phe Glu Pro Gly Arg Gly Asn Val Ser Cys
5 10 15
Gly Gly Pro Phe Leu Gly Cys Pro Asn Glu Ser Asn Pro Ala Pro Leu
20 25 30
Pro Leu Pro Gln Pro Leu Ala Val Ala Val Pro Val Val Tyr Gly Val
35 40 45
Ile Cys Ala Val Gly Leu Ala Gly Asn Ser Ala Val Leu Tyr Val Leu
50 55 60
Leu Arg Thr Pro Arg Met Lys Thr Val Thr Asn Val Phe Ile Leu Asn
65 70 75 80
Leu Ala Ile Ala Asp Glu Leu Phe Thr Leu Val Leu Pro Ile Asn Ile
85 90 95
Ala Asp Phe Leu Leu Arg Arg Trp Pro Phe Gly Glu Val Met Cys Lys
100 105 110
Leu Ile Val Ala Val Asp Gln Tyr Asn Thr Phe Ser Ser Leu Tyr Phe
115 120 125
Leu Ala Val Met Ser Ala Asp Arg Tyr Leu Val Val Leu Ala Thr Ala
130 135 140
Glu Ser Arg Arg Val Ser Gly Arg Thr Tyr Gly Ala Ala Arg Ala Val
145 150 155 160
Ser Leu Ala Val Trp Ala Leu Val Thr Leu Val Val Leu Pro Phe Ala
165 170 175
Val Phe Ala Arg Leu Asp Glu Glu Gln Gly Arg Arg Gln Cys Val Leu
180 185 190
Val Phe Pro Gln Pro Glu Ala Phe Trp Trp Arg Ala Ser Arg Leu Tyr
195 200 205
Thr Leu Val Leu Gly Phe Ala Ile Pro Val Ser Thr Ile Cys Ala Leu
210 215 220
Tyr Ile Thr Leu Leu Cys Arg Leu Arg Ala Ile Gln Leu Asp Ser His
225 230 235 240
Ala Lys Ala Leu Asp Arg Ala Lys Lys Arg Val Thr Leu Leu Val Val
245 250 255
Ala Ile Leu Ala Val Cys Leu Leu Cys Trp Thr Pro Tyr His Leu Ser
260 265 270

Thr Ile Val Ala Leu Thr Thr Asp Leu Pro Gln Thr Pro Leu Val Ile
275 280 285
Gly Ile Ser Tyr Phe Ile Thr Ser Leu Ser Tyr Ala Asn Ser Cys Leu
290 295 300
Asn Pro Phe Leu Tyr Ala Phe Leu Asp Asp Ser Phe Arg Arg Ser Leu
305 310 315 320
Arg Gln Leu Val Ser Cys Arg Thr Ala
325

<210> 76
<211> 987
<212> DNA
<213> Rattus norvegicus

<400> 76
atgcacaact tgtcgctctt cgagcctggc aggggcaatg tgtcttgcgg cggcccattt 60
ttgggctgtc ctaacgagtc gaacccagcg cctctgccac tgccgcagcc tctggcggta 120
gcagtgcctg tggtctacgg ggtgatctgc gcggtgggac tggcgggcaa ctccgcggtg 180
ctgtacgtac tgctgcgcac gccgcgcatg aagactgtta ccaacgtgtt cattctcaac 240
ctggctatcg cggacgagct cttcaccctc gtgctgccca tcaacatcgc ggacttcctg 300
ctgaggcgct ggcccttcgg ggaagtcatg tgcaagctca tcgtggctgt cgaccagtac 360
aacactttct ctagcctcta cttcctcgcc gtcatgagcg cagaccgcta cctggttgtc 420
ctggccacag ccgagtcgcg ccgggtgtcc gggcgcactt atggtgcagc gcgggctgtc 480
agtctggcgg tgtgggcgct ggtgacattg gtcgtgctgc cttttgcggt attcgcccgg 540
ctggacgaag agcagggtcg gcgtcagtgc gtgctggtct tcccgcagcc tgaggccttc 600
tggtggcgcg ccagccgtct gtacactcta gtgttgggct tcgccatccc ggtgtccacc 660
atctgcgccc tctatatcac cctgttgtgc cgactgcgtg ctatccagct agacagccac 720
gccaaggccc tggaccgtgc caagaagcgc gtgaccttgt tggtggtggc gattctggct 780
gtgtgcctcc tctgctggac accgtaccac ctgagcacca tagtggcgct caccaccgac 840
ctcccgcaaa caccgttggt catcggcatc tcttacttca tcaccagtct gagctatgcc 900
aacagctgcc tcaacccttt cctctatgcc ttcctggacg acagcttccg caggagcctg 960
cggcagctgg tgtcatgccg cacagcc 987

<210> 77
<211> 329
<212> PRT

<213> Mus musculus

<400> 77

Met His Asn Leu Thr Leu Phe Glu Ser Gly Gly Asp Asn Val Ser Cys
5 10 15

Gly Gly Ser Ser Leu Gly Cys Pro Asn Gly Ser Ser Leu Ala Pro Leu
20 25 30

Pro Leu Pro Gln Pro Leu Ala Val Ala Val Pro Val Val Tyr Gly Val
35 40 45

Ile Cys Ala Val Gly Leu Ala Gly Asn Ser Ala Val Leu Tyr Val Leu
50 55 60

Leu Arg Thr Pro Arg Met Lys Thr Val Thr Asn Val Phe Ile Leu Asn
65 70 75 80

Leu Ala Ile Ala Asp Glu Leu Phe Thr Leu Val Leu Pro Ile Asn Ile
85 90 95

Ala Asp Phe Leu Leu Arg Arg Trp Pro Phe Gly Glu Val Met Cys Lys
100 105 110

Leu Ile Val Ala Val Asp Gln Tyr Asn Thr Phe Ser Ser Leu Tyr Phe
115 120 125

Leu Ala Val Met Ser Ala Asp Arg Tyr Leu Val Val Leu Ala Thr Ala
130 135 140

Glu Ser Arg Arg Val Ser Gly Arg Thr Tyr Gly Ala Ala Arg Ala Val
145 150 155 160

Ser Leu Ala Val Trp Ala Leu Val Thr Leu Val Val Leu Pro Phe Ala
165 170 175

Val Phe Ala Arg Leu Asp Glu Glu Gln Gly Arg Arg Gln Cys Val Leu
180 185 190

Val Phe Pro Gln Pro Glu Ala Phe Trp Trp Arg Ala Ser Arg Leu Tyr
195 200 205

Thr Leu Val Leu Gly Phe Ala Ile Pro Val Thr Thr Ile Cys Ala Leu
210 215 220

Tyr Thr Thr Leu Leu Cys Arg Leu Arg Ala Ile Gln Leu Asp Ser His
225 230 235 240

Ala Lys Ala Leu Asp Arg Ala Lys Lys Arg Val Thr Leu Leu Val Ala
245 250 255

Ala Ile Leu Ala Val Cys Leu Leu Cys Trp Thr Pro Tyr His Leu Ser

260 265 270
Thr Ile Val Ala Leu Thr Thr Asp Leu Pro Gln Thr Pro Leu Val Ile
275 280 285
Gly Ile Ser Tyr Phe Ile Thr Ser Leu Ser Tyr Ala Asn Ser Cys Leu
290 295 300
Asn Pro Phe Leu Tyr Ala Phe Leu Asp Asp Ser Phe Arg Arg Ser Leu
305 310 315 320
Arg Gln Leu Val Ser Cys Arg Ser Ala
325

<210> 78
<211> 987
<212> DNA
<213> Mus musculus

<400> 78
atgcataact taacgctttt cgagtctgga ggggacaacg tgtcttgcgg cggctcatct 60
ttgggctgtc ccaacgggtc cagcctggct cctctgccgc tgccgcagcc actggcggta 120
gcagtgcctg tcgtctacgg ggtaatttgc gccgtgggac tggctggcaa ctctgcggtg 180
ctgtacgtac tgctgcgcac gccgcgcatg aagactgtca ccaacgtgtt catcctcaac 240
ctggctatcg ccgatgagct cttcaccctc gtgctgccca tcaacatcgc ggacttcctg 300
ctgaggcgct ggcccttcgg ggaggtcatg tgcaagctca ttgtagccgt cgaccagtac 360
aacactttct ctagcctcta cttcctcgcc gtcatgagcg ccgaccgata cctggtggtt 420
ctggccacag cagagtcgcg ccgggtgtcc gggcgcactt acggtgcagc gcgtgctgtc 480
agtctggcgg tgtgggcgct ggtgacgctg gtcgtgctgc cctttgcggt attcgctcgg 540
ctggacgagg agcagggtcg gcgccagtgc gtgctggtct tcccgcagcc cgaggccttc 600
tggtggcgtg ccagccgtct ctacacacta gtattgggct ttgccatccc ggtgaccacc 660
atctgtgctc tctataccac tctgctctgc cgactgcgtg ctatccagct agatagccac 720
gccaaggccc tggatcgtgc caagaagcgc gtgaccttgt tggtggcggc gattctggct 780
gtgtgcctcc tctgctggac gccttatcac ctgagtacca tagtggccct caccaccgac 840
ctcccgcaaa cgccgctggt catcggcatc tcttacttca tcaccagcct gagctatgct 900
aacagctgcc tcaacccttt cctctatgcc ttcctggacg acagcttccg cagaagcctc 960
cggcaattgg tgtcatgccg ttcagcc 987

<210> 79
<211> 328

<212> PRT
<213> Homo sapiens

<400> 79
Met Asp Asn Ala Ser Phe Ser Glu Pro Trp Pro Ala Asn Ala Ser Gly
5 10 15
Pro Asp Pro Ala Leu Ser Cys Ser Asn Ala Ser Thr Leu Ala Pro Leu
20 25 30
Pro Ala Pro Leu Ala Val Ala Val Pro Val Val Tyr Ala Val Ile Cys
35 40 45
Ala Val Gly Leu Ala Gly Asn Ser Ala Val Leu Tyr Val Leu Leu Arg
50 55 60
Ala Pro Arg Met Lys Thr Val Thr Asn Leu Phe Ile Leu Asn Leu Ala
65 70 75 80
Ile Ala Asp Glu Leu Phe Thr Leu Val Leu Pro Ile Asn Ile Ala Asp
85 90 95
Phe Leu Leu Arg Gln Trp Pro Phe Gly Glu Leu Met Cys Lys Leu Ile
100 105 110
Val Ala Ile Asp Gln Tyr Asn Thr Phe Ser Ser Leu Tyr Phe Leu Thr
115 120 125
Val Met Ser Ala Asp Arg Tyr Leu Val Val Leu Ala Thr Ala Glu Ser
130 135 140
Arg Arg Val Ala Gly Arg Thr Tyr Ser Ala Ala Arg Ala Val Ser Leu
145 150 155 160
Ala Val Trp Gly Ile Val Thr Leu Val Val Leu Pro Phe Ala Val Phe
165 170 175
Ala Arg Leu Asp Asp Glu Gln Gly Arg Arg Gln Cys Val Leu Val Phe
180 185 190
Pro Gln Pro Glu Ala Phe Trp Trp Arg Ala Ser Arg Leu Tyr Thr Leu
195 200 205
Val Leu Gly Phe Ala Ile Pro Val Ser Thr Ile Cys Val Leu Tyr Thr
210 215 220
Thr Leu Leu Cys Arg Leu His Ala Met Arg Leu Asp Ser His Ala Lys
225 230 235 240
Ala Leu Glu Arg Ala Lys Lys Arg Val Thr Phe Leu Val Val Ala Ile
245 250 255

Leu Ala Val Cys Leu Leu Cys Trp Thr Pro Tyr His Leu Ser Thr Val
260 265 270
Val Ala Leu Thr Thr Asp Leu Pro Gln Thr Pro Leu Val Ile Ala Ile
275 280 285
Ser Tyr Phe Ile Thr Ser Leu Ser Tyr Ala Asn Ser Cys Leu Asn Pro
290 295 300
Phe Leu Tyr Ala Phe Leu Asp Ala Ser Phe Arg Arg Asn Leu Arg Gln
305 310 315 320
Leu Ile Thr Cys Arg Ala Ala Ala
325

<210> 80
<211> 984
<212> DNA
<213> Homo sapiens

<400> 80
atggacaacg cctcgttctc ggagccctgg cccgccaacg catcgggccc ggacccggcg 60
ctgagctgct ccaacgcgtc gactctggcg ccgctgccgg cgccgctggc ggtggctgta 120
ccagttgtct acgcggtgat ctgcgccgtg ggtctggcgg gcaactccgc cgtgctgtac 180
gtgttgctgc gggcgccccg catgaagacc gtcaccaacc tgttcatcct caacctggcc 240
atcgccgacg agctcttcac gctggtgctg cccatcaaca tcgccgactt cctgctgcgg 300
cagtggccct tcggggagct catgtgcaag ctcatcgtgg ctatcgacca gtacaacacc 360
ttctccagcc tctacttcct caccgtcatg agcgccgacc gctacctggt ggtgttggcc 420
actgcggagt cgcgccgggt ggccggccgc acctacagcg ccgcgcgcgc ggtgagcctg 480
gccgtgtggg ggatcgtcac actcgtcgtg ctgcccttcg cagtcttcgc ccggctagac 540
gacgagcagg gccggcgcca gtgcgtgcta gtctttccgc agcccgaggc cttctggtgg 600
cgcgcgagcc gcctctacac gctcgtgctg ggcttcgcca tccccgtgtc caccatctgt 660
gtcctctata ccaccctgct gtgccggctg catgccatgc ggctggacag ccacgccaag 720
gccctggagc gcgccaagaa gcgggtgacc ttcctggtgg tggcaatcct ggcggtgtgc 780
ctcctctgct ggacgcccta ccacctgagc accgtggtgg cgctcaccac cgacctcccg 840
cagacgccgc tggtcatcgc tatctcctac ttcatcacca gcctgagcta cgccaacagc 900
tgcctcaacc ccttcctcta cgccttcctg gacgccagct tccgcaggaa cctccgccag 960
ctgataactt gccgcgcggc agcc 984

## Description

### TECHNICAL FIELD

The present invention relates to agents for preventing/treating upper digestive tract disorders and screening thereof. More particularly, the present invention relates to agents for preventing/treating dyspepsia, bone metabolism disorders, anemia, etc.; gastric acid secretion inhibitors and gastric acid secretion promoters as well as screening thereof; in addition, test agents for gastric secretory function, and so on.

### BACKGROUND ART

Digestive tract disorders are one of the common diseases in routine clinical practice. Among them, reflux esophagitis, gastritis and peptic ulcer are diseases closely associated with gastric acid secretion. Reflux esophagitis is a disease that entails the regurgitation of stomach fluid or duodenal fluid into the esophagus due to an impaired mechanism of reflux prevention in the lower esophageal sphincter, which damages the esophageal mucosa. Patients with reflux esophagitis are increasing, as the population of elderly people is rising. Further when reflux esophagitis is prolonged, Barrett's epithelium is formed at the lower end of the esophagus, which may still be at high risk for development of columnar epithelial carcinoma of the esophagus. Reportedly, approximately 80% of causes of gastritis is infection from Helicobacter and the rest are drugs, stress, spicy food, etc. The pathogenesis of peptic ulcer disease is multifactorial and roughly assigned to the three causes of (i) infection with Helicobacter pylori, (ii) lesions induced by nonsteroidal anti-inflammatory drugs, (iii) hyperchlorhydria which may mimic Zollinger-Ellison syndrome. The fundamental principle of treatment for these diseases is concomitant administration of attacking factor retardants, defending factor potentiators and antibacterial agents to Helicobacter pylori. As the attacking factor retardants, gastric acid secretion inhibitors (dried aluminum hydroxide gel, magnesium oxide, etc.), antipeptic drugs (sucralfate, ecabet sodium, etc.), gastric acid secretion inhibitors (anticholinergic drugs, $H_2$ blockers, proton pump inhibitors, etc.) and the like are used clinically. Development of $H_2$ blockers and proton pump inhibitors achieved groundbreaking progress in the treatment of upper digestive tract disorders. In the clinical field, however, gastric acid secretion inhibitors tend to be administered over a long term to patients who are unsuitable for surgical treatment, including elderly patients with reflux esophagitis, patients with Helicobacter pylori infection who developed acute gastroduodenal ulcer or reflux esophagitis after the bacteria were controlled, and to treat intractable ulcer, etc. It is reported in animal tests that long-term medication of these drugs causes abnormal hyperplasia of gastric mucosa, leading to carcinoma (gastric cancer). In clinical tests, when gastric acid secretion inhibitors are administered for long periods of time, proliferation of gastric mucosa is observed and relapse of ulcers etc. occurs by rebound acid secretion after discontinuation of the long-term medication. Also, $H_2$ blockers and proton pump inhibitors are required to be carefully administered to the patients with renal or hepatic dysfunction; known side effects include shock, anaphylactic reaction, pancytopenia, thrombocytopenia, agranulocytosis, hemolytic anemia, granulocytosis, anemia, toxic epidermal necrolysis, mucocutaneous ocular syndrome, rash, itching paraesthesia, hepatic dysfunction, icterus, hypereosinophilic syndrome, gastrointestinal symptom, headache, drowsiness/insomnia, dizziness, tremor, fever, total cholesterol, increased uric acid levels, gynecomastia, blurred vision, edema, feeling of exhaustion, boredom, numbness of tongue, mouth and lips, arthralgia, myalgia, alopecia, etc.

On the other hand, peptides having increased food intake, stimulated prolactin release, etc. are reported (WO 01/98494, J. Biol. Chem., 277, 35826-35832, 2002) (Neuropeptide W, NPW, termed GPR8 ligands, etc.) as ligands for human GPR8 (Genomics, 28, 84-91, 1995).

As stated above, it has been desired to provide gastric acid secretion inhibitors free from rebound phenomenon in acid secretion and minimizing those side effects.

### DISCLOSURE OF THE INVENTION

In view of the current state of the art, the present inventors made extensive investigations and found that GPR8 ligands promote gastric acid secretion in rats. The inventors have pursued further investigations and as a result, have come to accomplish the present invention.

That is, the present invention provides the following features and so on.

(1) An agent for preventing/treating upper digestive tract disorders, which comprises a compound or its salt that inhibits the activity of a polypeptide comprising the same or substantially the same amino acid sequence as the amino acid sequence represented by SEQ ID NO: 1, its amide or ester, or a salt thereof.

(1a) An agent for preventing/treating upper digestive tract disorders, which comprises a compound or its salt that inhibits the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

(1b) An agent for preventing/treating upper digestive tract disorders, which comprises an antagonist to a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

(2) An agent for preventing/treating upper digestive tract disorders, which comprises (i) an antibody to a polypeptide comprising the same or substantially the same amino acid sequence as the amino acid sequence represented by SEQ ID NO: 1, its amide or ester, or a salt thereof, or (ii) an antibody to a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

(3) An agent for preventing/treating upper digestive tract disorders, which comprises an antisense polynucleotide comprising the entire or part of base sequence complementary or substantially complementary to a base sequence for (i) a polynucleotide encoding a polypeptide comprising the same or substantially the same amino acid sequence as the amino acid sequence represented by SEQ ID NO: 1, its amide or ester, or a salt thereof, or (ii) a polynucleotide encoding a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

(4) A gastric acid secretion inhibitor, which comprises a compound or its salt that inhibits the activity of a polypeptide comprising the same or substantially the same amino acid sequence as the amino acid sequence represented by SEQ ID NO: 1, its amide or ester, or a salt thereof.

(4a) A gastric acid secretion inhibitor, which comprises a compound or its salt that inhibits the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof.

(4b) A gastric acid secretion inhibitor, which comprises an antagonist to a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

(5) An agent for preventing/treating dyspepsia, bone metabolism disorders or anemia, which comprises a compound or its salt that promotes the activity of a polypeptide comprising the same or substantially the same amino acid sequence as the amino acid sequence represented by SEQ ID NO: 1, its amide or ester, or a salt thereof.

(5a) An agent for preventing/treating dyspepsia, bone metabolism disorders or anemia, which comprises a compound or its salt that promotes the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

(5b) An agent for preventing/treating dyspepsia, bone metabolism disorders or anemia, which comprises an agonist to a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

(6) An agent for preventing/treating dyspepsia, bone metabolism disorders or anemia, which comprises a polypeptide comprising the same or substantially the same amino acid sequence as the amino acid sequence represented by SEQ ID NO: 1, its amide or ester, or a salt thereof.

(7) An agent for preventing/treating dyspepsia, bone metabolism disorders or anemia, which comprises a polynucleotide encoding a polypeptide comprising the same or substantially the same amino acid sequence as the amino acid sequence represented by SEQ ID NO: 1, its amide or ester, or a salt thereof.

(8) A gastric acid secretion promoter, which comprises a compound or its salt that promotes the activity of a polypeptide comprising the same or substantially the same amino acid sequence as the amino acid sequence represented by SEQ ID NO: 1, its amide or ester, or a salt thereof.

(8a) A gastric acid secretion promoter, which comprises a compound or its salt that promotes the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

(8b) A gastric acid secretion promoter, which comprises an agonist to a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

(9) A test agent for gastric secretory function, which comprises a polypeptide comprising the same or substantially the same amino acid sequence as the amino acid sequence represented by SEQ ID NO: 1, its amide or ester, or a salt thereof.
(10) A test agent for gastric secretory function, which comprises a compound or its salt that promotes the activity of a polypeptide comprising the same or substantially the same amino acid sequence as the amino acid sequence represented by SEQ ID NO: 1, its amide or ester, or a salt thereof.
(11) A method of screening a preventive/therapeutic agent for upper digestive tract disorders, which comprises using (i) a polypeptide comprising the same or substantially the same amino acid sequence as the amino acid sequence represented by SEQ ID NO: 1, its amide or ester, or a salt thereof, and/or (ii) a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.
(12) A kit for screening a preventive/therapeutic agent for upper digestive tract disorders, which comprises (i) a polypeptide comprising the same or substantially the same amino acid sequence as the amino acid sequence represented by SEQ ID NO: 1, its amide or ester, or a salt thereof, and/or (ii) a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

(12a) An agent for preventing/treating upper digestive tract disorders, which is obtainable by using the screening method according to (11) or the screening kit according to (12).

(13) A method of screening a preventive/therapeutic agent for upper digestive tract disorders, which comprises using (i) a polynucleotide encoding a polypeptide comprising the same or substantially the same amino acid sequence as the amino acid sequence represented by SEQ ID NO: 1, its amide or ester, or a salt thereof, and/or (ii) a polynucleotide encoding a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.
(14) A kit for screening a preventive/therapeutic agent for upper digestive tract disorders, which comprises (i) a polynucleotide encoding a polypeptide comprising the same or substantially the same amino acid sequence as the amino acid sequence represented by SEQ ID NO: 1, its amide or ester, or a salt thereof, and/or (ii) a polynucleotide encoding a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

(14a) An agent for preventing/treating upper digestive tract disorders, which is obtainable by using the screening method according to (13) or the screening kit according to (14).

(15) A method of screening a gastric acid secretion inhibitor, which comprises using (i) a polypeptide comprising the same or substantially the same amino acid sequence as the amino acid sequence represented by SEQ ID NO: 1, its amide or ester, or a salt thereof, and/or (ii) a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

(15a) A gastric acid secretion inhibitor, which is obtainable by using the screening method according to (15).

(16) A method of screening a preventive/therapeutic agent for dyspepsia, bone metabolism disorders or anemia, which comprises using (i) a polypeptide comprising the same or substantially the same amino acid sequence as the amino acid sequence represented by SEQ ID NO: 1, its amide or ester, or a salt thereof, and/or (ii) a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

(16a) An agent for preventing/treating dyspepsia, bone metabolism disorders or anemia, which is obtainable by using the screening method according to (16).

(17) A method of screening a gastric acid secretion promoter, which comprises using (i) a polypeptide comprising the same or substantially the same amino acid sequence as the amino acid sequence represented by SEQ ID NO: 1, its amide or ester, or a salt thereof, and/or (ii) a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

(17a) A gastric acid secretion promoter, which is obtainable by using the screening method according to (17).

(18) A method of preventing/treating upper digestive tract disorders, which comprises administering to a mammal an effective dose of (i) a compound or its salt that inhibits the activity of a polypeptide comprising the same or substantially the same amino acid sequence as the amino acid sequence represented by SEQ ID NO: 1, its amide or ester, or a salt thereof, (ii) an antibody to said polypeptide, its amide or ester, or a salt thereof, (iii) an antisense polynucleotide comprising the entire or part of base sequence complementary or substantially complementary to a base sequence for a polynucleotide encoding said polypeptide, its amide or ester, or a salt thereof, (iv) a compound or its salt that inhibits the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) an antibody to said protein, its partial peptide, or a salt thereof, or (vi) an antisense polynucleotide comprising the entire or part of base sequence complementary or substantially complementary to a base sequence for a polynucleotide encoding said protein, its partial peptide, or a salt thereof.

(19) A method of preventing/treating upper digestive tract disorders, which comprises (a) inhibiting the activity of a polypeptide comprising the same or substantially the same amino acid sequence as the amino acid sequence represented by SEQ ID NO: 1, its amide or ester, or a salt thereof, or (b) inhibiting the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof.

(20) Use of (i) a compound or its salt that inhibits the activity of a polypeptide comprising the same or substantially the same amino acid sequence as the amino acid sequence represented by SEQ ID NO: 1, its amide or ester, or a salt thereof, (ii) an antibody to said polypeptide, its amide or ester, or a salt thereof, (iii) an antisense polynucleotide comprising the entire or part of base sequence complementary or substantially complementary to a base sequence for a polynucleotide encoding said polypeptide, its amide or ester, or a salt thereof, (iv) a compound or its salt that inhibits the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) an antibody to said protein, its partial peptide, or a salt thereof, or (vi) an antisense polynucleotide comprising the entire or part of base sequence complementary or substantially complementary to a base sequence for a polynucleotide encoding said protein, its partial peptide, or a salt thereof, to manufacture a gastric acid secretion inhibitor.

(21) A method of suppressing gastric acid secretion, which comprises administering to a mammal an effective dose of (i) a compound or its salt that inhibits the activity of a polypeptide comprising the same or substantially the same amino acid sequence as the amino acid sequence represented by SEQ ID NO: 1, its amide or ester, or a salt thereof, (ii) an antibody to said polypeptide, its amide or ester, or a salt thereof, (iii) an antisense polynucleotide comprising the entire or part of base sequence complementary or substantially complementary to a base sequence for a polynucleotide encoding said polypeptide, its amide or ester, or a salt thereof, (iv) a compound or its salt that inhibits the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) an antibody to said protein, its partial peptide, or a salt thereof, or (vi) an antisense polynucleotide comprising the entire or part of base sequence complementary or substantially complementary to a base sequence for a polynucleotide encoding said protein, its partial peptide, or a salt thereof.

(22) A method of suppressing gastric acid secretion, which comprises (a) inhibiting the activity of a polypeptide comprising the same or substantially the same amino acid sequence as the amino acid sequence represented by SEQ ID NO: 1, its amide or ester, or a salt thereof, or (b) inhibiting the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof.

(23) Use of (i) a compound or its salt that inhibits the activity of a polypeptide comprising the same or substantially the same amino acid sequence as the amino acid sequence represented by SEQ ID NO: 1, its amide or ester, or a salt thereof, (ii) an antibody to said polypeptide, its amide or ester, or a salt thereof, (iii) an antisense polynucleotide comprising the entire or part of base sequence complementary or substantially complementary to a base sequence for a polynucleotide encoding said polypeptide, its amide or ester, or a salt thereof, (iv) a compound or its salt that inhibits the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) an antibody to said protein, its partial peptide, or a salt thereof, or (vi) an antisense polynucleotide comprising the entire or part of base sequence complementary or substantially complementary to a base sequence for a polynucleotide encoding said protein, its partial peptide, or a salt thereof, to manufacture a preventive/therapeutic agent for upper digestive tract disorders.

(24) A method of preventing/treating dyspepsia, bone metabolism disorders or anemia, which comprises administering to a mammal an effective dose of (i) a compound or its salt that promotes the activity of a polypeptide comprising the same or substantially the same amino acid sequence as the amino acid sequence represented by SEQ ID NO: 1, its amide or ester, or a salt thereof, (ii) said polypeptide, its amide or ester, or a salt thereof, (iii) a polynucleotide encoding said polypeptide, its amide or ester, or a salt thereof, (iv) a compound or its salt that promotes the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) said protein, its partial peptide, or a salt thereof, or (vi) a polynucleotide encoding said protein, its partial peptide, or a salt thereof.

(25) A method of preventing/treating dyspepsia, bone metabolism disorders or anemia, which comprises (a) promoting the activity of a polypeptide comprising the same or substantially the same amino acid sequence as the amino acid sequence represented by SEQ ID NO: 1, its amide or ester, or a salt thereof, or (b) promoting the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof.

(26) Use of (i) a compound or its salt that promotes the activity of a polypeptide comprising the same or substantially the same amino acid sequence as the amino acid sequence represented by SEQ ID NO: 1, its amide or ester, or a salt thereof, (ii) said polypeptide, its amide or ester, or a salt thereof, (iii) a polynucleotide encoding said polypeptide, its amide or ester, or a salt thereof, (iv) a compound or its salt that promotes the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) said protein, its partial peptide, or a salt thereof, or (vi) a polynucleotide encoding said protein, its partial peptide, or a salt thereof, to manufacture a preventive/therapeutic agent for dyspepsia, bone metabolism disorders or anemia.

(27) A method of promoting gastric acid secretion, which comprises administering to a mammal an effective dose of (i) a compound or its salt that promotes the activity of a polypeptide comprising the same or substantially the same amino acid sequence as the amino acid sequence represented by SEQ ID NO: 1, its amide or ester, or a salt thereof, (ii) said polypeptide, its amide or ester, or a salt thereof, (iii) a polynucleotide encoding said polypeptide, its amide or ester, or a salt thereof, (iv) a compound or its salt that promotes the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) said protein, its partial peptide, or a salt thereof, or (vi) a polynucleotide encoding said protein, its partial peptide, or a salt thereof.

(28) A method of promoting gastric acid secretion, which comprises (a) promoting the activity of a polypeptide comprising the same or substantially the same amino acid sequence as the amino acid sequence represented by SEQ ID NO: 1, its amide or ester, or a salt thereof, or (b) promoting the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof.

(29) Use of (i) a compound or its salt that promotes the activity of a polypeptide comprising the same or substantially the same amino acid sequence as the amino acid sequence represented by SEQ ID NO: 1, its amide or ester, or a salt thereof, (ii) said polypeptide, its amide or ester, or a salt thereof, (iii) a polynucleotide encoding said polypeptide, its amide or ester, or a salt thereof, (iv) a compound or its salt that promotes the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) said protein, its partial peptide, or a salt thereof, or (vi) a polynucleotide encoding said protein, its partial peptide, or a salt thereof, to manufacture a gastric acid secretion promoter.

(30) A method of testing gastric secretory function, which comprises using a polypeptide comprising the same or substantially the same amino acid sequence as the amino acid sequence represented by SEQ ID NO: 1, its amide or ester, or a salt thereof, or a compound or its salt that promotes the activity of said polypeptide, its amide or ester, or a salt thereof.

BRIEF DESCRIPTION OF THE DRAWINGS

FIG. 1 shows changes in blood gastrin levels by administration of NPW, wherein the values indicate mean ± standard error (mean ± SE) (n=8) and symbol * designates the p-value is not greater than 0.05, as compared to the saline group.

BEST MODE FOR CARRYING OUT THE INVENTION

The polypeptide having the same or substantially the same amino acid sequence as the amino acid sequence represented by SEQ ID NO: 1 (hereinafter sometimes referred to as the polypeptide of the present invention) may be any polypeptide derived from any cells of human and other warm-blooded animals (e.g. guinea pig, rat, mouse, fowl, rabbit, swine, sheep, bovine, monkey, etc.) (for example, retina cell, liver cell, splenocyte, nerve cell, glial cell, β cell of pancreas, bone marrow cell, mesangial cell, Langerhans' cell, epidermic cell, epithelial cell, endothelial cell, fibroblast, fibrocyte, myocyte, fat cell, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte, interstitial cell, etc., or the corresponding precursor cells, stem cells, cancer cells, etc.), or any tissues where such cells are present, such as brain or any of brain regions (e.g., retina, olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; polypeptides derived from hemocyte type cells or their cultured cells (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.); the polypeptides may also be synthetic polypeptides.
Substantially the same amino acid sequence as the amino acid sequence represented by SEQ ID NO: 1 includes an amino acid sequence having at least about 70% homology, preferably at least about 80% homology, preferably at least about 90% homology, preferably at least about 95% homology, and more preferably at least about 98% homology, to the amino acid sequence represented by SEQ ID NO: 1.
Specifically, substantially the same amino acid sequence as the amino acid sequence represented by SEQ ID NO: 1 includes, in addition to the amino acid sequences described above: (i) the amino acid sequence represented by SEQ ID NO: 1, of which 1 to 5 (preferably 1 to 3, more preferably 1 or 2, and most preferably 1) amino acids are deleted; (ii) the amino acid sequence represented by SEQ ID NO: 1, to which 1 to 5 (preferably 1 to 3, more preferably 1 or 2, and most preferably 1) amino acids are added; (iii) the amino acid sequence represented by SEQ ID NO: 1, in which 1 to 5 (preferably 1 to 3, more preferably 1 or 2, and most preferably 1) amino acids are inserted; (iv) the amino acid sequence represented by SEQ ID NO: 1, in which 1 to 5 (preferably 1 to 3, more preferably 1 or 2, and most preferably 1) amino acids are substituted with other amino acids; and (v) a combination of the amino acid sequences (i) through (iv) described above, etc.
Examples of the polypeptide which has substantially the same amino acid sequence as the amino acid sequence represented by SEQ ID NO: 1 include a polypeptide containing substantially the same amino acid sequence as the amino acid sequence represented by SEQ ID NO: 1 and having an activity substantially equivalent to that of the amino acid sequence represented by SEQ ID NO: 1, and the like.
The activity which is substantially equivalent includes, for example, the activities possessed by the polypeptide of the present invention (e.g., the activity of promoting gastric acid secretion, etc.) and the like.
The term "activity which is substantially equivalent" is used to mean that these activities are equivalent in nature (for example, biochemically or pharmacologically).
Gastric acid secretion promotion may be determined by modifications of publicly known methods, for example, by the method described in Gastroenterology, 5, 43-45, 1945, or its modifications, the method described in EXAMPLES later given, etc.
Specific examples of the amino acid sequences, which are substantially the same as the amino acid sequence represented by SEQ ID NO: 1, include amino acid sequences represented by SEQ ID NO: 2, SEQ ID NO: 7, SEQ ID NO: 8, SEQ ID NO: 9, SEQ ID NO: 10, SEQ ID NO: 11, SEQ ID NO: 12, SEQ ID NO: 21, SEQ ID NO: 24, SEQ ID NO: 25, SEQ ID NO: 30, SEQ ID NO: 31, SEQ ID NO: 36, SEQ ID NO: 37, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58 or SEQ ID NO: 71, and the like.
Specific examples of the polypeptide of the present invention are polypeptides capable of specifically binding to the polypeptide of the present invention, including a polypeptide having the amino acid sequence represented by SEQ ID NO: 1, a polypeptide having the amino acid sequence represented by SEQ ID NO: 2, a polypeptide having the amino acid sequence represented by SEQ ID NO: 21, a polypeptide having the amino acid sequence represented by SEQ ID NO: 7, a polypeptide having the amino acid sequence represented by SEQ ID NO: 8, a polypeptide having the amino acid sequence represented by SEQ ID NO: 9, a polypeptide having the amino acid sequence represented by SEQ ID NO: 10, a polypeptide having the amino acid sequence represented by SEQ ID NO: 11, a polypeptide having the amino acid sequence represented by SEQ ID NO: 12, a polypeptide having the amino acid sequence represented by SEQ ID NO: 24, a polypeptide having the amino acid sequence represented by SEQ ID NO: 25, a polypeptide having the amino acid sequence represented by SEQ ID NO: 30, a polypeptide having the amino acid sequence represented by SEQ ID NO: 31, a polypeptide having the amino acid sequence represented by SEQ ID NO: 36, a polypeptide having the amino acid sequence represented by SEQ ID NO: 37, a polypeptide having the amino acid sequence represented by SEQ ID NO: 40, a polypeptide having the amino acid sequence represented by SEQ ID NO: 41, a polypeptide having the amino acid sequence represented by SEQ ID NO: 42, a polypeptide having the amino acid sequence represented by SEQ ID NO: 43, a polypeptide having the amino acid sequence represented by SEQ ID NO: 44, a polypeptide having the amino acid sequence represented by SEQ ID NO: 45, a polypeptide having the amino acid sequence represented by SEQ ID NO: 46, a polypeptide having the amino acid sequence represented by SEQ ID NO: 47, a polypeptide having the amino acid sequence represented by SEQ ID NO: 48, a polypeptide having the amino acid sequence represented by SEQ ID NO: 49, a polypeptide having the amino acid sequence represented by SEQ ID NO: 50, a polypeptide having the amino acid sequence represented by SEQ ID NO: 51, a polypeptide having the amino acid sequence represented by SEQ ID NO: 52, a polypeptide having the amino acid sequence represented by SEQ ID NO: 53, a polypeptide having the amino acid sequence represented by SEQ ID NO: 54, a polypeptide having the amino acid sequence represented by SEQ ID NO: 55, a polypeptide having the amino acid sequence represented by SEQ ID NO: 56, a polypeptide having the amino acid sequence represented by SEQ ID NO: 57, a polypeptide having the amino acid sequence represented by SEQ ID NO: 58 or a polypeptide having the amino acid sequence represented by SEQ ID NO: 71, etc.

The polypeptide of the present invention is used to also mean a precursor polypeptide of the polypeptide of the present invention.

Specific examples of the precursor polypeptides are polypeptides characterized by comprising the same or substantially the same amino acid sequence as the amino acid sequence represented by SEQ ID NO: 6, etc.

More specifically, the amino acid sequence, which is substantially the same as the amino acid sequence represented by SEQ ID NO: 6, includes amino acid sequences having at least about 80% homology, preferably at least about 90% homology, and more preferably at least about 95% homology, to the amino acid sequence represented by SEQ ID NO: 6, etc.

In particular, the amino acid sequence, which is substantially the same as the amino acid sequence represented by SEQ ID NO: 6 includes, in addition to the amino acid sequences described above, (i) the amino acid sequence represented by SEQ ID NO: 6, of which 1 to 15 (preferably 1 to 10, more preferably 1 to 5, and most preferably 1 to 3) amino acids are deleted; (ii) the amino acid sequence represented by SEQ ID NO: 6, to which 1 to 100 (preferably 1 to 50, more preferably 1 to 5, and most preferably I to 3) amino acids are added; (iii) the amino acid sequence represented by SEQ ID NO: 6, in which 1 to 15 (preferably 1 to 10, more preferably 1 to 5, and most preferably 1 to 3) amino acids are inserted; (iv) the amino acid sequence represented by SEQ ID NO: 6, in which I to 15 (preferably 1 to 10, more preferably 1 to 5, and most preferably 1 to 3) amino acids are substituted with other amino acids; and (v) a combination of the amino acid sequences (i) through (iv) described above, etc.

Specific examples of the amino acid sequence, which is substantially the same as the amino acid sequence represented by SEQ ID NO: 6, include an amino acid sequence represented by SEQ ID NO: 20, SEQ ID NO: 23, SEQ ID NO: 29 or SEQ ID NO: 35, and the like.

Specific examples of the precursor polypeptide described above are a polypeptide having the amino acid sequence represented by SEQ ID NO: 6, a polypeptide having the amino acid sequence represented by SEQ ID NO: 20, a polypeptide having the amino acid sequence represented by SEQ ID NO: 23, a polypeptide having the amino acid sequence represented by SEQ ID NO: 29 or a polypeptide having the amino acid sequence represented by SEQ ID NO: 35, and the like.

In various receptors, the receptors to the polypeptide of the present invention are used to mean those that have the activity binding to the polypeptide of the present invention and the cell-stimulating activity of the receptor-expressed cells (e.g., the activity that promotes arachidonic acid release, acetylcholine release, intracellular $Ca^{2+}$ release, intracellular cAMP production/suppression, intracellular cGMP production, inositol phosphate production, change in cell membrane potential, phosphorylation of intracellular proteins, activation of c-fos, pH reduction, $GTP_{\gamma}S$ binding activity, etc.) is observed by the polypeptide of the present invention, and the like.

Specifically, the receptors include (1) GPR8 (SEQ ID NO: 73, Genomics, 28, 84-91, 1995), or a protein comprising an amino acid sequence, which is substantially the same as the amino acid sequence represented by SEQ ID NO: 73, (2) rat TGR26 (SEQ ID NO: 75; WO 02/44368) or a receptor comprising an amino acid sequence, which is substantially the same as the amino acid sequence represented by SEQ ID NO: 75, (3) mouse TGR 26 (SEQ ID NO: 77; WO 02/44368) or a receptor comprising an amino acid sequence, which is substantially the same as the amino acid sequence represented by SEQ ID NO: 77, (4) GPR7 (SEQ ID NO: 79; Genomics, 28, 84-91, 1995) or a receptor comprising an amino acid sequence, which is substantially the same as the amino acid sequence represented by SEQ ID NO: 79, and the like.

The protein having the same or substantially the same amino acid sequence as the amino acid sequence represented by SEQ ID NO: 73, the protein having the same or substantially the same amino acid sequence as the amino acid sequence represented by SEQ ID NO: 76, The protein having the same or substantially the same amino acid sequence as the amino acid sequence represented by SEQ ID NO: 77, The protein having the same or substantially the same amino acid sequence as the amino acid sequence represented by SEQ ID NO: 79 (hereinafter sometimes collectively referred to as the receptor of the present invention) may be any protein derived from any cells of human and other warm-blooded animals (e.g. guinea pig, rat, mouse, fowl, rabbit, swine, sheep, bovine, monkey, etc.) (for example, retina cell, liver cell, splenocyte, nerve cell, glial cell, β cell of pancreas, bone marrow cell, mesangial cell, Langerhans' cell, epidermic cell, epithelial cell, endothelial cell, fibroblast, fibrocyte, myocyte, fat cell, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte, interstitial cell, etc., or the corresponding precursor cells, stem cells, cancer cells, etc.), or any tissues where such cells are present, such as brain or any of brain regions (e.g., retina, olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; proteins derived from hemocyte type cells or their cultured cells (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.); the proteins may also be synthetic proteins.

The amino acid sequence substantially the same as the amino acid sequence represented by SEQ ID NO: 73 includes amino acid sequences having at least about 70% homology, preferably at least about 80% homology, and more preferably at least about 90% homology, to the amino acid sequence represented by SEQ ID NO: 73, etc.

The amino acid sequence substantially the same as the amino acid sequence represented by SEQ ID NO: 75 includes amino acid sequences having at least about 85% homology, preferably at least about 90% homology, and more preferably at least about 95% homology, to the amino acid sequence represented by SEQ ID NO: 75, etc.

The amino acid sequence substantially the same as the amino acid sequence represented by SEQ ID NO: 77 includes amino acid sequences having at least 86% homology, preferably at least about 90% homology, and more preferably at least about 95% homology, to the amino acid sequence represented by SEQ ID NO: 77, etc.

The amino acid sequence substantially the same as the amino acid sequence represented by SEQ ID NO: 79 includes amino acid sequences having at least about 70% homology, preferably at least about 80% homology, and more preferably at least about 90% homology, to the amino acid sequence represented by SEQ ID NO: 79, etc.

Examples of the protein, which comprises substantially the same amino acid sequence as the amino acid sequence represented by SEQ ID NO: 73, SEQ ID NO: 75, SEQ ID NO: 77 or SEQ ID NO: 79, include a protein containing substantially the same amino acid sequence as the amino acid sequence represented by SEQ ID NO: 73, SEQ ID NO: 76, SEQ ID NO: 77 or SEQ ID NO: 79 and having an activity substantially equivalent to that of the amino acid sequence represented by SEQ ID NO: 73, SEQ ID NO: 76, SEQ ID NO: 77 or SEQ ID NO: 79, and the like.

In particular, the amino acid sequences substantially the same as the amino acid sequence represented by SEQ ID NO: 73, SEQ ID NO: 75, SEQ ID NO: 77 or SEQ ID NO: 79 include, (i) the amino acid sequence represented by SEQ ID NO: 73, SEQ ID NO: 75, SEQ ID NO: 77 or SEQ ID NO: 79, of which 1 to 15 (preferably 1 to 10, more preferably 1 to 5, and most preferably I to 3) amino acids are deleted; (ii) the amino acid sequence represented by SEQ ID NO: 73, SEQ ID NO: 75, SEQ ID NO: 77 or SEQ ID NO: 79, to which 1 to 15 (preferably 1 to 10, more preferably 1 to 5, and most preferably 1 to 3) amino acids are added; (iii) the amino acid sequence represented by SEQ ID NO: 73, SEQ ID NO: 75, SEQ ID NO: 77 or SEQ ID NO: 79, in which 1 to 15 (preferably 1 to 10, more preferably 1 to 5, and most preferably 1 to 3) amino acids are inserted; (iv) the amino acid sequence represented by SEQ ID NO: 73, SEQ ID NO: 75, SEQ ID NO: 77 or SEQ ID NO: 79, in which 1 to 15 (preferably 1 to 10, more preferably 1 to 5, and most preferably 1 to 3) amino acids are substituted with other amino acids; and (v) a combination of the amino acid sequences (i) through (iv) described above, etc.

Specific examples of the receptor of the present invention include a protein comprising the amino acid sequence represented by SEQ ID NO: 73, a protein comprising the amino acid sequence represented by SEQ ID NO: 75, a protein comprising the amino acid sequence represented by SEQ ID NO: 77 and a protein comprising the amino acid sequence represented by SEQ ID NO: 79 etc.

Any partial peptide can be used as the partial peptide of the receptor to the polypeptide of the present invention (hereinafter sometimes referred to as the partial peptide of the present invention), as long as it is a partial peptide available for the method of screening drugs, etc. later described. Preferably, there may be employed partial peptides capable of binding to the polypeptide of the present invention, partial peptides containing an amino acid sequence corresponding to the extracellular region, and the like. Peptides having sequences of at least 20, preferably at least 50 and more preferably at least 100 amino acids, in the amino acid sequences, which constitute the receptor of the present invention, etc. are preferred. (i) At least 1 or 2 (preferably approximately 1 to 10 and more preferably several (1 to 5)) amino acids may be deleted of the amino acid sequence described above; (ii) at least 1 to 2 (preferably approximately 1 to 20, more preferably approximately 1 to 10 and most preferably several (1 to 5)) amino acids may be added to the amino acid sequence described above; or (iii) at least 1 to 2 (preferably approximately 1 to 10, more preferably several and most preferably approximately 1 to 5) amino acids may be substituted with other amino acids in the amino acid sequence described above, etc.

Specifically, the partial peptides include (a), in the amino acid sequence represented by SEQ ID NO: 73, partial peptides comprising at least 1 or 2 partial amino acid sequences selected from the partial amino acid sequences consisting of the 1 (Met) - 123 (Phe) amino acid residues or a part thereof, the 301 (Asn) - 358 (Lys) amino acid residues or a part thereof, the 548 (Tyr) - 593 (Arg) amino acid residues or a part thereof and the 843 (Ala) - 895 (Ile) amino acid residues or a part thereof, (b), in the amino acid sequence represented by SEQ ID NO: 75, partial peptides comprising at least 1 or 2 partial amino acid sequences selected from the partial amino acid sequences consisting of the 1(Met) - 85(Asp) amino acid residues or a part thereof, partial amino acid sequence consisting of the 222(Cys) - 329 (Ala) amino acid residues or a part thereof, and the like.

The polypeptides, receptors or partial peptides of the present invention are represented in accordance with the conventional way of describing peptides, that is, the N-terminus (amino terminus) at the left hand and the C-terminus (carboxyl terminus) at the right hand. In the polypeptides receptors or partial peptides of the present invention, the C-terminus is usually in the form of a carboxyl group (-COOH) or a carboxylate (-COO-) but the C-terminus may be in the form of an amide ($-CONH_2$) or an ester (-COOR).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; an aralkyl having 7 to 14 carbon atoms such as a phenyl-$C_{1-2}$ alkyl group, e.g., benzyl, phenethyl, etc.; an α-naphthyl-$C_{1-2}$ alkyl group such as α-naphthylmethyl, etc.; and the like. In addition, pivaloyloxymethyl or the like, which is used widely as an ester for oral administration may also be used.

Where the polypeptides, receptors or partial peptides of the present invention contain a carboxyl group (or a carboxylate) at a position other than the C-terminus, it may be amidated or esterified and such an amide or ester is also included within the polypeptide of the present invention. In this case, the ester group may be the C-terminal esters, etc. described above.

The polypeptides, receptors or partial peptides of the present invention further include those wherein the amino group at the N-terminal amino acid residues (e.g., methionine residue) is protected with a protecting group (e. g., a $C_{1-6}$ acyl group, e.g., a $C_{1-6}$ alkanoyl group such as formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{1-6}$ acyl group, e.g., a $C_{1-6}$ alkanoyl group such as formyl group, acetyl group, etc.), or conjugated proteins such as so-called glycoproteins having sugar chains, and the like.

As salts of the polypeptides, receptors or partial peptides of the present invention, there are salts with physiologically acceptable acids (e.g., inorganic acids, organic acids) or bases (e.g., alkali metal bases), etc., with particular preference in the form of physiologically acceptable acid addition salts. Examples of such salts are salts with inorganic acids (e.g., hydrochloric acid, phosphoric acid, hydrobromic acid, and sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The polypeptides, receptors or partial peptides of the present invention may be manufactured by a publicly known method used to purify polypeptides from human or other warm-blooded animal cells or tissues described above, or may also be manufactured by culturing a transformant containing a DNA encoding the polypeptide, as will be later described. The polypeptides, receptors or partial peptides can be manufactured by modifications of the methods described in, e.g., WO 01/98494, WO 02/44368, etc.

Where the polypeptides, receptors or partial peptides are manufactured from human or mammalian tissues or cells, human or mammalian tissues or cells are homogenized and extracted with an acid or the like, and the extract is purified and isolated by a combination of chromatography techniques such as reversed phase chromatography, ion exchange chromatography, and the like.

To synthesize the polypeptides, receptors or partial peptides of the present invention or salts thereof, or amides thereof, commercially available resins that are used for polypeptide synthesis may normally be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in the order of the sequences of the objective polypeptide according to various condensation methods publicly known in the art. At the end of the reaction, the polypeptide is excised from the resin and at the same time, the protecting groups are removed. Then, intramolecular disulfide bond-forming reaction is performed in a highly diluted solution to obtain the objective polypeptides, receptors or partial peptides, or amides thereof.

For condensation of the protected amino acids described above, a variety of activation reagents for polypeptide synthesis may be used, but carbodiimides are particularly preferably employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents that are known to be usable for polypeptide condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxan, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to polypeptide bond-forming reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower ($C_{1-6}$) alkanoyl group, such as acetyl group, an aroyl group such as benzoyl group, and a group derived from carbonic acid such as benzyloxycarbonyl group and ethoxycarbonyl group. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting amino acids include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)], etc. As the activated amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group for the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the polypeptides, receptors or partial peptides of the present invention, for example, the α-carboxyl group of the carboxy terminal amino acid is first protected by amidation; the peptide (polypeptide) chain is then extended from the amino group side to a desired length. Thereafter, a polypeptide in which only the protecting group of the N-terminal α-amino group of the peptide chain has been eliminated from the polypeptide and a polypeptide in which only the protecting group of the C-terminal carboxyl group has been eliminated are prepared. The two polypeptides are condensed in a mixture of the solvents described above. The details of the condensation reaction are the same as described above. After the protected polypeptide obtained by the condensation is purified, all the protecting groups are eliminated by the method described above to obtain the desired crude polypeptide. This crude polypeptide is purified by various known purification means. Lyophilization of the major fraction gives the amide of the desired polypeptides, receptors or partial peptides thereof.

To prepare the esterified polypeptides, receptors or partial peptides of the present invention, for example, the α-carboxyl group of the carboxy terminal amino acid is condensed with a desired alcohol to prepare the amino acid ester, which is followed by procedure similar to the preparation of the amidated polypeptides, receptors or partial peptides to give the desired esterified polypeptides, receptors or partial peptides thereof.

The polypeptides, receptors or partial peptides of the present invention can be manufactured by publicly known methods for peptide synthesis; or the partial peptides of the receptors may be manufactured by cleaving the receptors with an appropriate peptidase. For the peptide synthesis, for example, either solid phase synthesis or liquid phase synthesis may be used. That is, the partial peptides or amino acids that can construct the polypeptides, receptors or partial peptides of the present invention are condensed with the remaining part. Where the product contains protecting groups, these protecting groups are removed to give the desired peptide. Publicly known methods for condensation and elimination of the protecting groups are described in (a) - (e) below.

(a) M. Bodanszky & M.A. Ondetti: Peptide Synthesis, Interscience Publishers, New York (1966)
(b) Schroeder & Luebke: The Peptide, Academic Press, New York (1965)
(c) Nobuo Izumiya, et al.: Peptide Gosei-no-Kiso to Jikken (Basics and experiments of peptide synthesis), published by Maruzen Co. (1975)
(d) Haruaki Yajima & Shunpei Sakakibara: Seikagaku Jikken Koza (Biochemical Experiment) 1, Tanpakushitsu no Kagaku (Chemistry of Proteins) IV, 205 (1977)
(e) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography, recrystallization, etc. to give the polypeptides, receptors or partial peptides of the present invention. When the polypeptides, receptors or partial peptides obtained by the above methods is in a free form, they may be converted into appropriate salts by publicly known methods or modifications thereof; when they are obtained in a salt form, they may be converted into their free form or in the form of different salts by publicly known methods or modifications thereof.

For the DNA encoding the polypeptides, receptors or partial peptides of the present invention, any DNA can be used so long as it contains the base sequence encoding the polypeptides, receptors or partial peptides of the present invention described above. The DNA may be any of genomic DNA, genomic DNA library, cDNA derived from the cells/ tissues described above, cDNA library derived from the cells/tissues described above, and synthetic DNA.

The vector to be used for the library may be any of bacteriophage, plasmid, cosmid, phagemid, and the like. In addition, the DNA can be directly amplified by reverse transcriptase polymerase chain reaction (hereinafter abbreviated as RT-PCR) with total RNA or mRNA fraction prepared from the above-described cells or tissues.

The DNA encoding the polypeptide of the present invention may be any DNA, so long as the DNA is, for example, (a) a DNA comprising the base sequence represented by SEQ ID NO: 3, SEQ ID NO: 4, SEQ ID NO: 13, SEQ ID NO: 14, SEQ ID NO: 15, SEQ ID NO: 16, SEQ ID NO: 17, SEQ ID NO: 18, SEQ ID NO: 26, SEQ ID NO: 27, SEQ ID NO: 32, SEQ ID NO: 33, SEQ ID NO: 38, SEQ ID NO: 39, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69,SEQ ID NO: 70 or SEQ ID NO: 72, (b) a DNA having a base sequence hybridizable under high stringent conditions to the base sequence represented by SEQ ID NO: 3, SEQ ID NO: 4, SEQ ID NO: 13, SEQ ID NO: 14, SEQ ID NO: 15, SEQ ID NO: 16, SEQ ID NO: 17, SEQ ID NO: 18, SEQ ID NO: 26, SEQ ID NO: 27, SEQ ID NO: 32, SEQ ID NO: 33, SEQ ID NO: 38, SEQ ID NO: 39, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69,SEQ ID NO: 70 or SEQ ID NO: 72 and encoding a polypeptide having the activity which is substantially equivalent to that of the polypeptide of the present invention, (c) a DNA comprising the base sequence represented by SEQ ID NO: 5, SEQ ID NO: 19, SEQ ID NO: 22, SEQ ID NO: 28 or SEQ ID NO: 34, or (d) a DNA having a base sequence hybridizable to the base sequence represented by SEQ ID NO: 5, SEQ ID NO: 19, SEQ ID NO: 22, SEQ ID NO: 28 or SEQ ID NO:

34 under high stringent conditions; etc.

As the DNA that is hybridizable under high stringent conditions (i) to the base sequence represented by SEQ ID NO: 3, SEQ ID NO: 4, SEQ ID NO: 13, SEQ ID NO: 14, SEQ ID NO: 15, SEQ ID NO: 16, SEQ ID NO: 17, SEQ ID NO: 18, SEQ ID NO: 26, SEQ ID NO: 27, SEQ ID NO: 32, SEQ ID NO: 33, SEQ ID NO: 38, SEQ ID NO: 39, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69,SEQ ID NO: 70 or SEQ ID NO: 72, or (ii) to the base sequence represented by SEQ ID NO: 5, SEQ ID NO: 19, SEQ ID NO: 22, SEQ ID NO: 28 or SEQ ID NO: 34, there may be used DNAs comprising base sequences having at least about 70% homology, preferably at least about 80% homology, more preferably at least about 90% homology and most preferably at least about 95% homology, (i) to the base sequence represented by SEQ ID NO: 3, SEQ ID NO: 4, SEQ ID NO: 13, SEQ ID NO: 14, SEQ ID NO: 15, SEQ ID NO: 16, SEQ ID NO: 17, SEQ ID NO: 18, SEQ ID NO: 26, SEQ ID NO: 27, SEQ ID NO: 32, SEQ ID NO: 33, SEQ ID NO: 38, SEQ ID NO: 39, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69,SEQ ID NO: 70 or SEQ ID NO: 72 or (ii) to the base sequence represented by SEQ ID NO: 5, SEQ ID NO: 19, SEQ ID NO: 22, SEQ ID NO: 28 or SEQ ID NO: 34; and the like.

The hybridization can be carried out by publicly known methods or by modifications thereof, for example, according to the method described in Molecular Cloning, 2nd (J. Sambrook et al., Cold Spring Harbor Lab. Press, 1989), etc. A commercially available library may also be used according to the instructions of the attached manufacturer's protocol. The hybridization can be carried out preferably under high stringent conditions.

The high stringent conditions used herein are, for example, those in a sodium concentration at approximately 19 to 40 mM, preferably approximately 19 to 20 mM at a temperature of approximately 50 to 70°C, preferably approximately 60 to 65°C. In particular, hybridization conditions in a sodium concentration at about 19 mM at a temperature of about 65°C are most preferred.

More specifically;

(i) a DNA comprising the base sequence represented by SEQ ID NO: 3 or the like is used as the DNA encoding the polypeptide comprising the amino acid sequence represented by SEQ ID NO: 1;
(ii) a DNA comprising the base sequence represented by SEQ ID NO: 4 or the like is used as the DNA encoding the polypeptide comprising the amino acid sequence represented by SEQ ID NO: 2;
(iii) a DNA comprising the base sequence represented by SEQ ID NO: 13 or the like is used as the DNA encoding the polypeptide comprising the amino acid sequence represented by SEQ ID NO: 7;
(iv) a DNA comprising the base sequence represented by SEQ ID NO: 14 or the like is used as the DNA encoding the polypeptide comprising the amino acid sequence represented by SEQ ID NO: 8;
(v) a DNA comprising the base sequence represented by SEQ ID NO: 15 or the like is used as the DNA encoding the polypeptide comprising the amino acid sequence represented by SEQ ID NO: 9;
(vi) a DNA comprising the base sequence represented by SEQ ID NO: 16 or the like is used as the DNA encoding the polypeptide comprising the amino acid sequence represented by SEQ ID NO: 10;
(vii) a DNA comprising the base sequence represented by SEQ ID NO: 17 or the like is used as the DNA encoding the polypeptide comprising the amino acid sequence represented by SEQ ID NO: 11;
(viii) a DNA comprising the base sequence represented by SEQ ID NO: 18 or the like is used as the DNA encoding the polypeptide comprising the amino acid sequence represented by SEQ ID NO: 12;
(ix) a DNA comprising the base sequence represented by SEQ ID NO: 26 or the like is used as the DNA encoding the polypeptide comprising the amino acid sequence represented by SEQ ID NO: 24;
(x) a DNA comprising the base sequence represented by SEQ ID NO: 27 or the like is used as the DNA encoding the polypeptide comprising the amino acid sequence represented by SEQ ID NO: 25;
(xi) a DNA comprising the base sequence represented by SEQ ID NO: 32 or the like is used as the DNA encoding the polypeptide comprising the amino acid sequence represented by SEQ ID NO: 30;
(xii) a DNA comprising the base sequence represented by SEQ ID NO: 33 or the like is used as the DNA encoding the polypeptide comprising the amino acid sequence represented by SEQ ID NO: 31;
(xiii) a DNA comprising the base sequence represented by SEQ ID NO: 38 or the like is used as the DNA encoding the polypeptide comprising the amino acid sequence represented by SEQ ID NO: 36;
(xiv) a DNA comprising the base sequence represented by SEQ ID NO: 39 or the like is used as the DNA encoding the polypeptide comprising the amino acid sequence represented by SEQ ID NO: 37;
(xv) a DNA comprising the base sequence represented by SEQ ID NO: 3 or the like is used as the DNA encoding the polypeptide comprising the amino acid sequence represented by SEQ ID NO: 40;
(xvi) a DNA comprising the base sequence represented by SEQ ID NO: 59 or the like is used as the DNA encoding the polypeptide comprising the amino acid sequence represented by SEQ ID NO: 41;
(xvii) a DNA comprising the base sequence represented by SEQ ID NO: 60 or the like is used as the DNA encoding the polypeptide comprising the amino acid sequence represented by SEQ ID NO: 42;
(xviii) a DNA comprising the base sequence represented by SEQ ID NO: 61 or the like is used as the DNA encoding the polypeptide comprising the amino acid sequence represented by SEQ ID NO: 43;
(xix) a DNA comprising the base sequence represented by SEQ ID NO: 62 or the like is used as the DNA encoding the polypeptide comprising the amino acid sequence represented by SEQ ID NO: 44;
(xx) a DNA comprising the base sequence represented by SEQ 1D NO: 63 or the like is used as the DNA encoding the polypeptide comprising the amino acid sequence represented by SEQ ID NO: 45;
(xxi) a DNA comprising the base sequence represented by SEQ ID NO: 64 or the like is used as the DNA encoding the polypeptide comprising the amino acid sequence represented by SEQ ID NO: 46;
(xxii) a DNA comprising the base sequence represented by SEQ ID NO: 65 or the like is used as the DNA encoding the polypeptide comprising the amino acid sequence represented by SEQ ID NO: 47;
(xxiii) a DNA comprising the base sequence represented by SEQ ID NO: 26 or the like is used as the DNA encoding the polypeptide comprising the amino acid sequence represented by SEQ ID NO: 48;
(xxiv) a DNA comprising the base sequence represented by SEQ ID NO: 32 or the like is used as the DNA encoding the polypeptide comprising the amino acid sequence represented by SEQ ID NO: 49;
(xxv) a DNA comprising the base sequence represented by SEQ ID NO: 3 or the like is used as the DNA encoding the polypeptide comprising the amino acid sequence represented by SEQ ID NO: 50;
(xxvi) a DNA comprising the base sequence represented by SEQ ID NO: 3 or the like is used as the DNA encoding the polypeptide comprising the amino acid sequence represented by SEQ ID NO: 51;
(xxvii) a DNA comprising the base sequence represented by SEQ ID NO: 66 or the like is used as the DNA encoding the polypeptide comprising the amino acid sequence represented by SEQ ID NO: 52;
(xxviii) a DNA comprising the base sequence represented by SEQ ID NO: 67 or the like is used as the DNA encoding the polypeptide comprising the amino acid sequence represented by SEQ ID NO: 53;
(xxix) a DNA comprising the base sequence represented by SEQ ID NO: 68 or the like is used as the DNA encoding the polypeptide comprising the amino acid sequence represented by SEQ ID NO: 54;
(xxx) a DNA comprising the base sequence represented by SEQ ID NO: 69 or the like is used as the DNA encoding the polypeptide comprising the amino acid sequence represented by SEQ ID NO: 55;
(xxxi) a DNA comprising the base sequence represented by SEQ ID NO: 70 or the like is used as the DNA encoding the polypeptide comprising the amino acid sequence represented by SEQ ID NO: 21;
(xxxii) a DNA comprising the base sequence represented by SEQ ID NO: 66 or the like is used as the DNA encoding the polypeptide comprising the amino acid sequence represented by SEQ ID NO: 56;
(xxxiii) a DNA comprising the base sequence represented by SEQ ID NO: 3 or the like is used as the DNA encoding the polypeptide comprising the amino acid sequence represented by SEQ ID NO: 57;
(xxxiv) a DNA comprising the base sequence represented by SEQ ID NO: 66 or the like is used as the DNA encoding the polypeptide comprising the amino acid sequence represented by SEQ ID NO: 58;
(xxxv) a DNA comprising the base sequence represented by SEQ ID NO: 72 or the like is used as the DNA encoding the polypeptide comprising the amino acid sequence represented by SEQ ID NO: 71; and the like.

The DNA encoding the receptor of the present invention includes, for example, (1) a DNA having the base sequence represented by SEQ ID NO: 74, or a DNA having a base sequence hybridizable to the base sequence represented by SEQ ID NO: 74 under high stringent conditions and encoding a protein having an activity substantially equivalent to that of a protein comprising the amino acid sequence represented by SEQ ID NO: 73, (2) a DNA comprising the base sequence represented by SEQ ID NO: 76, or a DNA having a base sequence hybridizable to the base sequence represented by SEQ ID NO: 76 under high stringent conditions and encoding a protein having an activity substantially equivalent to that of a protein comprising the amino acid sequence represented by SEQ ID NO: 75, (3) a DNA comprising the base sequence represented by SEQ ID NO: 78, or a DNA having a base sequence hybridizable to the base sequence represented by SEQ ID NO: 78 under high stringent conditions and encoding a protein having an activity substantially equivalent to that of a protein comprising the amino acid sequence represented by SEQ ID NO: 77, (4) a DNA having the base sequence represented by SEQ ID NO: 80, or a DNA having a base sequence hybridizable to the base sequence represented by SEQ ID NO: 80 under high stringent conditions and encoding a protein having an activity substantially equivalent to that of a protein comprising the amino acid sequence represented by SEQ ID NO: 79 and the like. Any of such DNAs may be employed.

Examples of the DNA that is hybridizable to the base sequence represented by SEQ ID NO: 74, SEQ ID NO: 76, SEQ ID NO: 78 or SEQ ID NO: 80 include a DNA comprising a base sequence having at least about 70% homology, preferably at least about 80% homology, more preferably at least about 90% homology and most preferably at least about 95% homology, to the base sequence represented by SEQ ID NO: 74, SEQ ID NO: 76, SEQ ID NO: 78 or SEQ ID NO: 80, and the like.

The hybridization can be carried out by publicly known methods or by modifications thereof, for example, according to the method described in Molecular Cloning, 2nd (J. Sambrook et al., Cold Spring Harbor Lab. Press, 1989), etc. A commercially available library may also be used according to the instructions of the attached manufacturer's protocol. The hybridization can be carried out preferably under high stringent conditions.

The high stringent conditions used herein are, for example, those in a sodium concentration at approximately 19 to 40 mM, preferably approximately 19 to 20 mM at a temperature of approximately 50 to 70°C, preferably approximately 60 to 65°C. In particular, hybridization conditions in a sodium concentration at about 19 mM at a temperature of about 65°C are most preferred.

More specifically, a DNA comprising the base sequence represented by SEQ ID NO: 74, or the like is used as the DNA encoding the protein comprising the amino acid sequence represented by SEQ ID NO: 73; a DNA comprising the base sequence represented by SEQ ID NO: 76, or the like is used as the DNA encoding the protein comprising the amino acid sequence represented by SEQ ID NO: 75; a DNA comprising the base sequence represented by SEQ ID NO: 78, or the like is used as the DNA encoding the protein comprising the amino acid sequence represented by SEQ ID NO: 77, a DNA comprising the base sequence represented by SEQ ID NO: 80, or the like is used as the DNA encoding the protein comprising the amino acid sequence represented by SEQ ID NO: 79; etc.

As the DNA encoding the partial peptide of the receptor of the present invention, any DNA can be used, so far as it contains a base sequence encoding the partial peptide of the receptor of the present invention described above. The DNA may be any of genomic DNA, genomic DNA library, cDNA derived from the cells/tissues described above, cDNA library derived from the cells/tissues described above and synthetic DNA.

The DNA encoding the partial peptide of the receptor of the present invention includes, for example, (1) a DNA having a partial base sequence of DNA containing the base sequence represented by SEQ ID NO: 74, or a DNA having a base sequence hybridizable to the base sequence represented by SEQ ID NO: 74 under high stringent conditions and having a partial base sequence of DNA encoding a protein having an activity substantially equivalent to that of a protein having the amino acid sequence represented by SEQ ID NO: 73, (2) a DNA having a partial base sequence of DNA containing the base sequence represented by SEQ ID NO: 76, or a DNA having a base sequence hybridizable to the base sequence represented by SEQ ID NO: 76 under high stringent conditions and having a partial base sequence of DNA encoding a protein having an activity substantially equivalent to that of a protein having the amino acid sequence represented by SEQ ID NO: 75, (3) a DNA having a partial base sequence of DNA containing the base sequence represented by SEQ ID NO: 78, or a DNA having a base sequence hybridizable to the base sequence represented by SEQ ID NO: 77 under high stringent conditions and having a partial base sequence of DNA encoding a protein having an activity substantially equivalent to that of a protein having the amino acid sequence represented by SEQ ID NO: 75, (4) a DNA having a partial base sequence of DNA containing the base sequence represented by SEQ ID NO: 80, or a DNA having a base sequence hybridizable to the base sequence represented by SEQ ID NO: 80 under high stringent conditions and having a partial base sequence of DNA encoding a protein having an activity substantially equivalent to that of a protein having the amino acid sequence represented by SEQ ID NO: 79, and the like.

The DNA that is hybridizable to the base sequence represented by SEQ ID NO: 74, SEQ ID NO: 76, SEQ ID NO: 78 or SEQ ID NO: 80 has the same significance as described above.

For the methods for hybridization and high stringent conditions, those described above are similarly used.

More specifically, examples of the DNA encoding the partial peptide of the receptor of the present invention include a DNA containing a DNA having a base sequence encoding a partial peptide comprising 1 or more partial amino acid sequences selected from the partial amino acid sequences of 1 (Met) - 43 (Phe), 101 (Asn) - 118 (Lys), 188 (Tyr) - 213 (Arg) and 283 (Ala) - 295 (Ile), in the amino acid sequence represented by SEQ ID NO: 73, or a DNA containing a DNA having a base sequence hybridizable to such a DNA under high stringent conditions; and the like.

The DNA encoding the polypeptide, receptor or partial peptide of the present invention may be labeled by publicly known methods. Specific examples include those labeled with an isotope, those labeled with fluorescence (labeling with, e.g., fluorescein, etc.), those biotinylated, those labeled with enzyme, etc. Preferably used is the polypeptide of the present invention, which is labeled with an isotope.

For cloning of the DNA that completely encodes the polypeptide, receptor or partial peptide of the present invention (hereinafter these polypeptides, etc. are sometimes briefly referred to as the polypeptide of the present invention in the following description of cloning and expression of the DNA encoding these polypeptides, etc.), the DNA may be either amplified by publicly known PCR using synthetic DNA primers containing a part of the base sequence of the polypeptide of the present invention, or the DNA inserted into an appropriate vector can be selected by hybridization with a labeled DNA fragment or synthetic DNA that encodes a part or entire region of the polypeptide of the present invention. The hybridization can be carried out, for example, according to the method described in Molecular Cloning, 2nd (J. Sambrook et al., Cold Spring Harbor Lab. Press, 1989), etc. The hybridization may also be performed using commercially available library in accordance with the protocol described in the attached instructions.

Conversion of the base sequence of DNA can be made by publicly known methods such as the ODA-LA PCR method, the Gapped duplex method or the Kunkel method, or modifications thereof, by using a publicly known kit available as Mutan™ -super Express Km (manufactured by Takara Shuzo Co., Ltd., trademark), Mutan™ -K (manufactured by Takara Shuzo Co., Ltd., trademark), etc.

The cloned DNA encoding the polypeptide can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector of the polypeptide of the present invention can be manufactured, for example, by (a) excising the desired DNA fragment from the DNA encoding the polypeptide of the present invention, (b) and then ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, HIV·LTR promoter, CMV promoter, HSV-TK promoter, etc.

Among them, CMV (cytomegalovirus) promoter or SRα promoter is preferably used. Where the host is bacteria of the genus Escherichia, preferred examples of the promoter include trp promoter, lac promoter, recA promoter, λPL promoter, 1pp promoter, T7 promoter, etc. In the case of using bacteria of the genus Bacillus as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin promoter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is employed as the selection marker using dhfr gene-deficient Chinese hamster cells, selection can also be made on thymidine free media.

If necessary, a signal sequence that matches with a host is added to the N-terminus of the polypeptide of the present invention. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector comprising the DNA encoding the polypeptide of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R-, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are describe in Vaughn, J. L. et al., In Vivo, 13, 213-217 (1977)), etc.

As the insect, for example, a larva of Bombyx mori, etc. can be used [Maeda et al., Nature, 315, 592 (1985)].

Examples of animal cells include monkey cell COS-7, Vero, Chinese hamster cell CHO (hereinafter referred to as CHO cell), dhfr gene deficient Chinese hamster cell CHO (hereinafter simply referred to as CHO (dhfr-) cell), mouse L cell, mouse AtT-20, mouse myeloma cell, rat GH 3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformant transformed with the expression vector containing the DNA encoding the polypeptide can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, etc. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary and desired, a chemical such as 3β-indolylacrylic acid can be added to the medium thereby to activate the promoter efficiently.

Where the bacteria belonging to the genus Escherichia are used as the host, the transformant is usually cultivated at approximately 15 to 43°C for approximately 3 to 24 hours. If necessary, the culture may be aerated or agitated.

Where the bacteria belonging to the genus Bacillus are used as the host, the transformant is cultivated generally at approximately 30 to 40°C for approximately 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to about 8. In general, the transformant is cultivated at approximately 20 to 35°C for approximately 24 to 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultivated in, for example, MEM medium containing about 5% to about 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 hours to about 60 hours and, if necessary, the culture can be aerated or agitated.

As described above, the polypeptide of the present invention can be produced in the inside, the cell membrane or outside of the transformant, etc.

The polypeptide of the present invention can be separated and purified from the culture described above, e. g., by the following procedures.

When the polypeptide of the present invention is extracted from the culture or cells, after cultivation the transformant or cell is collected by a publicly known method and suspended in an appropriate buffer. The transformant or cell is then disrupted by publicly known methods such as ultrasonication, a treatment with lysozyme and/or freeze-thaw cycling, followed by centrifugation, filtration, etc. Thus, the crude extract of the polypeptide can be obtained. The buffer used for the procedures may contain a protein modifier such as urea or guanidine hydrochloride, or a surfactant such as Triton X-100™, etc. When the polypeptide is secreted in the culture broth, after completion of the cultivation the supernatant can be separated from the transformant or cell to collect the supernatant by a publicly known method.

The supernatant or the polypeptide contained in the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reversed phase high performance liquid chromatography, etc.; a method utilizing difference in isoelectric point such as isoelectrofocusing electrophoresis; and the like.

When the polypeptide thus obtained is in a free form, it can be converted into the salt by publicly known methods or modifications thereof. On the other hand, when the polypeptide is obtained in the form of a salt, it can be converted into the free form or in the form of a different salt by publicly known methods or modifications thereof.

The polypeptide produced by the recombinant can be treated, prior to or after the purification, with an appropriate protein modifying enzyme to appropriately modify or partially remove the polypeptide. Examples of the protein-modifying enzyme include trypsin, chymotrypsin, arginyl endopeptidase, protein kinase, glycosidase and the like.

Antibodies to the polypeptide or receptor of the present invention (hereinafter sometimes simply referred to as the antibody(ies) of the present invention) may be any of polyclonal antibodies and monoclonal antibodies, as long as they are capable of recognizing the polypeptide or receptor of the present invention.

The antibodies to the polypeptide or receptor of the present invention may be manufactured by publicly known methods for manufacturing antibodies or antisera, using as antigens the polypeptide or receptor of the present invention.

[Production of monoclonal antibody]

(a) Production of monoclonal antibody-producing cells

The polypeptide or receptor of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every two to six weeks and two to ten times in total. Examples of the applicable warm-blooded animals are monkeys, rabbits, dogs, guinea pigs, mice, rats, sheep, goats and fowl, with the use of mice and rats being preferred.

In the preparation of monoclonal antibody-producing cells, a warm-blooded animal, e.g., mice, immunized with an antigen wherein the antibody titer is noted is selected, then spleen or lymph node is collected after two to five days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled polypeptide, which will be described later, with the antiserum followed by assaying the binding activity of the labeling agent bound to the antibody. The fusion may be carried out, for example, by the known method by Koehler and Milstein [Nature, 256, 495 (1975)]. Examples of the fusion promoter are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by culturing at 20 to 40°C, preferably at 30 to 37°C for 1 to 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the polypeptide (protein) as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the polypeptide labeled with a radioactive substance or an enzyme and detecting the monoclonal antibody bound to the solid phase.

The monoclonal antibody can be selected according to publicly known methods or their modifications. In general, the selection can be effected in a medium for animal cells supplemented with HAT (hypoxanthine, aminopterin and thymidine). Any selection and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like can be used for the selection and growth medium. The cultivation is carried out generally at 20 to 40°C, preferably at 37°C, for about 5 days to about 3 weeks, preferably 1 to 2 weeks, normally in 5% $CO_2$. The antibody titer of the culture supernatant of a hybridoma can be determined as in the assay for the antibody titer in antisera described above.

(b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody].

[Production of polyclonal antibody]

The polyclonal antibody of the present invention can be manufactured by publicly known methods or modifications thereof. For example, a warm-blooded animal is immunized with an immunogen (polypeptide antigen) per se, or a complex of immunogen and a carrier protein is formed and a warm-blooded animal is immunized with the complex in a manner similar to the method described above for the manufacture of monoclonal antibodies. The product containing the antibody to the polypeptide of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin, hemocyanin or the like is coupled to hapten in a carrier-to-hapten weight ratio of approximately 0.1 to 20, preferably about 1 to about 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once approximately every 2 to 6 weeks and approximately 3 to 10 times in total.

The polyclonal antibody can be collected from blood, ascites, etc., preferably blood of the warm-blooded animals immunized by the methods described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The antisense polynucleotides (preferably DNAs) (hereinafter sometimes briefly referred to as the antisense DNA) having a complementary or substantially complementary base sequence to the DNA encoding the polypeptide, receptor or its partial peptide of the present invention (hereinafter these DNAs are sometimes briefly referred to as the DNA of the present invention) can be any antisense DNA, so long as they possess a base sequence complementary or substantially complementary to that of the DNA of the present invention and capable of suppressing the expression of said DNA.

The base sequence substantially complementary to the DNA of the present invention may, for example, be a base sequence having at least about 70% homology, preferably at least about 80% homology, more preferably at least about 90% homology and most preferably at least about 95% homology, to the full-length base sequence or partial base sequence of the base sequence complementary to the DNA of the present invention (i.e., complementary strand to the DNA of the present invention). In the entire base sequence of the complementary strand to the DNA of the present invention, an antisense DNA having at least about 70% homology, preferably at least about 80% homology, more preferably at least about 90% homology and most preferably at least about 95% homology, to the complementary strand of the base sequence which encodes the N-terminal region of the polypeptide of the present invention (e.g., the base sequence around the initiation codon). These antisense DNAs can be synthesized using a publicly known DNA synthesizer, etc.

The antisense DNA of the present invention may contain altered or modified sugars, bases or linkages. The antisense DNA may also be provided in a specialized form such as liposomes, microspheres or may be applied to gene therapy or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached at the 3' or 5' ends of the nucleic acid and may be also attached through a base, sugar, or intramolecular nucleoside linkage. Other moieties may be capping groups specifically placed at the 3' or 5' ends of the nucleic acid to prevent degradation by nuclease such as exonuclease, RNase, etc. Such capping groups include, but are not limited to, hydroxyl protecting groups known in the art, including glycols such as polyethylene glycol, tetraethylene glycol and the like.

The inhibitory activity of the antisense DNA can be examined using the transformant of the present invention, the gene expression system of the present invention in vitro and in vivo, or the translation system of the peptide or receptor of the present invention in vitro and in vivo.

Hereinafter, utilities are explained with respect to (a) the polypeptide of the present invention, (b) the receptor of the present invention (hereinafter including its partial peptides), (c) the DNA of the present invention, (d) the antibody of the present invention, and (e) the antisense DNA of the present invention, etc.

(1) Agents for preventing/treating diseases with which the polypeptide of the present invention is associated and test agents for gastric secretory function

The polypeptide of the present invention possesses cell-stimulating activities of expression cells of the receptor of the present invention, e.g., GPR8, GPR7, rat TGR26, mouse TGR26, etc. and is an endogenous ligand for the receptor of the present invention. The polypeptide of the present invention has the effects of promoting gastric acid secretion, etc.

Therefore, when the polypeptide of the present invention or the DNA of the present invention involves any abnormality or deficiency, or when the receptor of the present invention or the DNA encoding the receptor involves any abnormality or deficiency, it is highly likely to cause various diseases including dyspepsia (e.g., pituitary dyspepsia, renal dyspepsia, etc.), bone metabolism disorders (e.g., osteoporosis, osteomalacia, etc.), anemia (e.g., iron deficiency anemia, etc.), or the like. Therefore, the polypeptide of the present invention or the DNA of the present invention are usable as, e.g., gastric acid secretion promoters and can be used as agents for preventing/treating, e.g., dyspepsia (e.g., pituitary dyspepsia, renal dyspepsia, etc.), bone metabolism disorders (e.g., osteoporosis, osteomalacia, etc.), anemia (e.g., iron deficiency anemia, etc.), or the like. Furthermore, the polypeptide of the present invention can be used as test agents for gastric secretory function.

When a patient has a reduced level of, or deficient in the polypeptide of the present invention in his or her body, the polypeptide of the present invention and the DNA of the present invention can provide a role of the polypeptide of the present invention sufficiently or properly for the patient, (a) by administering the DNA of the present invention to the patient to express the polypeptide of the present invention in the body, (b) by inserting the DNA of the present invention into a cell, expressing the polypeptide of the present invention and then transplanting the cell to the patient, or (c) by administering the polypeptide of the present invention to the patient, or the like.

When the DNA of the present invention is used as the preventive/therapeutic agents described above, the DNA is administered directly to human or other warm-blooded animal; alternatively, the DNA is inserted into an appropriate vector such as retrovirus vector, adenovirus vector, adenovirus-associated virus vector, etc. and then administered to human or other warm-blooded animal in a conventional manner. The DNA of the present invention may also be administered as naked DNA, or with adjuvants to assist its uptake by gene gun or through a catheter such as a catheter with a hydrogel.

Where the polypeptide of the present invention is used as the aforesaid preventive/therapeutic agents, the polypeptide is advantageously used on a purity level of at least 90%, preferably at least 95%, more preferably at least 98% and most preferably at least 99%.

The polypeptide of the present invention can be used orally, for example, in the form of tablets which may be sugar coated if necessary and desired, capsules, elixirs, microcapsules etc., or parenterally (preferably subcutaneous administration) in the form of injectable preparations such as a sterile solution and a suspension in water or with other pharmaceutically acceptable liquid. These preparations can be manufactured by mixing the polypeptide of the present invention with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin, alginic acid, etc., a lubricant such as magnesium stearate, a sweetening agent such as sucrose, lactose and saccharin, and a flavoring agent such as peppermint, akamono oil or cherry, etc. When the unit dosage is in the form of capsules, liquid carriers such as oils and fats may further be used together with the additives described above. A sterile composition for injection may be formulated according to a conventional manner used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol. The agent may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

The vector in which the DNA of the present invention is inserted may also be prepared into pharmaceutical preparations in a manner similar to the procedures above. Such preparations are generally used parenterally.

Since the thus obtained pharmaceutical preparation is safe and low toxic, the preparation can be administered to human or other warm-blooded animal (e.g., rat, mouse, guinea pig, rabbit, fowl, sheep, swine, bovine, horse, cat, dog, monkey, etc.).

The dose of the polypeptide of the present invention varies depending on target disease, subject to be administered, route for administration, etc. Where the polypeptide of the present invention is subcutaneously administered for the treatment of, e.g., osteoporosis, the polypeptide is administered normally at a dose of about 0.1 mg to about 100 mg, preferably about 1.0 to about 50 mg, and more preferably about 1.0 to about 20 mg per day for adult (as 60 kg body weight). For other animal species, the corresponding dose as converted per 60 kg body weight can be administered.

The polypeptide of the present invention can be used as a test agent for gastric secretory function. The polypeptide of the present invention is administered to a subject, and the gastric secretory activity is assayed using BAO (basal acid output), MAO (maximal acid output), etc. as indicators. This assay clarifies a residual level of the function of gastric secretory cells, an atrophy level of gastric cells, etc. and can be an indicator both for estimating therapeutic effects of duodenal ulcer, reflux esophagitis, gastritis, gastric ulcer, atrophic gastritis, gastric cancer, etc., and for follow-up, relapse and prophylaxis and for determining the range of operation.

(2) Screening of drug candidate compounds having the effects of promoting or inhibiting gastric acid secretion

Since the polypeptide of the present invention has a function to act as the ligand for the receptor of the present invention, the compound or its salts that promote the function/activity of the polypeptide of the present invention (having the activity of promoting gastric acid secretion, etc.) are useful as gastric acid secretion promoters, etc. and can be used as agents for preventing/treating dyspepsia (e.g., pituitary dyspepsia, renal dyspepsia, etc.), bone metabolism disorders (e.g., osteoporosis, osteomalacia, etc.), anemia (e.g., iron deficiency anemia, etc.), or the like. Furthermore, the compound or its salts can be used as test agents for gastric secretory function.

On the other hand, the compound or its salts that inhibit the function/activity of the polypeptide of the present invention (the activity of promoting gastric acid secretion, etc.) are useful as, e.g., gastric acid secretion inhibitors, mucosa protectants, mineral absorption promoters, etc. and can be used as agents for preventing/treating, for example, upper gastrointestinal diseases [for example, peptic ulcers (e.g., gastric ulcer, duodenal ulcer, marginal ulcer, Zollinger-Ellison syndrome, etc.), gastritis, reflux esophagitis, NUD (non-ulcer dyspepsia), gastric cancer, gastric MALT lymphoma, nonsteroidal anti-inflammatory drug-induced ulcer, postoperative, stress-induced hyperacidity and ulcer, etc.], antibacterial agents to Helicobacter pylori, or the like.

By using the polypeptide of the present invention, or by constructing the expression system of recombinant polypeptide of the present invention and using the receptor-binding assay system via the expression system, screening can be performed efficiently on the compound (the compound that promotes or inhibits the activity of the polypeptide of the present invention) or its salts that change the binding property of the polypeptide of the present invention to its receptor (e.g., peptide, protein, a non-peptide compound, a synthetic compound, fermentation product, etc.). Such compounds include compounds (i.e., agonists for the receptor of the polypeptide of the present invention) that have the cell-stimulating activity (e.g., the activity that promotes arachidonic acid release, acetylcholine release, intracellular $Ca^{2+}$ release, intracellular cAMP production/suppression, intracellular cGMP production, inositol phosphate production, change in cell membrane potential, phosphorylation of intracellular proteins, activation of c-fos, pH reduction, GTPγS binding activity, etc.) mediated by the receptors; compounds that do not have the cell-stimulating activity (i.e., antagonists to the receptor of the polypeptide of the present invention); and the like. The term "alters the binding property to the polypeptide of the present invention" is used to include both cases where binding to the polypeptide of the present invention is inhibited and binding to the polypeptide of the present invention is promoted.

A specific example of the method of screening the compound or its salts that promote or inhibit the activity of the polypeptide of the present invention, which comprises using the polypeptide of the present invention, includes a method of screening a compound (a compound that promotes or inhibit the activity of the polypeptide of the present invention) or its salts that change the binding property of the polypeptide of the present invention to its receptor, which comprises comparing (i) the case wherein the polypeptide of the present invention is brought in contact with the receptor of the present invention or its partial peptide (hereinafter they are sometimes merely referred to as the receptor of the present invention) and (ii) the case wherein the polypeptide of the present invention and a test compound are brought in contact with the receptor of the present invention.

According to the screening method of the present invention, the method comprises assaying, for example, the binding amount of the polypeptide to the receptor of the present invention, the cell-stimulating activity, or the like, (i) when the polypeptide of the present invention is brought in contact with the receptor of the present invention described above and (ii) when the polypeptide of the present invention and a test compound are brought in contact with the receptor of the present invention described above, and comparing (i) and (ii).

Specifically, the screening method of the present invention further includes:

(a) a method of screening a compound that changes the binding property of the polypeptide of the present invention to the receptor of the present invention (a compound that promotes or inhibits the activity of the polypeptide of the present invention) or its salt, which comprises assaying the binding amount of a labeled form of the polypeptide of the present invention to the receptor of the present invention, (i) in the case wherein a labeled form of the polypeptide of the present invention is brought in contact with the receptor of the present invention above and (ii) in the case wherein a labeled form of the polypeptide of the present invention and a test compound are brought in contact with the receptor of the present invention, and comparing (i) and (ii);

(b) a method of screening a compound that changes the binding property of the polypeptide of the present invention to the receptor of the present invention (a compound that promotes or inhibits the activity of the polypeptide of the present invention) or its salt, which comprises assaying the binding amount of a labeled form of the polypeptide of the present invention to a cell containing the receptor of the present invention or a membrane fraction of said cell, (i) in the case wherein a labeled form of the polypeptide of the present invention is brought in contact with a cell containing the receptor of the present invention or a membrane fraction of said cell and (ii) in the case wherein a labeled form of the polypeptide of the present invention and a test compound are brought in contact with a cell containing the receptor of the present invention or its membrane fraction, and comparing (i) and (ii);

(c) a method of screening a compound that changes the binding property of the polypeptide of the present invention to the receptor of the present invention (a compound that promotes or inhibits the activity of the polypeptide of the present invention) or its salt, which comprises assaying the binding amount of a labeled form of the polypeptide of the present invention to the receptor of the present invention, (i) in the case wherein a labeled form of the polypeptide of the present invention is brought in contact with the receptor of the polypeptide of the present invention expressed on a cell membrane by culturing a transformant containing a DNA encoding the receptor of the present invention and (ii) in the case wherein a labeled form of the polypeptide of the present invention and a test compound are brought in contact with the receptor of the present invention expressed on a cell membrane by culturing a transformant containing a DNA encoding the receptor of the present invention, and comparing (i) and (ii);

(d) a method of screening a compound that changes the binding property of the polypeptide of the present invention to the receptor of the present invention (a compound that promotes or inhibits the activity of the polypeptide of the present invention) or its salt, which comprises assaying the cell-stimulating activity mediated by the receptor of the present invention (e.g., the activity that promotes or suppresses arachidonic acid release, acetylcholine release, intracellular $Ca^{2+}$ release, intracellular cAMP production/suppression, intracellular cGMP production, inositol phosphate production, change in cell membrane potential, phosphorylation of intracellular proteins, activation of c-fos, pH reduction, GTPγS binding activity, etc.), when a compound that activates the receptor of the present invention (e.g., the polypeptide of the present invention) is brought in contact with a cell containing the receptor of the present invention and when the compound that activates the receptor of the present invention and a test compound are brought in contact with a cell containing the receptor of the present invention, and comparing the activity; and,

(e) a method of screening a compound that changes the binding property of the polypeptide of the present invention to the receptor of the present invention (a compound that promotes or inhibits the activity of the polypeptide of the present invention) or its salt, which comprises assaying the cell-stimulating activity mediated by the receptor of the present invention (e.g., the activity that promotes or suppresses arachidonic acid release, acetylcholine release, intracellular $Ca^{2+}$ release, intracellular cAMP production/suppression , intracellular cGMP production, inositol phosphate production, change in cell membrane potential, phosphorylation of intracellular proteins, activation of c-fos, pH reduction, $GTP_{\gamma}S$ binding activity, etc.), when a compound that activates the receptor of the present invention (e.g., the polypeptide of the present invention, etc.) is brought in contact with the receptor of the present invention expressed on a cell membrane by culturing a transformant containing a DNA encoding the receptor of the present invention and when the compound that activates the receptor of the present invention and a test compound are brought in contact with the receptor of the present invention expressed on a cell membrane by culturing a transformant containing a DNA encoding the receptor of the present invention, and comparing the activity; etc.

Preferred examples of the labeled polypeptide of the present invention include polypeptides having the amino acid sequence represented by SEQ ID NO: 1, SEQ ID NO: 2, SEQ ID NO: 7, SEQ ID NO: 8, SEQ ID NO: 9, SEQ ID NO: 10, SEQ m NO: 11, SEQ ID NO: 12, SEQ ID NO: 21, SEQ ID NO: 24, SEQ ID NO: 25, SEQ ID NO: 30, SEQ ID NO: 31, SEQ ID NO: 36, SEQ ID NO: 37, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58 or SEQ ID NO: 71, and the like, each of which is labeled with [$^{125}$I].

The screening method will be described below more specifically.

First, the receptor of the present invention, which is used for the screening method of the present invention, may be any receptor, so long as it recognizes the polypeptide of the present invention as a ligand, and membrane fractions from human or other warm-blooded animal organs are preferably employed. However, it is very difficult to obtain human-derived organs especially, and the receptor of the present invention, etc. expressed abundantly by use of recombinants are suitable for use in the screening.

In producing the receptor of the present invention, the manufacturing methods described above, etc. may be used.

Where the cells containing the receptor of the present invention or membrane fractions of the cells are used in the screening method of the present invention, they are prepared according to the procedures later described.

Where cells containing the receptor of the present invention are used, the cells may be fixed with glutaraldehyde, formalin, etc. The fixation may be carried out by a publicly known method.

The cell containing the receptor of the present invention refers to a host cell in which the receptor of the present invention has been expressed. Examples of such a host cell include Escherichia coli, Bacillus subtilis, yeast, insect cells, animal cells, etc. Host cells in which the receptor of the present invention has been expressed may be prepared in a manner similar to the above-stated method for manufacturing transformants transformed by expression vectors containing the polypeptide of the present invention.

The membrane fraction refers to a fraction that abundantly contains cell membranes prepared by publicly known methods after disrupting cells. Examples of the cell disruption include cell squashing using a Potter-Elvehjem homogenizer, disruption using a Waring blender or Polytron (manufactured by Kinematica Inc.), disruption by ultrasonication, disruption by cell spraying via a thin nozzle under increasing pressure using a French press, etc., and the like. Cell membranes are fractionated mainly by fractionation using a centrifugal force such as for fractionation centrifugation, density gradient centrifugation, etc. For example, cell disruption fluid is centrifuged at a low rate (500 rpm to 3,000 rpm) for a short period of time (normally about 1 minute to about 10 minutes), the resulting supernatant is then centrifuged at a higher rate (15,000 rpm to 30,000 rpm) normally for 30 minutes to 2 hours. The precipitate thus obtained is used as the membrane fraction. The membrane fraction is rich in the receptor of the present invention expressed and membrane components such as cell-derived phospholipids, membrane proteins, or the like.

The amount of the receptor of the present invention contained in the cells containing the receptor of the present invention or in the membrane fraction is preferably $10^3$ to $10^8$ molecules per cell, more preferably $10^5$ to $10^7$ molecules per cell. As the amount of expression increases, the ligand binding activity per unit of membrane fraction (specific activity) increases so that not only the highly sensitive screening system can be constructed but also large quantities of samples can be assayed with the same lot.

To perform the methods (a) through (c) for screening the compound that changes the binding property of the polypeptide of the present invention to the receptor of the present invention (the compound that promotes or inhibits the activity of the polypeptide of the present invention), an appropriate fraction of the receptor of the present invention and a labeled form of the polypeptide of the present invention, etc. are required. The fraction of the receptor of the present invention is preferably a fraction of a naturally occurring form of the receptor of the present invention or a fraction of a recombinant type of the receptor of the present invention having an equivalent activity. Herein, the term equivalent activity is intended to mean an equivalent ligand binding activity, etc. As the labeled polypeptide, there may be used polypeptides labeled with, e.g., [$^3$H], [$^{125}$I], [$^{14}$C], [$^{35}$S], etc. Of these, [$^{125}$I]-labeled polypeptide is preferred.

Specifically, the compound that changes the binding property of the polypeptide of the present invention to the receptor of the present invention is screened by the following procedures. First, a receptor preparation is prepared by suspending cells containing the receptor of the present invention or the membrane fraction thereof in a buffer appropriate for use in the screening method. Any buffer can be used so long as it does not interfere the ligand-receptor binding, including a phosphate buffer or a Tris-HCl buffer, having pH of 4 to 10 (preferably pH of 6 to 8), etc. For the purpose of minimizing non-specific binding, a surfactant such as CHAPS, Tween-80™ (Kao-Atlas Inc.), digitonin, deoxycholate, etc., may optionally be added to the buffer. Further for the purpose of suppressing the degradation of the receptor of the present invention or the polypeptide of the present invention with a protease, a protease inhibitor such as PMSF, leupeptin, E-64 (manufactured by Peptide Institute, Inc.), pepstatin, etc. may also be added. A given amount (5,000 cpm to 500,000 cpm) of the labeled polypeptide of the present invention is added to 0.01 ml to 10 ml of the receptor solution, in which $10^{-10}$ M to $10^{-7}$ M of a test compound is co-present. To determine the amount of non-specific binding (NSB), a reaction tube charged with an unlabeled form of the polypeptide of the present invention in a large excess is also provided. The reaction is carried out at approximately 0°C to 50°C, preferably 4°C to 37°C for 20 minutes to 24 hours, preferably 30 minutes to 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or γ-counter. When nonspecific binding (NSB) is subtracted from the count ($B_0$) where any antagonizing substance is absent and the resulting count ($B_0$ minus NSB) is made 100%, the test compound showing the specific binding amount (B minus NSB) of, e.g., 50% or less may be selected as a candidate compound.

The method (d) or (e) above for screening the compound that changes the binding property of the polypeptide of the present invention to the receptor of the present invention (the compound that promotes or inhibits the activity of the polypeptide of the present invention) can be performed as follows. For example, the cell stimulating activity mediated by the receptor of the present invention (e.g., the activity that promotes or suppresses arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, GTPγS binding activity, etc.) may be determined by a publicly known method, or using an assay kit commercially available. Specifically, the cells containing the receptor of the present invention are first cultured on a multiwell plate, etc. Prior to screening, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the cell-stimulating activity indicator (e.g., arachidonic acid, etc.) due to a degrading enzyme contained in the cells, an inhibitor against such as a degrading enzyme may be added prior to the assay. For detecting the activity such as the cAMP production suppression, the baseline production in the cells is increased by forskolin or the like and the suppressing effect on the increased baseline production can be detected.

For screening through the assay of the cell stimulating activity, appropriate cells, in which the receptor of the present invention has been expressed, are required. Preferred cells, in which the receptor of the present invention has been expressed, are the aforesaid cell line in which the receptor of the present invention is expressed, etc.

Examples of the test compounds include peptides, proteins, non-peptide compounds, synthetic compounds, fermentation products, cell extracts, plant extracts, animal tissue extracts, etc.

A kit for screening the compound or a salt thereof that changes the binding property of the polypeptide of the present invention (the compound that promotes or inhibits the activity of the polypeptide of the present invention) to the receptor of the present invention comprises the receptor of the present invention or its salt, a partial peptide of the receptor of the present invention or its salt, cells containing the receptor of the present invention or a membrane fraction of the cells containing the receptor of the present invention, and the polypeptide of the present invention.

Examples of the screening kit of the present invention are given below:

1. Reagent for screening

(a) Assay buffer and wash buffer

Hanks' Balanced Salt Solution (manufactured by Gibco Co.) supplemented with 0.05% bovine serum albumin (Sigma Co.).

The solution is sterilized by filtration through a 0.45 μm filter and stored at 4°C. Alternatively, the solution may be prepared at use.

(b) Preparation of the receptor of the present invention

CHO cells on which the receptor of the present invention has been expressed are subcultured in a 12-well plate at the rate of 5 x 105 cells/well and then cultured at 37°C under 5% $CO_2$ and 95% air for 2 days.

(c) Labeled ligand

The polypeptide of the present invention, which is labeled with commercially available [$^{3}$H], [$^{125}$I], [$^{14}$C], [$^{35}$S], etc., is dissolved in a suitable solvent or buffer. The solution is stored at 4°C or -20°C, which is diluted to 1 μM with an assay buffer at use.

(d) Standard ligand solution

The polypeptide of the present invention is dissolved in PBS supplemented with 0.1% bovine serum albumin (manufactured by Sigma, Inc.) in a concentration of 1 mM, and the solution is stored at -20°C.

2. Assay method

(a) Cells are cultured in a 12-well tissue culture plate to express the receptor of the present invention. After washing the cells twice with 1 ml of the assay buffer, 490 μl of the assay buffer is added to each well.
(b) After 5 μl of a test compound solution of $10^{-3}$ to $10^{-10}$ M is added, 5 μl of a labeled form of the peptide of the present invention is added to the system followed by reacting at room temperature for an hour. To determine the amount of the non-specific binding, the polypeptide of the present invention of $10^{-3}$ M is added in an amount of 5 μl, instead of the test compound.
(c) The reaction mixture is removed and washed 3 times with 1 ml each of the wash buffer. The labeled polypeptide of the present invention bound to the cells is dissolved in 0.2N NaOH-1% SDS and mixed with 4 ml of a liquid scintillator A (manufactured by Wako Pure Chemical Industries, Ltd.).
(d) Radioactivity is measured using a liquid scintillation counter (manufactured by Beckmann) and PMB (percent of the maximum binding) is calculated in accordance with the following equation 1:

$$PMB = [(B-NSB)/(B_0 - NSB)] \times 100$$

wherein:

| | |
|---|---|
| PMB | percent of the maximum binding |
| B | value when a sample is added |
| NSB | non-specific binding |
| $B_0$ | maximum binding |

The compound or its salts, which are obtainable by the screening method or the screening kit of the present invention, are compounds that change the binding (promote or inhibit the binding) of the polypeptide of the present invention to the receptor of the present invention (compounds that promote or inhibit the activity of the polypeptide of the present invention). Specifically, these compounds are compounds or salts thereof that exhibit the cell stimulating activity mediated by the receptor of the present invention (so-called agonists for the receptor of the present invention), or compounds that do not exhibit the cell stimulating activity (so-called antagonists to the receptor of the present invention). Examples of such compounds include peptides, proteins, non-peptide compounds, synthetic compounds and fermentation products. These compounds may be either novel or publicly known compounds.

In order to evaluate whether a compound is an agonist for or an antagonist to the receptor of the present invention described above, it is determined by (i) or (ii) below.
(i) According to the screening methods (a) to (c), binding assay is carried out to obtain a compound that changes the binding property of the polypeptide of the present invention to the receptor of the present invention (especially, the compound that inhibits the binding). It is then determined if the compound has the above cell-stimulating activity mediated by the receptor of the present invention. The compound having the cell-stimulating activity or its salt is an agonist for the receptor of the present invention, whereas the compound or its salt having no such an activity is an antagonist to the receptor of the present invention.
(ii)

(a) A test compound is brought in contact with a cell containing the receptor of the present invention, whereby the aforesaid cell-stimulating activity mediated by the receptor of the present invention is assayed. The compound having the cell-stimulating activity or its salt is an agonist for the receptor of the present invention.
(b) The cell-stimulating activity mediated by the receptor of the present invention is assayed in the case where a compound that activates the receptor of the present invention (e.g., an agonist for the polypeptide of the present invention or the receptor of the present invention, etc.) is brought in contact with cells containing the receptor of the present invention and in the case where the compound that activates the receptor of the present invention and a test compound are brought in contact with cells containing the receptor of the present invention, and compared therebetween. The compound or its salt that can reduce the cell-stimulating activity induced by the compound that activates the receptor of the present invention is an antagonist to the receptor of the present invention.

The agonists for the receptor of the present invention exhibit similar physiological activity of the polypeptide of the present invention on the receptor of the present invention, and thus can be used as safe and low-toxic agents for preventing/treating, for example, dyspepsia (e.g., pituitary dyspepsia, renal dyspepsia, etc.), bone metabolism disorders (e.g., osteoporosis, osteomalacia, etc.), anemia (e.g., iron deficiency anemia, etc.), or the like.

Since the antagonists to the receptor of the present invention can inhibit the physiological activity that the polypeptide of the present invention has on the receptor of the present invention, the antagonists are thus useful as gastric acid secretion inhibitors, mucosa protectants, mineral absorption promoters, etc. and can be used as safe and low-toxic agents for preventing/treating, for example, upper gastrointestinal diseases [for example, peptic ulcers (e.g., gastric ulcer, duodenal ulcer, marginal ulcer, Zollinger-Ellison syndrome, etc.), gastritis, reflux esophagitis, NUD (non-ulcer dyspepsia), gastric cancer, gastric MALT lymphoma, nonsteroidal anti-inflammatory drug-induced ulcer, postoperative, stress-induced hyperacidity and ulcer, etc.], antibacterial agents to Helicobacter pylori, or the like.

The agonist for the receptor of the present invention described above can also be used as test agents for gastric secretory function. The agonist is administered to a subject, and the gastric secretory activity is assayed using BAO (basal acid output), MAO (maximal acid output), etc. as indicators. This assay clarifies a residual level of the function of gastric secretory cells, an atrophy level of gastric cells, etc. and can be an indicator both for estimating therapeutic effects of duodenal ulcer, reflux esophagitis, gastritis, gastric ulcer, atrophic gastritis, gastric cancer, etc., and for follow-up, relapse and prophylaxis and for determining the range of operation.

The compound or its salt, which is obtainable by using the screening method or the screening kit of the present invention, is selected from, e.g., peptides, proteins, non-peptide compounds, synthetic compounds, fermentation products, cell extracts, plant extracts, animal tissue extracts, plasma, etc., and is the compound that promotes or inhibits the function of the polypeptide of the present invention.

As salts of the compound, there may be used those similar to the salts of the polypeptide of the present invention described above.

When the compound obtained by the screening method or screening kit of the present invention is used as the preventive/therapeutic agents described above, the compound can be prepared into pharmaceutical preparations in a conventional manner. For example, the compound may be prepared in the form of tablets, capsules, elixir, microcapsule, a sterile solution, a suspension, etc., as in the aforesaid drugs containing the polypeptide of the present invention.

Since the thus obtained pharmaceutical preparation is safe and low toxic, the preparation may be administered to human or other warm-blooded animal (e.g., mouse, rat, rabbit, sheep, swine, bovine, horse, fowl, cat, dog, monkey, chimpanzee, etc.).

The dose of the compound or its salt varies depending on its effect, target disease, subject to be administered, route for administration, etc.; for example, where the compound that inhibits the activity of the polypeptide of the present invention is subcutaneously administered for the treatment of reflux esophagitis, the compound is administered normally at a dose of about 0.1 to about 100 mg, preferably about 1.0 to about 50 mg, and more preferably about 1.0 to about 20 mg per day for an adult (as 60 kg body weight). For other animal species, the corresponding dose as converted per 60 kg body weight can be administered. Also, when the compound that promotes the function of the polypeptide of the present invention is subcutaneously administered to an adult (per 60 kg body weight) for the treatment of dyspepsia, the compound is administered generally at a daily dose of approximately 0.1 to 100 mg, preferably approximately 1.0 to 50 mg, and more preferably approximately 1.0 to 20 mg. For other animal species, the corresponding dose as converted per 60 kg body weight can be administered.

(3) Quantification of the polypeptide of the present invention

The antibody of the present invention is capable of specifically recognizing the polypeptide or receptor of the present invention, and can thus be used for quantification of the polypeptide or receptor of the present invention in a sample fluid, in particular, for quantification by sandwich immunoassay.

That is, the present invention provides:

(i) a method for quantification of the polypeptide of the present invention in a sample fluid, which comprises competitively reacting the antibody of the present invention with a sample fluid and a labeled form of the polypeptide or receptor of the present invention, and measuring the ratio of the labeled polypeptide of the present invention bound to said antibody; and,

(ii) a method for quantification of the polypeptide of the present invention in a sample fluid, which comprises simultaneously or continuously reacting the sample fluid with the antibody of the present invention and a labeled form of another antibody of the present invention immobilized on an insoluble carrier, and measuring the activity of the labeling agent on the immobilized carrier.

In the quantification method (ii) described above, it is preferred that one antibody is capable of recognizing the N-terminal region of the polypeptide of the present invention, while another antibody is capable of recognizing the C-terminal region of the polypeptide of the present invention.
The monoclonal antibody to the polypeptide or receptor of the present invention may be used to quantify the polypeptide or receptor of the present invention. In addition, the polypeptide may also be detected by means of a tissue staining, etc. For these purposes, the antibody molecule per se may be used, or $F(ab')_2$, Fab' or Fab fractions of the antibody molecule may be used as well.
The method of quantifying the polypeptide or receptor of the present invention using the antibody of the present invention is not particularly limited, and any method may be used so far as it relates to a method, in which the amount of an antibody, antigen or antibody-antigen complex can be detected by a chemical or a physical means, depending on or corresponding to the amount of antigen (e.g., the amount of polypeptide) in a sample fluid to be assayed, and then calculated using a standard curve prepared by a standard solution containing the known amount of antigen. Advantageously used are, for example, nephrometry, competitive method, immunometric method and sandwich method; in terms of sensitivity and specificity, the sandwich method, which will be described later, is particularly preferred.
Examples of labeling agents, which are employed for the assay method using the same, are radioisotopes (e.g., [$^{125}$I], [$^{131}$I], [$^{3}$H], [$^{14}$C], [$^{32}$P], [$^{33}$P], [$^{35}$S]), fluorescent substances (e.g., cyanine fluorescent dyes (e.g., Cy2, Cy3, Cy5, Cy5.5, Cy7 (manufactured by Amersham Bioscience), etc.), fluorescamine, fluorescein isothiocyanate), enzymes (e.g., β-galactosidase, β-glucosidase, alkaline phosphatase, peroxidase, malate dehydrogenase), luminescent substances (e.g., luminol, a luminol derivative, luciferin, lucigenin), biotin, lanthanide elements. Furthermore, a biotin-avidin system may be used as well for binding an antibody or antigen to a labeling agent.
In the immobilization of antigens or antibodies, physical adsorption may be used. Alternatively, chemical binding that is conventionally used for immobilization of polypeptides, enzymes, etc. may be used as well. Examples of the carrier include insoluble polysaccharides such as agarose, dextran, cellulose, etc.; synthetic resins such as polystyrene, polyacrylamide, silicone, etc.; or glass; and the like.
In the sandwich method, a sample fluid is reacted with an immobilized form of the monoclonal antibody of the present invention (primary reaction), then reacted with a labeled form of the monoclonal antibody of the present invention (secondary reaction) and the activity of the labeling agent on the insoluble carrier is assayed; thus, the amount of polypeptide of the present invention in a sample fluid can be determined. The primary and secondary reactions may be carried out in a reversed order, simultaneously or sequentially with intervals. The type of the labeling agent and the method of immobilization may be the same as those described hereinabove. In the immunoassay by the sandwich method, it is not always necessary that the antibody used for the labeled antibody and for the solid phase should be one type or one species but a mixture of two or more antibodies may also be used for the purpose of improving the assay sensitivity, etc.
In the method of assaying the polypeptide of the present invention by the sandwich method according to the present invention, preferred monoclonal antibodies of the present invention used for the primary and the secondary reactions are antibodies, which binding sites to the polypeptide of the present invention are different from each other. Thus, the antibodies used in the primary and secondary reactions are those wherein, when the antibody used in the secondary reaction recognizes the C-terminal region of the polypeptide of the present invention, the antibody recognizing the site other than the C-terminal regions, e.g., recognizing the N-terminal region, is preferably used in the primary reaction.
The monoclonal antibody of the present invention may be used in an assay system other than the sandwich method, such as the competitive method, the immunometric method or the nephrometry.
In the competitive method, an antigen in a sample fluid and a labeled antigen are competitively reacted with an antibody, then an unreacted labeled antigen (F) and a labeled antigen bound to the antibody (B) are separated (i. e., B/F separation) and the labeled amount of either B or F is measured to determine the amount of the antigen in the sample fluid. In the reactions for such a method, there are a liquid phase method in which a soluble antibody is used as the antibody and the B/F separation is effected by polyethylene glycol, while a second antibody to the antibody is used, and a solid phase method in which an immobilized antibody is used as the first antibody or a soluble antibody is used as the first antibody, while an immobilized antibody is used as the second antibody.
In the immunometric method, an antigen in a sample fluid and an immobilized antigen are competitively reacted with a given amount of a labeled antibody followed by separating the solid phase from the liquid phase; or an antigen in a sample fluid and an excess amount of labeled antibody are reacted, then an immobilized antigen is added to bind an unreacted labeled antibody to the solid phase and the solid phase is separated from the liquid phase. Thereafter, the labeled amount of any of the phases is measured to determine the antigen amount in the sample fluid.
In the nephrometry, the amount of insoluble sediment, which is produced as a result of the antigen-antibody reaction in a gel or in a solution, is measured. Even when the amount of an antigen in a sample fluid is small and only a small amount of the sediment is obtained, a laser nephrometry utilizing laser scattering can be suitably used.

In applying each of those immunoassays to the assay method of the present invention, any special conditions, operations, etc. are not required. The assay system for the polypeptide of the present invention may be constructed in addition to conditions or operations conventionally used for each of the methods, taking technical consideration by one skilled in the art into account. For the details of such conventional technical means, a variety of reviews, reference books, etc. may be referred to:

for example, Hiroshi Irie (ed.): "Radioimmunoassay" (published by Kodansha, 1974); Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "Methods in Enzymology" Vol. 70 (Immunochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies)) (all published by Academic Press); etc.

As described above, the polypeptide of the present invention can be quantified with high sensitivity, using the antibody of the present invention.

Furthermore when a reduced level of the polypeptide or receptor of the present invention is detected by quantifying a level of the polypeptide or receptor of the present invention using the antibody of the present invention, it can be diagnosed that one suffers from diseases, for example, dyspepsia (e.g., pituitary dyspepsia, renal dyspepsia, etc.), bone metabolism disorders (e.g., osteoporosis, osteomalacia, etc.), anemia (e.g., iron deficiency anemia, etc.), or the like, or it is highly likely for one to suffer from these diseases in the future.

When an increased level of the polypeptide or receptor of the present invention is detected, it can be diagnosed that one suffers from diseases, for example, upper gastrointestinal diseases [e.g., peptic ulcers (gastric ulcer, duodenal ulcer, marginal ulcer, Zollinger-Ellison syndrome, etc.), gastritis, reflux esophagitis, NUD (non-ulcer dyspepsia), gastric cancer, gastric MALT lymphoma, nonsteroidal anti-inflammatory drug-induced ulcer, postoperative, stress-induced hyperacidity and ulcer, etc.], or the like, or it is highly likely for one to suffer from these diseases in the future.

The antibody of the present invention can also be employed to detect the polypeptide or receptor of the present invention present in a sample fluid such as body fluids, tissues, etc. The antibody may further be used for the preparation of an antibody column used to purify the polypeptide or receptor of the present invention, detect the polypeptide or receptor of the present invention in each fraction upon purification, analysis of the behavior of the polypeptide or receptor of the present invention in the cells under investigation.

(4) Gene diagnostic agent

By using the DNA of the present invention, e.g., as a probe, abnormality (gene abnormality) of the DNA or mRNA encoding the polypeptide of the present invention in human or other warm-blooded animal (e.g., rat, mouse, guinea pig, rabbit, fowl, sheep, swine, bovine, horse, cat, dog, monkey, etc.) can be detected. Thus, the DNA of the present invention is useful as a gene diagnostic agent for the damage to the DNA or mRNA, mutation, a decreased expression or an increased expression, or overexpression of the DNA or mRNA.

The gene diagnosis described above using the DNA of the present invention can be performed by, for example, the publicly known Northern hybridization assay or the PCR-SSCP assay (Genonics, 5, 874-879 (1989); Proceedings of the National Academy of Sciences of the United States of America, 86, 2766-2770 (1989)).

When a decreased expression of the polypeptide or receptor of the present invention is detected, e.g., by the Northern hybridization, it can be diagnosed that one is likely to suffer from diseases, for example, dyspepsia (e.g., pituitary dyspepsia, renal dyspepsia, etc.), bone metabolism disorders (e.g., osteoporosis, osteomalacia, etc.), anemia (e.g., iron deficiency anemia, etc.), or the like, or it is highly likely for one to suffer from these diseases in the future.

When overexpression of the polypeptide or receptor of the present invention is detected by the Northern hybridization, it can be diagnosed that one is likely to suffer from diseases, for example, upper gastrointestinal diseases [e.g., peptic ulcers (gastric ulcer, duodenal ulcer, marginal ulcer, Zollinger-Ellison syndrome, etc.), gastritis, reflux esophagitis, NUD (non-ulcer dyspepsia), gastric cancer, gastric MALT lymphoma, nonsteroidal anti-inflammatory drug-induced ulcer, postoperative, stress-induced hyperacidity and ulcer, etc.] or the like, or it is highly likely for one to suffer from these diseases in the future.

(5) Pharmaceutical composition comprising antisense DNA

The antisense DNA of the present invention is useful as gastric acid secretion inhibitors, mucosa protectants, mineral absorption promoters, etc. and can be used as agents for preventing/treating, e.g., upper gastrointestinal diseases [for example, peptic ulcers (e.g., gastric ulcer, duodenal ulcer, marginal ulcer, Zollinger-Ellison syndrome, etc.), gastritis, reflux esophagitis, NUD (non-ulcer dyspepsia), gastric cancer, gastric MALT lymphoma, nonsteroidal anti-inflammatory drug-induced ulcer, postoperative, stress-induced hyperacidity and ulcer, etc.], antibacterial agents to Helicobacter pylori, or the like.
When the antisense DNA is used, the antisense DNA may be administered directly, or the DNA is inserted into an appropriate vector such as retrovirus vector, adenovirus vector, adenovirus-associated virus vector, etc. and then administered in a conventional manner. The antisense DNA may also be administered as intact DNA, or with adjuvants to assist its uptake by gene gun or through a catheter such as a catheter with a hydrogel.
In addition, the antisense DNA may also be employed as an oligonucleotide probe for diagnosis to examine the presence of the DNA of the present invention in tissues or cells and states of its expression.

(6) Pharmaceutical composition comprising the antibody of the present invention

The antibody of the present invention (e.g., the neutralizing antibody having the action of neutralizing the polypeptide of the present invention) is useful as gastric acid secretion inhibitors, mucosa protectants, mineral absorption promoters, etc. and can be used as agents for preventing/treating, e.g., upper gastrointestinal diseases [for example, peptic ulcers (e.g., gastric ulcer, duodenal ulcer, marginal ulcer, Zollinger-Ellison syndrome, etc.), gastritis, reflux esophagitis, NUD (non-ulcer dyspepsia), gastric cancer, gastric MALT lymphoma, nonsteroidal anti-inflammatory drug-induced ulcer, postoperative, stress-induced hyperacidity and ulcer, etc.], antibacterial agents to Helicobacter pylori, or the like.
The agents for diseases described above comprising the antibody of the present invention can be administered to human or mammal (e.g., rat, rabbit, sheep, swine, bovine, cat, dog, monkey, etc.) orally or parenterally directly as a liquid preparation, or as a pharmaceutical composition in an appropriate preparation form. The dose varies depending on subject to be administered, target disease, conditions, route for administration, etc.; in using the antibody for the treatment of the adult patient with, e.g., reflux esophagitis, it is advantageous to administer the antibody of the present invention to the patient by intravenous injection, normally in a single dose of approximately 0.01 to 20 mg/kg body weight, preferably about 0.1 to about 10 mg/kg body weight, and more preferably about 0.1 to about 5 mg/kg body weight, approximately 1 to 5 times, preferably approximately 1 to 3 times, per day; For other parenteral administration and oral administration, the corresponding dose may be administered. When the conditions are extremely serious, the dose may be increased depending on the conditions.
The antibody of the present invention may be administered directly as it is or as an appropriate pharmaceutical composition. The pharmaceutical composition used for the administration described above contains a pharmacologically acceptable carrier with the aforesaid compounds or salts thereof, a diluent or excipient. Such a composition is provided in the preparation suitable for oral or parenteral administration.
That is, examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or excipient conventionally used in the field of pharmaceutical preparations. Examples of the vehicle or excipient for tablets are lactose, starch, sucrose, magnesium stearate, etc.
Examples of the composition for parenteral administration that can be used are injections, suppositories, etc. and the injections include the form of intravenous, subcutaneous, transcutaneous, intramuscular and drip injections, etc. Such injections are prepared by publicly known methods, e.g., by dissolving, suspending or emulsifying the aforesaid antibody or its salts in a sterile aqueous or oily liquid medium. For the aqueous medium for injection, for example, physiological saline and isotonic solutions containing glucose and other adjuvant, etc. are used. Appropriate dissolution aids, for example, alcohol (e.g., ethanol), polyalcohol (e.g., propylene glycol or polyethylene glycol), nonionic surfactant [e.g., polysorbate 80, HCO-50 (polyoxyethylene (50 mol) adduct of hydrogenated castor oil)] may be used in combination. For the oily solution, for example, sesame oil, soybean oil and the like are used, and dissolution aids such as benzyl benzoate, benzyl alcohol, etc. may be used in combination. The thus-prepared liquid for injection is normally filled in an appropriate ampoule. The suppository used for rectal administration is prepared by mixing the aforesaid antibody or its salts with conventional suppository base.
The oral or parenteral pharmaceutical composition described above is advantageously prepared in a unit dosage form suitable for the dose of the active ingredient. Examples of such unit dosage form include tablets, pills, capsules, injections (ampoules), suppositories, etc. It is preferred that the antibody described above is contained generally at a dose of 5 to 500 mg per unit dosage form, 5 to 100 mg especially for injections and 10 to 250 mg for other preparations.

Each composition described above may further contain other active components unless formulation with the antibody causes any adverse interaction.

(7) DNA transgenic animal

A non-human mammal bearing an exogenous DNA encoding the polypeptide of the present invention (hereinafter merely referred to as the exogenous DNA of the present invention) or its variant DNA (sometimes simply referred to as the exogenous variant DNA of the present invention) is used to screen preventive/therapeutic agents for gastrointestinal diseases; etc.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the transgenic animal of the present invention.

Examples of the non-human mammal that can be used include bovine, swine, sheep, goats, rabbits, dogs, cats, guinea pigs, hamsters, mice, rats, and the like. Above all, preferred are rodents, especially mice (e.g., C57BL/6 strain, DBA2 strain, etc. for a pure line and for a cross line, $B6C3F_1$ strain, $BDF_1$ strain $B6D2F_1$ strain, BALB/c strain, ICR strain, etc.) or rats (Wistar, SD, etc.), since they are relatively short in ontogeny and life cycle from a standpoint of preparing model animals for human disease.

"Mammals" in a recombinant vector that can be expressed in the mammals include the aforesaid non-human mammals and human.

The exogenous DNA of the present invention refers to the DNA of the present invention that is once isolated/extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean such a DNA that expresses the abnormal polypeptide of the present invention and exemplified by the DNA that expresses a polypeptide to suppress the functions of the normal polypeptide of the present invention.

The exogenous DNA of the present invention may be any one of those derived from a mammal of the same species as, or a different species from, the mammal as the target animal. In transfecting the DNA of the present invention, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters, which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the polypeptide of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression include (a) promoters for the DNA derived from viruses (e.g., simian virus, cytomegalovirus, Moloney leukemia virus, JC virus, breast cancer virus, poliovirus, etc.), and (b) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), polypeptide chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among others them, cytomegalovirus promoters, human polypeptide elongation factor 1α (EF-1α) promoters, human and chicken β actin promoters etc., which protein can highly express in the whole body are preferred.

It is preferred that the vectors described above have a sequence for terminating the transcription of the desired messenger RNA in the DNA transgenic animal (generally called a terminator); for example, a sequence of each DNA derived from viruses and various mammals. SV40 terminator of the simian virus, etc. are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal polypeptide of the present invention can be acquired as a whole or a part of RNA derived from liver, kidney, thyroid cell or fibroblast of human or various mammals (e.g., rabbits, dogs, cats, guinea pigs, hamsters, rats, nice, etc.) or of the genomic DNA from various commercially available genomic DNA libraries, or using as a starting material complementary DNA prepared by a publicly known method from RNA derived from liver, kidney, thyroid cell or fibroblast. Also, an exogenous abnormal DNA can produce a translational region, which is obtained by point mutagenesis variation of the translational region in a normal polypeptide obtained from the cells or tissues described above.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by mating.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the exogenous DNA of the present invention is excessively present in all of the germinal cells and somatic cells thereof. The offspring of the animal that inherits the exogenous DNA of the present invention have excessively the exogenous DNA of the present invention in all of the germinal cells and somatic cells thereof.

By obtaining a homozygotic animal having the transfected DNA in both of homologous chromosomes and mating a male and female of the animal, all offspring can be passaged to retain the DNA.

A non-human mammal bearing the normal DNA of the present invention expresses the normal DNA of the present invention to a high level and may eventually develop the hyperfunction of the polypeptide of the present invention by promoting the function of endogenous normal DNA. Therefore, the animal can be utilized as a pathologic model animal for such a disease. Specifically, using the normal DNA transgenic animal of the present invention, it is possible to elucidate the mechanism of the hyperfunction of the polypeptide of the present invention and the pathological mechanism of the disease associated with the polypeptide of the present invention and to determine how to treat the disease.

Furthermore, a mammal transfected with the exogenous normal DNA of the present invention exhibits an increasing symptom of the polypeptide of the present invention in its librated form and the animal is usable for screening therapeutic agents for the disease associated with the polypeptide of the present invention.

On the other hand, non-human mammal having the exogenous abnormal DNA of the present invention can be passaged under normal breeding conditions as the DNA-bearing animal by confirming the stable retaining of the exogenous DNA via crossing. Further, the exogenous DNA to be subjected can be utilized as a starting material by inserting the DNA into the plasmid described above. The DNA construct with promoter can be prepared by conventional DNA engineering techniques. The transfection of the abnormal DNA of the present invention at the fertilized egg cell stage is preserved to be present in all of the germinal and somatic cells of the mammals to be subjected. The fact that the abnormal DNA of the present invention is present in the germinal cells of the animal after DNA transfection means that all of the offspring of the prepared animal have the abnormal DNA of the present invention in all of the germinal and somatic cells. Such an offspring passaged the exogenous DNA of the present invention contains the abnormal DNA of the present invention in all of the germinal and somatic cells. A homozygous animal having the introduced DNA on both of homologous chromosomes can be acquired and then by mating these male and female animals, all the offspring can be bled to have the DNA.

Since non-human mammal having the abnormal DNA of the present invention may express the abnormal DNA of the present invention at a high level, the animal may be the function inactivation type inadaptability to the polypeptide of the present invention by inhibiting the function of the endogenous normal DNA and can be utilized as its disease model animal. For example, using the abnormal DNA-transgenic animal of the present invention, it is possible to elucidate the mechanism of inadaptability to the polypeptide of the present invention and to perform to study a method for treatment of this disease.

More specifically, the transgenic animal of the present invention expressing the abnormal DNA of the present invention to a high level is also expected to serve as an experimental model for the elucidation of the mechanism of the functional inhibition (dominant negative effect) of normal polypeptide by the abnormal polypeptide of the present invention in the function inactive type inadaptability to the polypeptide of the present invention.

The transgenic animal bearing the abnormal exogenous DNA of the present invention is also expected to serve for screening a candidate therapeutic drug for the function inactive type inadaptability to the polypeptide of the present invention, since the animal exhibits symptoms of increasing the polypeptide of the present invention in its free form.

Other potential applications of two kinds of the transgenic animals described above include:

(a) use as a cell source for tissue culture;
(b) elucidation of the relation to a polypeptide that is specifically expressed or activated by the polypeptide of the present invention, by direct analysis of DNA or RNA in tissue of the DNA transgenic animal of the present invention or by analysis of the polypeptide tissue expressed by the DNA;
(c) research in the function of cells derived from tissues that are cultured usually only with difficulty, using cells of tissue bearing the DNA cultured by a standard tissue culture technique;
(d) screening of a drug that enhances the functions of cells using the cells described in (c) above; and,
(e) isolation and purification of the variant polypeptide of the present invention and preparation of an antibody thereto, etc.

In addition, clinical conditions of a disease associated with the polypeptide of the present invention, including the function inactive type inadaptability to the polypeptide of the present invention can be determined using the DNA transgenic animal of the present invention. Also, pathological findings on each organ in a disease model associated with the polypeptide of the present invention can be obtained in more detail, leading to the development of a new method for treatment as well as the research and therapy of any secondary diseases associated with the disease.

Also, the DNA transgenic animal of the present invention can be used to collect the released DNA-transfected cells, which involves withdrawing each organ from the animal, mincing the organ and digesting with a proteinase such as trypsin, etc., and futher used to culture the cells and establish the line of cultured cells. In addition, the DNA transgenic animal of the present invention can serve as identification of cells capable of producing the polypeptide of the present invention, and as studies on association with apoptosis, differentiation or proliferation or on the mechanism of signal transduction in these properties to inspect any abnormality therein. Thus, the DNA transgenic animal can provide an effective research material to elucidate the polypeptide of the present invention and its function and effect.

To develop a therapeutic drug for the treatment of diseases associated with the polypeptide of the present invention, including the function inactive type inadaptability to the polypeptide of the present invention, using the DNA transgenic animal of the present invention, an effective and rapid method for screening can be provided by using the method for inspection and the method for quantification, etc. described above. It is also possible to investigate and develop a method for DNA therapy for diseases associated with the polypeptide of the present invention, using the DNA transgenic animal of the present invention or a vector capable of expressing the exogenous DNA of the present invention.

(8) Knockout animal

A non-human mammal embryonic stem cell bearing the DNA of the present invention inactivated and a non-human mammal deficient in expressing the DNA of the present invention are used to screen gastric acid promoters.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the polypeptide of the present invention (hereinafter sometimes referred to as the knockout DNA of the present invention) by substantially inactivating the activity of the polypeptide of the present invention encoded by the DNA (hereinafter merely referred to as ES cell).

As the non-human mammal used, the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention the desired non-human mammal has, inserting a reporter gene, etc. including a neomycin resistant gene or a hygromycin resistant gene, or a reporter gene such as lacZ (β-galactosidase gene) or cat (chloramphenicol acetyltransferase gene), etc. into its exon site thereby to disable the functions of exon; or by inserting a DNA sequence which terminates gene transcription (e.g., polyA additional signal, etc.) in the intron between exons thereby to inhibit the synthesis of complete messenger RNA to eventually destroy the gene, transfecting a DNA strand having the thus constructed DNA strand (hereinafter simply referred to as targeting vector) to a chromosome of the subject animal by, e.g., homologous recombination. The thus obtained ES cells are analyzed by the southern hybridization analysis using a DNA sequence on or near the DNA of the present invention as a probe, or by PCR analysis using a DNA sequence on the targeting vector and another DNA sequence near the DNA of the present invention, which is not included in the targeting vector as primers, thereby to screen the knockout ES cell of the present invention.

The parent ES cells to inactivate the DNA of the present invention by homologous recombination, etc. may be the strain already established as described above, or may be originally established by the publicly known method by Evans and Kaufman with modifications. For example, in the case of mouse ES cells, currently it is common practice to use ES cells of the 129 strain. However, since their immunological background is obscure, the C57BL/6 mouse or the $BDF_1$ mouse ($F_1$ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The $BDF_1$ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, in addition to the advantages that the ovum availability per animal is high and ova are robust.

In establishing ES cells, blastocytes at 3.5 days after fertilization are commonly used. Besides, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera and are therefore preferred. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about $10^6$ cells; therefore, the first selection of ES cells at the early stage of culture can be based on sex identification, and male cells can be selected early, which saves a significant amount of time at the early stage of culture.

Second selection can be achieved by, for example, the number of chromosome confirmation by the G-banding method. It is usually desirable that the chromosome number of the obtained ES cells be 100% of the normal number. However, when it is difficult to obtain the cells having the normal number of chromosomes due to physical operation etc. in cell establishment, it is desirable that the ES cell be again cloned to a normal cell (e.g., in mouse cells having the number of chromosomes being 2n = 40) after the gene of the ES cells is knocked out.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably about 5% carbon dioxide and about 95% air, or about 5% oxygen, about 5% carbon dioxide and 90% air) in the presence of LIF (1-10000 U/ml) on appropriate feeder cells such as STO fibroblasts, treated with a trypsin/EDTA solution (normally about 0.001 to about 0.5% trypsin/about 0.1 to about 5 mM EDTA, preferably about 0.1% trypsin/1 mM EDTA) at the time of passage to obtain separate single cells, which are then seeded on freshly prepared feeder cells. This passage is normally conducted every 1 to 3 other days; it is desirable that cells be observed at passage and cells found to be morphologically abnormal in culture, if any, be abandoned.

Where ES cells are allowed to reach a high density in mono-layers or to form cell aggregates in suspension under appropriate conditions, they will spontaneously differentiate to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtainable from the differentiated ES cells of the present invention, are useful for studying the polypeptide of the present invention or the receptor of the present invention from an aspect of cell biology.

The non-human mammal deficient in expression of the DNA of the present invention can be identified from a normal animal by measuring the mRNA amount in the subject animal by a publicly known method, and indirectly comparing the levels of expression.

As the non-human mammal used, the same examples described above apply.

With respect to the non-human mammal deficient in expression of the DNA of the present invention, the DNA of the present invention can be made knockout by transfecting a targeting vector, prepared as described above, to non-human mammal embryonic stem cells or oocytes thereof, and conducting homologous recombination in which a targeting vector DNA sequence, wherein the DNA of the present invention is inactivated by the transfection, is replaced with the DNA of the present invention on a chromosome of a non-human mammal embryonic stem cell or embryo thereof.

The cells with the DNA of the present invention knockout can be identified by the Southern hybridization analysis using a DNA sequence on or near the DNA of the present invention as a probe, or by PCR analysis using as primers a DNA sequence on the targeting vector and another DNA sequence, which is not included in the targeting vector. When non-human mammalian embryonic stem cells are used, a cell line wherein the DNA of the present invention is inactivated by homologous recombination is cloned; the resulting cloned cell line is injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal composed of both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the polypeptide of the present invention. The individuals deficient in homozygous expression of the polypeptide of the present invention or the receptor of the present invention can be obtained from offspring of the intercross between the heterozygotes of the polypeptide of the present invention or the receptor of the present invention.

When an oocyte or egg cell is used, a DNA solution may be injected, e.g., to the prenucleus by microinjection thereby to obtain a transgenic non-human mammal having a targeting vector introduced in a chromosome thereof. From such transgenic non-human mammals, those having a mutation at the locus of the DNA of the present invention can be obtained by selection based on homologous recombination.

As described above, individuals in which the DNA of the present invention is knocked out permit passage rearing under ordinary rearing conditions, after the individuals obtained by their crossing have proven to have been knockout.

Furthermore, the genital system may be obtained and maintained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammalian embryonic cells bearing the inactivated DNA of the present invention are extremely useful for preparing the non-human mammal deficient in expression of the DNA of the present invention

Since the non-human mammal, in which the DNA of the present invention is inactivated, lacks various biological activities derived from the polypeptide of the present invention or the receptor of the present invention, such an animal can be a disease model suspected of inactivated biological activities of the polypeptide of the present invention or the receptor of the present invention and thus, offers an effective study to investigate causes for and therapy for these diseases.

(8a) Method of screening compounds having therapeutic/preventive effects on diseases caused by deficiency, damages, etc. of the DNA of the present invention

The non-human mammal deficient in expression of the DNA of the present invention can be employed to screen the compounds having therapeutic/preventive effects on diseases caused by deficiency, damages, etc. of the DNA of the present invention.

That is, the present invention provides a method of screening a compound having therapeutic/preventive effects for diseases caused by deficiency, damages, etc. of the DNA of the present invention, which comprises administering a test compound to the non-human mammal deficient in expression of the DNA of the present invention and observing and measuring a change occurred in the animal.

As the non-human mammal deficient in expression of the DNA of the present invention which can be employed for the screening method, the same examples as given hereinabove apply.

Examples of the test compounds include peptides, proteins, non-peptide compounds, synthetic compounds, fermentation products, cell extracts, vegetable extracts, animal tissue extracts, blood plasma, etc. These compounds may be novel compounds or publicly known compounds.

Specifically, the non-human mammal deficient in expression of the DNA of the present invention is treated with a test compound, comparison is made with an intact animal for control and a change in each organ, tissue, disease conditions, etc. of the animal is used as an indicator to assess the therapeutic/preventive effects of the test compound.

For treating an animal to be tested with a test compound, for example, oral administration, intravenous injection, etc. are applied and the treatment is appropriately selected depending upon conditions of the test animal, properties of the test compound, etc. Furthermore, a dose of test compound to be administered can be appropriately chosen depending on method for administration, nature of test compound, etc.

In screening, e.g., a gastric acid secretion promoter, a pylorus ligature is done in the non-human mammal deficient in expression of the DNA of the present invention and a test compound is administered to determine changes in gastric acid outputs of the animal with passage of time.

In the screening method described above, when a test compound is administered to a test animal and found to increase the gastric acid output in the test animal to at least about 10%, preferably at least about 30% and more preferably at least about 50%, the test compound can be selected to be a compound having effects of preventing/treating the diseases described above.

The compound obtained using the screening method above is a compound selected from the test compounds described above and exhibits effects of treating/preventing the diseases described above caused by deficiencies, damages, etc. of the polypeptide of the present invention. Therefore, the compound can be employed as a safe and low toxic drug for preventing/treating the diseases. Furthermore, compounds derived from such compounds obtained by the screening described above can be used as well.

The compound obtained by the screening method above may be in the form of salts. As such salts, there may be used salts with physiologically acceptable acids (e.g., inorganic acids, organic acids, etc.) or bases (e.g., alkali metal salts, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A pharmaceutical composition comprising the compound obtained by the above screening method or salts thereof can be manufactured in a manner similar to the method for preparing the pharmaceutical composition comprising the polypeptide of the present invention described hereinabove.

Since the pharmaceutical composition thus obtained is safe and low toxic, it can be administered to human and another mammal (e.g., rat, mouse, guinea pig, rabbit, sheep, swine, bovine, horse, cat, dog, monkey, etc.).

A dose of the compound or its salt to be administered varies depending upon particular disease, subject to be administered, route of administration, etc., and when the compound is subcutaneously administered, the compound is administered to an adult patient (as 60 kg body weight) generally at a dose of approximately 0.1 to 100 mg, preferably approximately 1.0 to 50 mg, more preferably approximately 1.0 to 20 mg per day. For parenteral administration, a single dose of the compound varies depending upon subject to be administered, target disease, etc. Where a compound that promotes the activity of a promoter for the DNA of the present invention is administered to an adult patient (as 60 kg body weight) with, e.g., dyspepsia, it is advantageous to administer the compound intravenously in the form of an injectable preparation at a dose of approximately 0.01 to 30 mg, preferably approximately 0.1 to 20 mg, more preferably approximately 0.1 to 10 mg per day. For other animals, the compound can be administered in the corresponding dose with converting it into that for the 60 kg body weight.

(8b) Method of screening a compound that promotes or inhibits the activity of a promoter for the DNA of the present invention

The present invention provides a method of screening a compound or its salt that promotes or inhibits the activity of a promoter for the DNA of the present invention, which comprises administering a test compound to a non-human mammal deficient in expression of the DNA of the present invention and detecting the expression of a reporter gene.

In the screening method above, the non-human mammal deficient in expression of the DNA of the present invention is selected from the aforesaid non-human mammal deficient in expression of the DNA of the present invention, as an animal in which the DNA of the present invention is inactivated by introducing a reporter gene and the reporter gene is expressed under control of a promoter for the DNA of the present invention.

The same examples of the test compound apply to specific compounds used for the screening.

As the reporter gene, the same specific examples apply to this screening method. Preferably employed are β-galactosidase (lacZ), soluble alkaline phosphatase gene, luciferase gene and the like.

Since a reporter gene is present under control of a promoter for the DNA of the present invention in the non-human mammal deficient in expression of the DNA of the present invention wherein the DNA of the present invention is substituted with the reporter gene, the activity of the promoter can be detected by tracing expression of a substance encoded by the reporter gene.

When a part of the DNA region encoding the polypeptide of the present invention is substituted with, e.g., β-galactosidase gene (lacZ) derived from Escherichia coli, β-galactosidase is expressed in a tissue where the polypeptide of the present invention should originally be expressed, instead of the polypeptide of the present invention. Thus, the state of expression condition of the polypeptide of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the polypeptide of the present invention, or its tissue slice section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the aforesaid screening method are compounds that are selected from the test compounds described above and the compounds that promote or inhibit the activity of a promoter for the DNA of the present invention.

The compound obtained by the screening method above may form salts. As salts of the compound, there may be used salts with physiologically acceptable acids (e.g., inorganic acids, etc.) or bases (e.g., organic acids, etc.), and especially preferred are physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salts that promote the activity of a promoter for the DNA of the present invention can promote expression of the polypeptide of the present invention thereby to promote the function of the polypeptide. Thus, these compounds can be used as, e.g., gastric acid secretion promoters and are useful as agents for preventing/treating, for example, dyspepsia (e.g., pituitary dyspepsia, renal dyspepsia, etc.), bone metabolism disorders (e.g., osteoporosis, osteomalacia, etc.), anemia (e.g., iron deficiency anemia, etc.), or the like.

Since the compound or its salts that inhibit the activity of a promoter for the DNA of the present invention can inhibit expression of the polypeptide of the present invention thereby to inhibit the function of the polypeptide, these compounds are useful as, e.g., gastric acid secretion inhibitors, mucosa protectants, mineral absorption promoters, etc. and can be used as agents for preventing/treating, e.g., upper gastrointestinal diseases [for example, peptic ulcers (e.g., gastric ulcer, duodenal ulcer, marginal ulcer, Zollinger-Ellison syndrome, etc.), gastritis, reflux esophagitis, NUD (non-ulcer dyspepsia), gastric cancer, gastric MALT lymphoma, nonsteroidal anti-inflammatory drug-induced ulcer, postoperative, stress-induced hyperacidity and ulcer, etc.], antibacterial agents to Helicobacter pylori, or the like.

Furthermore, compounds derived from the compounds obtained by the screening described above may also be used similarly.

The pharmaceuticals comprising the compound obtained by the screening method or its salt can be manufactured in a manner similar to the aforesaid pharmaceuticals comprising the polypeptide of the present invention or its salt.

Since the pharmaceutical composition thus obtained is safe and low toxic, it can be administered to human and another mammal (e.g., rat, mouse, guinea pig, rabbit, sheep, swine, bovine, horse, cat, dog, monkey, etc.).

A dose of the compound or its salt to be administered varies depending upon target disease, subject to be administered, route of administration, etc. Where the compound that promotes the activity of a promoter for the DNA of the present invention is orally administered, the compound is administered to an adult patient (as 60 kg body weight) generally at a dose of approximately 0.1 to 100 mg, preferably approximately 1.0 to 50 mg, more preferably approximately 1.0 to 20 mg per day. In parenteral administration, a single dose of the compound varies depending upon subject to be administered, target disease, etc. Where the compound that promotes the activity of a promoter for the DNA of the present invention is administered to an adult patient (as 60 kg body weight) in the form of an injectable preparation, it is advantageous to administer the compound intravenously at a dose of approximately 0.01 to 30 mg, preferably approximately 0.1 to 20 mg, more preferably approximately 0.1 to 10 mg per day. For other animals, the compound can be administered in the Corresponding dose with converting it into that for the 60 kg body weight.

On the other hand, when a compound that inhibits the activity of a promoter for the DNA of the present invention is orally administered, the compound is administered to an adult patient (as 60 kg body weight) generally at a dose of approximately 0.1 to 100 mg, preferably approximately 1.0 to 50 mg, more preferably approximately 1.0 to 20 mg per day. In parenteral administration, a single dose of the compound varies depending upon subject to be administered, target disease, etc. When the compound that inhibits the activity of a promoter for the DNA of the present invention is administered to an adult patient (as 60 kg body weight) in the form of an injectable preparation, it is advantageous to intravenously inject the compound at a dose of approximately 0.01 to 30 mg, preferably approximately 0.1 to 20 mg, more preferably approximately 0.1 to 10 mg per day. For other animals, the compound can be administered in the corresponding dose with converting it into that for the 60 kg body weight.

As described above, the non-human mammal deficient in expressing the DNA of the present invention is extremely useful for screening a compound or its salt that promotes or inhibits the activity of promoter for the DNA of the present invention, and can thus greatly contribute to investigations of causes for various diseases caused by failure to express the DNA of the present invention or to development of preventive/therapeutic drugs for these diseases.

Moreover, when a so-called transgenic animal (gene-transfected animal) is prepared by using a DNA containing the promoter region of the polypeptide of the present invention, ligating genes encoding various proteins downstream the same and injecting the genes into animal oocyte, the polypeptide can be specifically synthesized by the animal so that it becomes possible to investigate the activity in vivo. Furthermore, when an appropriate reporter gene is ligated to the promoter region described above to establish a cell line so as to express the gene, such can be used as a survey system of low molecular weight compounds that specifically promotes or suppresses the ability of producing the polypeptide itself of the present invention in vivo.

In the specification and drawings, when the codes of bases, amino acids, etc. are shown by abbreviations and in this case, they are denoted in accordance with the IUPAC-IUB Commission on Biochemical Nomenclature or by the common codes in the art, examples of which are described below. For amino acids that may have the optical isomer, L form is presented unless otherwise indicated.

| | |
|---|---|
| DNA | : deoxyribonucleic acid |
| cDNA | : complementary deoxyribonucleic acid |
| A | : adenine |
| T | : thymine |
| G | : guanine |
| C | : cytosine |
| I | : inosine |
| R | : adenine (A) or guanine (G) |
| Y | : thymine (T) or cytosine (C) |
| M | : adenine (A) or cytosine (C) |
| K | : guanine (G) or thymine (T) |
| S | : guanine (G) or cytosine (C) |
| W | : adenine (A) or thymine (T) |
| B | : guanine (G), guanine (G) or thymine (T) |
| D | : adenine (A), guanine (G) or thymine (T) |
| V | : adenine (A), guanine (G) or cytosine (C) |
| N | : adenine (A), guanine (G), cytosine (C) or thymine (T), or unknown or other base |
| RNA | : ribonucleic acid |
| mRNA | : messenger ribonucleic acid |
| dATP | : deoxyadenosine triphosphate |
| dTTP | : deoxythymidine triphosphate |
| dGTP | : deoxyguanosine triphosphate |
| dCTP | : deoxycytidine triphosphate |
| ATP | : adenosine triphosphate |
| EDTA | : ethylenediaminetetraacetic acid |
| SDS | : sodium dodecyl sulfate |
| BHA | : benzhydrylamine |
| pMBHA | : p-methylbenzhydrylamine |
| Tos | : p-toluenesulfonyl |
| Bzl | : benzyl |
| Bom | : benzyloxymethyl |
| Boc | : t-butyloxycarbonyl |
| DCM | : dichloromethane |
| HOBt | : 1-hydroxybenztriazole |
| DCC | : N,N'-dicyclohexylcarbodiimide |
| TFA | : trifluoroacetic acid |
| DIEA | : diisopropylethylamine |

| | |
|---|---|
| BSA | : bovine serum albumin |
| CHAPS | : 3-[(3-Cholamidopropyl)dimethylammonio]propanesulfonate |
| Gly or G | : glycine |
| Ala or A | : alanine |
| Val or V | : valine |
| Leu or L | : leucine |
| Ile or I | : isoleucine |
| Ser or S | : serine |
| Thr or T | : threonine |
| Cys or C | : cysteine |
| Met or M | : methionine |
| Glu or E | : glutamic acid |
| Asp or D | : aspartic acid |
| Lys or K | : lysine |
| Arg or R | : arginine |
| His or H | : histidine |
| Phe or F | : phenylalanine |
| Tyr or Y | : tyrosine |
| Trp or W | : tryptophan |
| Pro or P | : proline |
| Asn or N | : asparagine |
| Gln or Q | : glutamine |
| pGlu | : pyroglutamic acid |
| Tyr (I) | : 3-iodotyrosine |
| DMF | : N,N-dimethylformamide |
| Fmoc | : N-9-fluorenylmethoxycarbonyl |
| Trt | : trityl |
| Pbf | : 2,2,4,6,7-pentamethyldihydrobenzofuran-5-sulfonyl |
| Clt | : 2-chlorotrityl |
| $Bu^t$ | : t-butyl |
| Met (O) | : methionine sulfoxide |

The sequence identification numbers in the sequence listing of the specification indicates the following sequences, respectively.

[SEQ ID NO: 1]

This shows the amino acid sequence of human GPR8 ligand peptide (hNPW23).

[SEQ ID NO: 2]

This shows the amino acid sequence of human GPR8 ligand peptide (hNPW30).

[SEQ ID NO: 3]

This shows the base sequence encoding the amino acid sequence represented by SEQ ID NO: 1.

[SEQ ID NO: 4]

This shows the base sequence encoding the amino acid sequence represented by SEQ ID NO: 2.

[SEQ ID NO: 5]

This shows the base sequence of DNA encoding a part of human GPR8 ligand peptide precursor.

[SEQ ID NO: 6]

This shows the amino acid sequence of a part of human GPR8 ligand peptide precursor.

[SEQ ID NO: 7]

This shows the amino acid sequence of synthetic human GPR8 ligand (1-29).

[SEQ ID NO: 8]

This shows the amino acid sequence of synthetic human GPR8 ligand (1-28).

[SEQ ID NO: 9]

This shows the amino acid sequence of synthetic human GPR8 ligand (1-27).

[SEQ ID NO: 10]

This shows the amino acid sequence of synthetic human GPR8 ligand (1-26).

[SEQ ID NO: 11]

This shows the amino acid sequence of synthetic human GPR8 ligand (1-25).

[SEQ ID NO: 12]

This shows the amino acid sequence of synthetic human GPR8 ligand (1-24).

[SEQ ID NO: 13]

This shows the base sequence encoding the amino acid sequence represented by SEQ ID NO: 7.

[SEQ ID NO: 14]

This shows the base sequence encoding the amino acid sequence represented by SEQ ID NO: 8.

[SEQ ID NO: 15]

This shows the base sequence encoding the amino acid sequence represented by SEQ ID NO: 9.

[SEQ ID NO: 16]

This shows the base sequence encoding the amino acid sequence represented by SEQ ID NO: 10.

[SEQ ID NO: 17]

This shows the base sequence encoding the amino acid sequence represented by SEQ ID NO: 11.

[SEQ ID NO: 18]

This shows the base sequence encoding the amino acid sequence represented by SEQ ID NO: 12.

[SEQ ID NO: 19]

This shows the base sequence of cDNA encoding human GPR8 ligand peptide precursor.

[SEQ ID NO: 20]

This shows the amino acid sequence of human GPR8 ligand peptide precursor.

[SEQ ID NO: 21]

This shows the amino acid sequence of porcine GPR8 ligand peptide.

[SEQ ID NO: 22]

This shows the base sequence of cDNA encoding porcine GPR8 ligand peptide precursor.

[SEQ ID NO: 23]

This shows the amino acid sequence of porcine GPR8 ligand peptide precursor.

[SEQ ID NO: 24]

This shows the amino acid sequence of porcine GPR8 ligand peptide.

[SEQ ID NO: 25]

This shows the amino acid sequence of porcine GPR8 ligand peptide.

[SEQ ID NO: 26]

This shows the base sequence encoding the amino acid sequence represented by SEQ ID NO: 24.

[SEQ ID NO: 27]

This shows the base sequence encoding the amino acid sequence represented by SEQ ID NO: 25.

[SEQ ID NO: 28]

This shows the base sequence of cDNA encoding rat GPR8 ligand peptide precursor.

[SEQ ID NO: 29]

This shows the amino acid sequence of rat GPR8 ligand peptide precursor.

[SEQ ID NO: 30]

This shows the amino acid sequence of rat GPR8 ligand peptide.

[SEQ ID NO: 31]

This shows the amino acid sequence of rat GPR8 ligand peptide.

[SEQ ID NO: 32]

This shows the base sequence encoding the amino acid sequence represented by SEQ ID NO: 30.

[SEQ ID NO: 33]

This shows the base sequence encoding the amino acid sequence represented by SEQ ID NO: 31.

[SEQ ID NO: 34]

This shows the base sequence of cDNA encoding mouse GPR8 ligand peptide precursor.

[SEQ ID NO: 35]

This shows the amino acid sequence of mouse GPR8 ligand peptide precursor.

[SEQ ID NO: 36]

This shows the amino acid sequence of mouse GPR8 ligand peptide.

[SEQ ID NO: 37]

This shows the amino acid sequence of mouse GPR8 ligand peptide.

[SEQ ID NO: 38]

This shows the base sequence encoding the amino acid sequence represented by SEQ ID NO: 36.

[SEQ ID NO: 39]

This shows the base sequence encoding the amino acid sequence represented by SEQ ID NO: 37.

[SEQ ID NO: 40]

This shows the amino acid sequence of oxidized synthetic human GPR8 ligand (1-23).

[SEQ ID NO: 41]

This shows the amino acid sequence of synthetic human GPR8 ligand (1-22).

[SEQ ID NO: 42]

This shows the amino acid sequence of synthetic human GPR8 ligand (1-21).

[SEQ ID NO: 43]

This shows the amino acid sequence of synthetic human GPR8 ligand (1-20).

[SEQ ID NO: 44]

This shows the amino acid sequence of synthetic human GPR8 ligand (1-19).

[SEQ ID NO: 45]

This shows the amino acid sequence of synthetic human GPR8 ligand (1-18).

[SEQ ID NO: 46]

This shows the amino acid sequence of synthetic human GPR8 ligand (1-17).

[SEQ ID NO: 47]

This shows the amino acid sequence of synthetic human GPR8 ligand (1-16).

[SEQ ID NO: 48]

This shows the amino acid sequence of oxidized synthetic human GPR8 ligand (1-23).

[SEQ ID NO: 49]

This shows the amino acid sequence of oxidized synthetic rat or mouse GPR8 ligand (1-23).

[SEQ ID NO: 50]

This shows the amino acid sequence of synthetic Fmoc-protected human GPR8 ligand (1-23).

[SEQ ID NO: 51]

This shows the amino acid sequence of synthetic [$N^{\alpha}$-Acetyl-$Trp^1$]-human GPR8 ligand (1-23).

[SEQ ID NO: 52]

This shows the amino acid sequence of synthetic human GPR8 ligand (2-23).

[SEQ ID NO: 53]

This shows the amino acid sequence of synthetic human GPR8 ligand (4-23).

[SEQ ID NO: 54]

This shows the amino acid sequence of synthetic human GPR8 ligand (9-23).

[SEQ ID NO: 55]

This shows the amino acid sequence of synthetic human GPR8 ligand (15-23).

[SEQ ID NO: 56]

This shows the amino acid sequence of synthetic [N-Acetyl-$Tyr^2$]-human GPR8 ligand (2-23).

[SEQ ID NO: 57]

This shows the amino acid sequence of synthetic [D-$Trp^1$]-human GPR8 ligand (1-23).

[SEQ ID NO: 58]

This shows the amino acid sequence of synthetic [N-3-Indolepropanoyl-$Tyr^2$]-human GPR8 ligand (2-23).

[SEQ ID NO: 59]

This shows the base sequence encoding the amino acid sequence represented by SEQ ID NO: 41.

[SEQ ID NO: 60]

This shows the base sequence encoding the amino acid sequence represented by SEQ ID NO: 42.

[SEQ ID NO: 61]

This shows the base sequence encoding the amino acid sequence represented by SEQ ID NO: 43.

[SEQ ID NO: 62]

This shows the base sequence encoding the amino acid sequence represented by SEQ ID NO: 44.

[SEQ ID NO: 63]

This shows the base sequence encoding the amino acid sequence represented by SEQ ID NO: 45.

[SEQ ID NO: 64]

This shows the base sequence encoding the amino acid sequence represented by SEQ ID NO: 46.

[SEQ ID NO: 65]

This shows the base sequence encoding the amino acid sequence represented by SEQ ID NO: 47.

[SEQ ID NO: 66]

This shows the base sequence encoding the amino acid sequence represented by SEQ ID NO: 52.

[SEQ ID NO: 67]

This shows the base sequence encoding the amino acid sequence represented by SEQ ID NO: 53.

[SEQ ID NO: 68]

This shows the base sequence encoding the amino acid sequence represented by SEQ ID NO: 54.

[SEQ ID NO: 69]

This shows the base sequence encoding the amino acid sequence represented by SEQ ID NO: 55.

[SEQ ID NO: 70]

This shows the base sequence encoding the amino acid sequence represented by SEQ ID NO: 21.

[SEQ ID NO: 71]

This shows the amino acid sequence of [$Phe^2$]-human GPR8 ligand (1-20).

[SEQ ID NO: 72]

This shows the base sequence encoding the amino acid sequence represented by SEQ ID NO: 71.

[SEQ ID NO: 73]

This shows the amino acid sequence of human GPR8.

[SEQ ID NO: 74]

This shows the base sequence encoding the amino acid sequence represented by SEQ ID NO: 73.

[SEQ ID NO: 75]

This shows the amino acid sequence of rat TGR26.

[SEQ ID NO: 76]

This shows the base sequence of cDNA encoding rat TGR26.

[SEQ ID NO: 77]

This shows the amino acid sequence of mouse TGR26.

[SEQ ID NO: 78]

This shows the base sequence of cDNA encoding mouse TGR26.

[SEQ ID NO: 79]

This shows the amino acid sequence of human GPR7.

[SEQ ID NO: 80]

This shows the base sequence encoding human GPR7.
Hereinafter, the present invention will be described in more detail by referring to REFERENCE EXAMPLES and EXAMPLES but they are not deemed to limit the scope of the invention.

REFERENCE EXAMPLE 1

Production of [$Phe^2$] human GPR8 ligand (1-20) (SEQ ID NO: 71)

Following a program, a peptide chain was extended from the C terminus on an automated peptide synthesizer (ABI 433 model) of Applied Biosystems, Inc. in accordance with a sequential Fmoc strategy to synthesize the objective peptide resin protected.
Using Wang (p-benzyloxybenzyl alcohol) resin (0.25 mmol) as the starting amino acid resin carrier, Fmoc-amino acid derivatives of Fmoc-Leu, Fmoc-Gly, Fmoc-Ala, Fmoc-Arg(Pbf), Fmoc-Val, Fmoc-Thr($Bu^t$), Fmoc-His(Trt), Fmoc-Tyr($Bu^t$), Fmoc-Pro, Fmoc-Ser($Bu^t$), Fmoc-Lys(Boc), Fmoc-Phe and Fmoc-Trp(Boc) were sequentially condensed on the carrier by HBTU (2-(1H-benzotriazol-1-yl)-1,1,3,3-tetramethyluronium hexafluorophosphate), according to the sequence.
After construction of the peptide on the resin was completed, the protected peptide resin was dried. Deprotection of the protected peptide obtained and release of the peptide from the resin carrier were accomplished by treatment with TFA. The crude peptide obtained was extracted with 0.1% TFA in water and then lyophilized to give the crude peptide as a powdery solid. Subsequently, the crude peptide was applied on a reversed phase high performance chromatography (Shimadzu Corporation, preparative pump: Model LC8A) with the system of acetonitrile-0.1% TFA in water (15-35%, 80 mins.) for preparative purification to give 35 mg of the objective purified peptide.
The purified product was hydrolyzed with 4N methanesulfonic acid containing 0.2% 3-(2-aminoethyl)indole under conditions of 110°C for 22 hours. Amino acid analysis of the hydrolysate is as follows (calcd. is within parenthesis). Thr (1) 0.93, Ser (1) 0.92, Gly (2) 2.03, Ala (3) 3.09, Val (2) 1.90, Leu (2) 2.02, Tyr (1) 1.02, Phe (1) 1.00, His (2) 1.91, Lys (1) 0.98, Trp (1) 0.88, Arg (2) 2.06, Pro (1) 1.02
The purity was calculated on HPLC to be 98.8%. The mass spectrum was found to be 2266.6 (calcd. 2266.6).

REFERENCE EXAMPLE 2

Production of [$Phe^2$, $^{125}I$-$Tyr^{10}$] human GPR8 ligand (1-20) using the lactoperoxidase method

In 10 μl of DMSO, 10 nmol of [$Phe^2$] human GPR8 ligand (1-20) (SEQ ID NO: 71) obtained by a modification of the process described in REFERENCE EXAMPLE 1 was dissolved. The resulting solution was mixed and reacted with 10 μl of 0.1 M nickel chloride aqueous solution, 10 μl of 0.001 % aqueous hydrogen peroxide in 0.1 M HEPES (pH 7.6), 10 μl of 10 μg/ml lactoperoxidase (Sigma) in 0.1 M HEPES (pH 7.6) and 10 μl of [$^{125}I$] NaI 40 MBq (NEN Life Science Products) at room temperature for 50 minutes. The produced [$Phe^2$, $^{125}I$-$Tyr^{10}$] human GPR8 ligand (1-20) was fractionated on HPLC under the following conditions.
The column used was ODS-80TM (4.6 mm x 15 cm) (Toso Corporation); using 10% acetonitrile/0.1 % TFA as Eluent A and as Eluent B 60% acetonitrile/0.1% TFA, gradient elution with 0-0 (2 min), 0-27 (5 min) and 27-32 (40 min) % B/A+B was performed. The conditions were set for the flow rate of 1 mL/min, the column temperature at 40°C, and detection at 215 nm. Under the HPLC conditions, [$Phe^2$, $^{125}I$-$Tyr^{10}$] human GPR8 ligand (1-20) was eluted at about 25 mins.

EXAMPLE 1

Effects on gastric acid secretion in rat by intravenous injection of hNPW23

After Wistar male rats (Nippon Charles River, weighing 320-360 g) were fasted for 16 hours with water given *ad libitum,* the animal was injected intraperitoneally with ethyl carbamate (Wako Pure Chemical) (1.0 g/ml/kg) and firmly secured to the operating table under anesthesia to perform laparotomy. Stomach catheter (Medtop X2-50, 3.5 mm in outer diameter and 2.1 mm in inner diameter) was inserted into the stomach (2 cm) through an incision in the duodenum. After ligature at the pyloric canal, the right femoral vein was isolated to perform venous cannulation. The stomach was irrigated with distilled water (Otsuka Pharmaceutical Co., Ltd.) warmed at 37°C and allowed to stand for about an hour. hNPW23 was dissolved in saline (Otsuka Pharmaceutical Co., Ltd.) to become 80 μM. The control group was given saline. Test was performed with 8 rats in each group. hNPW23 and saline were given to the animal at a flow rate of 1 ml/h for 30 minutes through the femoral vein using Infusion Pump (Harvard Apparatus). Gastric acid output was determined as follows. Distilled water (1 ml) previously warmed at 37°C was perfused into the stomach and the gastric contents were collected 10 minutes after. After 30 μl of Bromthymol Blue indicator solution (BTB, Wako Pure Chemical) was added to each gastric juice collected, the mixture was titrated with 0.1 N NaOH solution and the consumed amount of NaOH required to reach the equivalent point (the amount consumed) was determined to calculate the gastric acid output. The gastric acid output before administration of hNPW23 was measured and T-assay was conducted on a percentage (%) of changes in gastric acid outputs before and after administration.

Changes in gastric acid outputs by administration of hNPW23 were shown by the following equation.

Gastric acid output (μEq) = 0.1N NaOH x Amount consumed (μl)

(%) = (gastric acid output after hNPW23 administration)/( gastric acid

output before hNPW23 administration) x 100

According to the results, the changes in gastric acid outputs were 103.7 ± 5.1 % in the control group and 145.6 ± 8.5% in the hNPW23 group, indicating that the gastric acid outputs significantly increased in the hNPW23 group, with P value of 0.01 or less. The numerical values designate mean ± standard error (mean ± SE).

As clearly noted from the foregoing, the gastric acid outputs significantly increased in the hNPW23 group, when compared to the control group.

EXAMPLE 2

Effects on the gastric fluid volume, pH of gastric fluid, gastric acidity and gastric acid output in pylorus ligated rats by hNPW23 injection

After Wistar male rats (340-360 g) were fasted for 16 hours with water given *ad libitum,* the animal was exposed at laparotomy under ether anesthesia and submitted to the pylorus ligature assay to close the abdomen. After hNPW23 was dissolved in saline (2 mg/ml), the solution was intraperitoneally injected (2 mg/kg). The control group was given saline. Test was performed with 8 rats in each group. Three hours after the pylorus ligature, the rat's abdomen was opened under anesthesia with Nembutal (40 mg/kg, Dainippon Pharmaceutical). After ligation of the cardia, the stomach was removed. Gastric contents were collected and centrifuged (2500 rpm x 10 min.). Using the supernatant, gastric fluid volume (ml), pH intragastric pH, gastric acidity (mEq/1) and total acid output (μq/kg/h) were measured.

To 1 ml of gastric juice, 30 μl of BTB (Wako Pure Chemical) solution was added and 0.1N NaOH solution was dropwise added to the mixture. The consumed amount of NaOH required to reach the equivalent point (pH 7.0) was measured to calculate the gastric acidity and total acid output.

The pH of gastric juice was measured using Twin pH meter B-212 (Horiba, Ltd.).

The respective measurements values were calculated according to the equation below and T-assay was conducted.

Gastric acidity (mEq/l) = 0.1N NaOH x consumed amount (μl)/gastric juice

(1000 μl)

$$\text{Total acid output } (\mu Eq/kg/h) = \text{gastric acidity x gastric fluid volume/weight/time}$$

The results are shown in Table 1.

[Table 1]

| | Intragastric pH | Gastric fluid volume (ml) | Gastric acidity (mEq/l) | Total acid output (μEq/kg/h) |
|---|---|---|---|---|
| Control | 1.12±0.08 | 4.0±0.7 | 69.4±6.2 | 294.6±86.6 |
| hNPW23 | 0.83±0.03* | 7.9±1.2* | 96.0±4.2** | 747.4±121.5** |

The values in the table are expressed as mean ± standard error (Mean ± SE).
Symbol * shows that p-value is 0.05 or less, as compared to the control group.
Symbol ** shows that p-value is 0.01 or less, as compared to the control group.
When hNPW23 was injected to rats (intravenously or intraperitoneally), the acid output increased significantly. This reveals that hNPW23 has the effect of promoting gastric acid secretion.

EXAMPLE 3

Effects on the amount of gastrin secretion in pylorus ligated rats by hNPW23 injection

After Wistar male rats (340-360 g) were fasted for 16 hours with water given *ad libitum* as in EXAMPLE 2 above, the animal was exposed at laparotomy under ether anesthesia and submitted to the pylorus ligature assay to close the abdomen. After hNPW23 was dissolved in saline (2 mg/ml), the solution was intraperitoneally injected (2 mg/kg). The control group was given saline. Test was performed with 8 rats in each group. Three hours after the pylorus ligature, the rat's abdomen was opened under anesthesia with Nembutal (40 mg/kg) and blood was collected through the abdominal aorta. The blood was placed in a blood tube of 7 ml (Terumo, containing EDTA), centrifuged (3000 rpm x 10 min.) and stored at -40°C in lyophilized state until assay. Gastrin level in blood was assayed with Gastrin RIA KIT II (ABBOTT JAPAN). The F-assay was conducted on mean measurement values. The gastrin level in blood increased significantly in the 2000 μg/kg group. This reveals that gastrin is involved in the effect of NPW for promoting gastric acid secretion and NPW has the action of promoting gastric acid secretion.

INDUSTRIAL APPLICABILITY

The compound or its salts that inhibit the activity of the polypeptide or receptor of the present invention, the antibody of the present invention and the antisense DNA of the present invention are low-toxic and safe and useful as, e.g., gastric acid secretion inhibitors, mucosa protectants, mineral absorption promoters, etc. and thus can be used as agents for preventing/treating, for example, upper digestive tract disorders [for example, peptic ulcers (e.g., gastric ulcer, duodenal ulcer, marginal ulcer, Zollinger-Ellison syndrome, etc.), gastritis, reflux esophagitis, NUD (non-ulcer dyspepsia), gastric cancer, gastric MALT lymphoma, nonsteroidal anti-inflammatory drug-induced ulcer, postoperative, stress-induced hyperacidity and ulcer, etc.], antibacterial agents to Helicobacter pylori, or the like.
The compound or its salts that promote the activity of the polypeptide or receptor of the present invention, the polypeptide or receptor of the present invention and the DNA of the present invention can be used as, e.g., gastric acid secretion promoters and can be used as agents for preventing/treating, for example, dyspepsia (e.g., pituitary dyspepsia, renal dyspepsia, etc.), bone metabolism disorders (e.g., osteoporosis, osteomalacia, etc.), anemia (e.g., iron deficiency anemia, etc.), or the like. Moreover, the compound or its salts that promote the activity of the polypeptide or receptor of the present invention, the polypeptide or receptor of the present invention and the DNA of the present invention can be used as test agents for gastric secretory function.
The polypeptide of the present invention and the DNA of the present invention are also useful for screening the preventive/therapeutic agents described above.

## Claims

1. An agent for preventing/treating upper digestive tract disorders, which comprises a compound or its salt that inhibits the activity of a polypeptide comprising the same or substantially the same amino acid sequence as the amino acid sequence represented by SEQ ID NO: 1, its amide or ester, or a salt thereof.

2. An agent for preventing/treating upper digestive tract disorders, which comprises (i) an antibody to a polypeptide comprising the same or substantially the same amino acid sequence as the amino acid sequence represented by SEQ ID NO: 1, its amide or ester, or a salt thereof, or (ii) an antibody to a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

3. An agent for preventing/treating upper digestive tract disorders, which comprises an antisense polynucleotide comprising the entire or part of base sequence complementary or substantially complementary to a base sequence for (i) a polynucleotide encoding a polypeptide comprising the same or substantially the same amino acid sequence as the amino acid sequence represented by SEQ ID NO: 1, its amide or ester, or a salt thereof, or (ii) a polynucleotide encoding a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

4. A gastric acid secretion inhibitor, which comprises a compound or its salt that inhibits the activity of a polypeptide comprising the same or substantially the same amino acid sequence as the amino acid sequence represented by SEQ ID NO: 1, its amide or ester, or a salt thereof.

5. An agent for preventing/treating dyspepsia, bone metabolism disorders or anemia, which comprises a compound or its salt that promotes the activity of a polypeptide comprising the same or substantially the same amino acid sequence as the amino acid sequence represented by SEQ ID NO: 1, its amide or ester, or a salt thereof.

6. An agent for preventing/treating dyspepsia, bone metabolism disorders or anemia, which comprises a polypeptide comprising the same or substantially the same amino acid sequence as the amino acid sequence represented by SEQ ID NO: 1, its amide or ester, or a salt thereof.

7. An agent for preventing/treating dyspepsia, bone metabolism disorders or anemia, which comprises a polynucleotide encoding a polypeptide comprising the same or substantially the same amino acid sequence as the amino acid sequence represented by SEQ ID NO: 1, its amide or ester, or a salt thereof.

8. A gastric acid secretion promoter, which comprises a compound or its salt that promotes the activity of a polypeptide comprising the same or substantially the same amino acid sequence as the amino acid sequence represented by SEQ ID NO: 1, its amide or ester, or a salt thereof.

9. A test agent for gastric secretory function, which comprises a polypeptide comprising the same or substantially the same amino acid sequence as the amino acid sequence represented by SEQ ID NO: 1, its amide or ester, or a salt thereof.

10. A test agent for gastric secretory function, which comprises a compound or its salt that promotes the activity of a polypeptide comprising the same or substantially the same amino acid sequence as the amino acid sequence represented by SEQ ID NO: 1, its amide or ester, or a salt thereof.

11. A method of screening a preventive/therapeutic agent for upper digestive tract disorders, which comprises using (i) a polypeptide comprising the same or substantially the same amino acid sequence as the amino acid sequence represented by SEQ ID NO: 1, its amide or ester, or a salt thereof, and/or (ii) a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

12. A kit for screening a preventive/therapeutic agent for upper digestive tract disorders, which comprises (i) a polypeptide comprising the same or substantially the same amino acid sequence as the amino acid sequence represented by SEQ ID NO: 1, its amide or ester, or a salt thereof, and/or (ii) a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

**13.** A method of screening a preventive/therapeutic agent for upper digestive tract disorders, which comprises using (i) a polynucleotide encoding a polypeptide comprising the same or substantially the same amino acid sequence as the amino acid sequence represented by SEQ ID NO: 1, its amide or ester, or a salt thereof, and/or (ii) a polynucleotide encoding a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

**14.** A kit for screening a preventive/therapeutic agent for upper digestive tract disorders, which comprises (i) a polynucleotide encoding a polypeptide comprising the same or substantially the same amino acid sequence as the amino acid sequence represented by SEQ ID NO: 1, its amide or ester, or a salt thereof, and/or (ii) a polynucleotide encoding a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

**15.** A method of screening a gastric acid secretion inhibitor, which comprises using (i) a polypeptide comprising the same or substantially the same amino acid sequence as the amino acid sequence represented by SEQ ID NO: 1, its amide or ester, or a salt thereof, and/or (ii) a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

**16.** A method of screening a preventive/therapeutic agent for dyspepsia, bone metabolism disorders or anemia, which comprises using (i) a polypeptide comprising the same or substantially the same amino acid sequence as the amino acid sequence represented by SEQ ID NO: 1, its amide or ester, or a salt thereof, and/or (ii) a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

**17.** A method of screening a gastric acid secretion promoter, which comprises using (i) a polypeptide comprising the same or substantially the same amino acid sequence as the amino acid sequence represented by SEQ ID NO: 1, its amide or ester, or a salt thereof, and/or (ii) a protein comprising the same or substantially the same amino acid sequence as the amino acid sequence represented by SEQ ID NO: 73, SEQ ID NO: 75, SEQ ID NO: 77 or SEQ ID NO: 79, its partial peptide, or a salt thereof.

**18.** A method of preventing/treating upper digestive tract disorders, which comprises administering to a mammal an effective dose of (i) a compound or its salt that inhibits the activity of a polypeptide comprising the same or substantially the same amino acid sequence as the amino acid sequence represented by SEQ ID NO: 1, its amide or ester, or a salt thereof, (ii) an antibody to said polypeptide, its amide or ester, or a salt thereof, (iii) an antisense polynucleotide comprising the entire or part of base sequence complementary or substantially complementary to a base sequence for a polynucleotide encoding said polypeptide, its amide or ester, or a salt thereof, (iv) a compound or its salt that inhibits the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) an antibody to said protein, its partial peptide, or a salt thereof, or (vi) an antisense polynucleotide comprising the entire or part of base sequence complementary or substantially complementary to a base sequence for a polynucleotide encoding said protein, its partial peptide, or a salt thereof.

**19.** A method of preventing/treating upper digestive tract disorders, which comprises (a) inhibiting the activity of a polypeptide comprising the same or substantially the same amino acid sequence as the amino acid sequence represented by SEQ ID NO: 1, its amide or ester, or a salt thereof, or (b) inhibiting the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof.

**20.** Use of (i) a compound or its salt that inhibits the activity of a polypeptide comprising the same or substantially the same amino acid sequence as the amino acid sequence represented by SEQ ID NO: 1, its amide or ester, or a salt thereof, (ii) an antibody to said polypeptide, its amide or ester, or a salt thereof, (iii) an antisense polynucleotide comprising the entire or part of base sequence complementary or substantially complementary to a base sequence for a polynucleotide encoding said polypeptide, its amide or ester, or a salt thereof, (iv) a compound or its salt that inhibits the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) an antibody to said protein, its partial peptide, or a salt thereof, or (vi) an antisense polynucleotide comprising the entire or part of base sequence complementary or substantially complementary to a base sequence for a polynucleotide encoding said protein, its partial peptide, or a salt thereof, to manufacture a gastric acid secretion inhibitor.

**21.** A method of suppressing gastric acid secretion, which comprises administering to a mammal an effective dose of (i) a compound or its salt that inhibits the activity of a polypeptide comprising the same or substantially the same amino acid sequence as the amino acid sequence represented by SEQ ID NO: 1, its amide or ester, or a salt thereof, (ii) an antibody to said polypeptide, its amide or ester, or a salt thereof, (iii) an antisense polynucleotide comprising the entire or part of base sequence complementary or substantially complementary to a base sequence for a polynucleotide encoding said polypeptide, its amide or ester, or a salt thereof, (iv) a compound or its salt that inhibits the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) an antibody to said protein, its partial peptide, or a salt thereof, or (vi) an antisense polynucleotide comprising the entire or part of base sequence complementary or substantially complementary to a base sequence for a polynucleotide encoding said protein, its partial peptide, or a salt thereof.

**22.** A method of suppressing gastric acid secretion, which comprises (a) inhibiting the activity of a polypeptide comprising the same or substantially the same amino acid sequence as the amino acid sequence represented by SEQ ID NO: 1, its amide or ester, or a salt thereof, or (b) inhibiting the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof.

**23.** Use of (i) a compound or its salt that inhibits the activity of a polypeptide comprising the same or substantially the same amino acid sequence as the amino acid sequence represented by SEQ ID NO: 1, its amide or ester, or a salt thereof, (ii) an antibody to said polypeptide, its amide or ester, or a salt thereof, (iii) an antisense polynucleotide comprising the entire or part of base sequence complementary or substantially complementary to a base sequence for a polynucleotide encoding said polypeptide, its amide or ester, or a salt thereof, (iv) a compound or its salt that inhibits the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) an antibody to said protein, its partial peptide, or a salt thereof, or (vi) an antisense polynucleotide comprising the entire or part of base sequence complementary or substantially complementary to a base sequence for a polynucleotide encoding said protein, its partial peptide, or a salt thereof, to manufacture a preventive/therapeutic agent for upper digestive tract disorders.

**24.** A method of preventing/treating dyspepsia, bone metabolism disorders or anemia, which comprises administering to a mammal an effective dose of (i) a compound or its salt that promotes the activity of a polypeptide comprising the same or substantially the same amino acid sequence as the amino acid sequence represented by SEQ ID NO: 1, its amide or ester, or a salt thereof, (ii) said polypeptide, its amide or ester, or a salt thereof, (iii) a polynucleotide encoding said polypeptide, its amide or ester, or a salt thereof, (iv) a compound or its salt that promotes the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) said protein, its partial peptide, or a salt thereof, or (vi) a polynucleotide encoding said protein, its partial peptide, or a salt thereof.

**25.** A method of preventing/treating dyspepsia, bone metabolism disorders or anemia, which comprises (a) promoting the activity of a polypeptide comprising the same or substantially the same amino acid sequence as the amino acid sequence represented by SEQ ID NO: 1, its amide or ester, or a salt thereof, or (b) promoting the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof.

**26.** Use of (i) a compound or its salt that promotes the activity of a polypeptide comprising the same or substantially the same amino acid sequence as the amino acid sequence represented by SEQ ID NO: 1, its amide or ester, or a salt thereof, (ii) said polypeptide, its amide or ester, or a salt thereof, (iii) a polynucleotide encoding said polypeptide, its amide or ester, or a salt thereof, (iv) a compound or its salt that promotes the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) said protein, its partial peptide, or a salt thereof, or (vi) a polynucleotide encoding said protein, its partial peptide, or a salt thereof, to manufacture a preventive/therapeutic agent for dyspepsia, bone metabolism disorders or anemia.

**27.** A method of promoting gastric acid secretion, which comprises administering to a mammal an effective dose of (i) a compound or its salt that promotes the activity of a polypeptide comprising the same or substantially the same amino acid sequence as the amino acid sequence represented by SEQ ID NO: 1, its amide or ester, or a salt thereof, (ii) said polypeptide, its amide or ester, or a salt thereof, (iii) a polynucleotide encoding said polypeptide, its amide or ester, or a salt thereof, (iv) a compound or its salt that promotes the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) said protein, its partial peptide, or a salt thereof, or (vi) a polynucleotide encoding said protein, its partial peptide, or a salt thereof.

**28.** A method of promoting gastric acid secretion, which comprises (a) promoting the activity of a polypeptide comprising the same or substantially the same amino acid sequence as the amino acid sequence represented by SEQ ID NO: 1, its amide or ester, or a salt thereof, or (b) promoting the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof.

**29.** Use of (i) a compound or its salt that promotes the activity of a polypeptide comprising the same or substantially the same amino acid sequence as the amino acid sequence represented by SEQ ID NO: 1, its amide or ester, or a salt thereof, (ii) said polypeptide, its amide or ester, or a salt thereof, (iii) a polynucleotide encoding said polypeptide, its amide or ester, or a salt thereof, (iv) a compound or its salt that promotes the activity of a protein comprising the same or substantially the same amino acid sequence as the amino acid sequence represented by SEQ ID NO: 73, SEQ ID NO: 75, SEQ ID NO: 77 or SEQ ID NO: 79, its partial peptide or a salt thereof, (v) said protein, its partial peptide, or a salt thereof, or (vi) a polynucleotide encoding said protein, its partial peptide, or a salt thereof, to manufacture a gastric acid secretion promoter.

**30.** A method of testing gastric secretory function, which comprises using a polypeptide comprising the same or substantially the same amino acid sequence as the amino acid sequence represented by SEQ ID NO: 1, its amide or ester, or a salt thereof, or a compound or its salt that promotes the activity of said polypeptide, its amide or ester, or a salt thereof.

FIG. 1

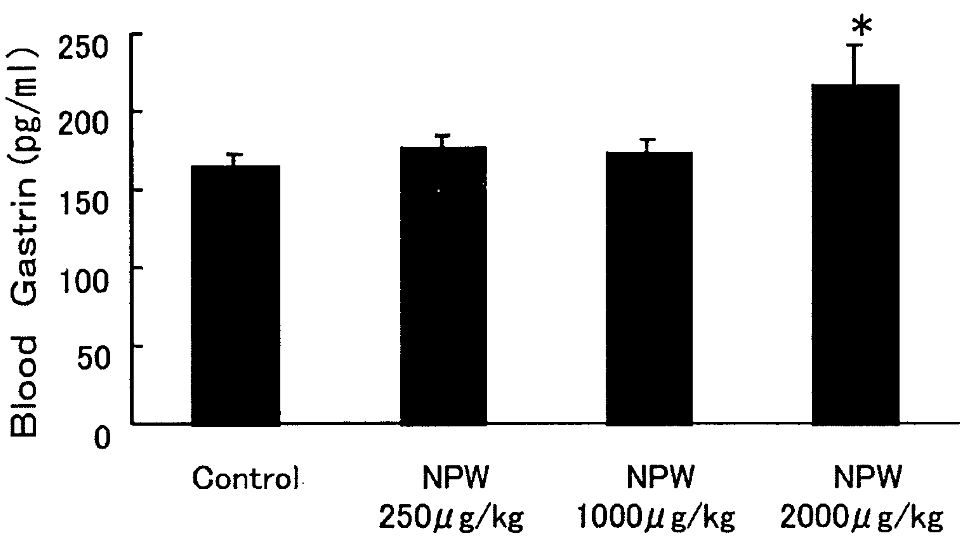
Blood Gastrin (pg/ml)
250
200
150
100
50
0
*
Control
NPW 250μg/kg
NPW 1000μg/kg
NPW 2000μg/kg

**INTERNATIONAL SEARCH REPORT**

International application No.
PCT/JP2004/003227

A. CLASSIFICATION OF SUBJECT MATTER
Int.Cl[7] A61K45/00, A61P1/14, 3/12, 7/06

According to International Patent Classification (IPC) or to both national classification and IPC

B. FIELDS SEARCHED

Minimum documentation searched (classification system followed by classification symbols)
Int.Cl[7] A61K45/00, A61P1/14, 3/12, 7/06

Documentation searched other than minimum documentation to the extent that such documents are included in the fields searched

Electronic data base consulted during the international search (name of data base and, where practicable, search terms used)
MEDLINE CAPLUS EMBASE BIOSIS(STN), JMEDPLUS(JOIS)

C. DOCUMENTS CONSIDERED TO BE RELEVANT

| Category* | Citation of document, with indication, where appropriate, of the relevant passages | Relevant to claim No. |
|---|---|---|
| A | WO 01/98494 A1 (Takeda Chemical Industries, Ltd.), 27 December, 2001 (27.12.01), Full text & EP 1293567 A1 | 1-17,20,23, 26,29 |
| A | SHIMOMURA Y. et al., 'Identification of neuro peptide W as endogenous ligand for orphan G-protein-coupled receptors GPR7 and GPR8; J.Biol. Chem., 27 September, 2002 (27.09.02); 277(39); 35826-32., Epub, 18 July, 2002 (18.07.02) | 1-17,20,23, 26,29 |
| A | Brezillon S. et al., 'Identification of natural ligands for the orphan G. Protein-coupled receptors GPR7 and GPR8; J.Biol.Chem., 10 January, 2003 (10.01.03); 278(2):776-83., Epub 24, October, 2002 (24.10.02) | 1-17,20,23, 26,29 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 April, 2004 (28.04.04) | 18 May, 2004 (18.05.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/003227

**Box No. I Nucleotide and/or amino acid sequence(s) (Continuation of item1.b of the first sheet)**

1. With regard to any nucleotide and/or amino acid sequence disclosed in the international application and necessary to the claimed invention, the international search was carried out on the basis of:

   a. type of material

      [X] a sequence listing

      [ ] table(s) related to the sequence listing

   b. format of material

      [ ] in written format

      [X] in computer readable form

   c. time of filing/furnishing

      [ ] contained in the international application as filed

      [X] filed together with the international application in computer readable form

      [ ] furnished subsequently to this Authority for the purposes of search

2. [X] In addition, in the case that more than one version or copy of a sequence listing and/or table relating thereto has been filed or furnished, the required statements that the information in the subsequent or additional copies is identical to that in the application as filed or does not go beyond the application as filed, as appropriate, were furnished.

3. Additional comments:

Form PCT/ISA/210 (continuation of first sheet (1)) (January 2004)

**INTERNATIONAL SEARCH REPORT**

International application No.
PCT/JP2004/003227

**Box No. II Observations where certain claims were found unsearchable (Continuation of item 2 of first sheet)**

This international search report has not been established in respect of certain claims under Article 17(2)(a) for the following reasons:

1. [x] Claims Nos.: 18, 19, 21, 22, 24, 25, 27, 28, 30
because they relate to subject matter not required to be searched by this Authority, namely:
Claims 18, 19, 21, 22, 24, 25, 27 and 28 pertain to methods for treatment of the human body by therapy and thus relate to a subject matter which this International Searching Authority is not required, under the provisions of Article 17(2)(a)(i) of the PCT and Rule 39.1(iv) (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

INTERNATIONAL SEARCH REPORT

International application No.
PCT/JP2004/003227

Continuation of Box No.II-1 of continuation of first sheet(2)

of the Regulations under the PCT, to search.

Claim 30 pertains to diagnostic methods to be practiced on the human body and thus relates to a subject matter which this International Searching Authority is not required, under the provisions of Article 17(2)(a)(i) of the PCT and Rule 39.1(iv) of the Regulations under the PCT, to search.

<Subject of Search>

Claim 1 relates to a preventive/remedy for upper digestive tract diseases which contains as the active ingredient a compound defined by a desired property "a compound or its salt inhibiting the activity of a polypeptide containing an amino acid sequence which is the same or substantially the same as the amino acid sequence represented by SEQ ID NO:1 or its amide, ester or salt". Although claim 1 involves any compound having this property, no compound is disclosed in the meaning within PCT Article 5. It is therefore recognized that claim 1 is not supported by the disclosure in the description in the meaning within PCT Article 6.

Although the common technical knowledge at the point of the application is taken into consideration, the scope of the compounds having the property as "a compound or its salt inhibiting the activity of a polypeptide containing an amino acid sequence which is the same or substantially the same as the amino acid sequence represented by SEQ ID NO:1 or its amide, ester or salt" cannot be specified. Thus, claim 1 does not comply with the requirement of clearness in the meaning within PCT Article 6.

Similarly, it is recognized that claims 2 to 5, 8, 10, 20, 23, 26 and 29 do not comply with the requirement of clearness in the meaning within PCT Article 6.

Such being the case, the search was made on the relationships among neuropeptides W and GPR8 and diseases in the upper digestive tract, gastric acid secretion, indigestion, bone metabolic error and anemia.

Form PCT/ISA/210 (extra sheet) (January 2004)